# EUROPEAN PATENT APPLICATION

(11) **EP 4 783 104 A1**
(43) Date of publication of application: **29.07.2026**
(21) Application number: 26185510.0
(22) Date of filing: 28.03.2024
(51) Int. Cl.: G06Q 99/00, G16H 40/60, H04L 67/12, H04L 67/303, H04W 84/00, G16H 40/67, G06Q 50/26, G16H 10/60, G16H 40/63

(54) **DECENTRALIZED MEDICAL DEVICE NETWORK**

(30) Priority: 31.03.2023 US 202363493419 P
(62) Divisional of application: 24721383.8
(71) Applicant: ZOLL Medical Corporation, Chelmsford, MA 01824-4105 (US)
(72) Inventor: GIACOMETTI, Paolo, Grafton, 01536 (US); FORESTER, Frederick W., Encinitas, 92024 (US); GOUTMANN, Peter G., Gibsonia, 15044 (US); JOHNSON, Guy R., Wilton, 03086 (US); BETZ, David M., Bedford, 03110 (US); WENG, Binwei, Andover, 01810 (US); FREEMAN, Gary A., Waltham, 02453 (US)
(74) Representative: Potter Clarkson

(57) **Abstract**

A computer-implemented method of sharing data between medical devices associated with a patient includes forming a decentralized network by the medical devices. Each medical device includes a network communications driver, at least one device configured to gather patient data from the patient, and a processor configured to generate a network message including device capabilities of a respective medical device. The method further includes publishing the network message to the decentralized network, ranking the device capabilities of first medical devices by a second medical device, selecting a desired source for patient data based on the ranking, publishing a request for the patient data, subscribing to the desired source, in response, publishing the patient data to the decentralized network by the desired source, routing the patient data to the second medical device by the decentralized network, and consuming the patient data from the desired source by the second medical device.

## Description

### CROSS-REFERENCE TO RELATED APPLICATIONS

This application claims the benefit under 35 U.S.C. §119(e) of U.S. Provisional Application No. 63/493,419 filed March 31, 2023. All subject matter set forth in the above referenced application is hereby incorporated by reference in its entirety into the present application as if fully set forth herein.

### BACKGROUND

A patient care environment is a patient-centric environment in which medical devices, computing devices and caregivers are focused on the care of a single patient. This environment may be situated, for example, at the scene of a collision or health emergency, on a battlefield or other military setting, in an ambulance, in an emergency room or hospital, etc. Within the patient care environment, one or more caregivers, or rescuers, attend to the needs of the patient. These caregivers may include a civilian responder with limited or no training in lifesaving techniques, a first responder with basic skills, such as an emergency medical technician (EMT), police officer, or firefighter, a paramedic or EMS medical director with advanced skills, or a medical professional, such as a physician or nurse. Often, care is provided in stages with transitions between groups of caregivers. For example, in an emergency response, care may begin with basic life support, transition to advanced life support, and transition again to hospital care. The caregivers may utilize a constellation or suite of medical and computing devices that are available to connect to and/or collect information from or about that single patient at any given point in time. The available and/or utilized medical and computing devices may change as a patient's medical status changes and may change when caregivers transition or are added to a patient's care team.

The patient care environment may be within a larger medical environment. For example, there may be multiple patients at a scene of a car crash, a fire, or in a mass casualty situation. There may be multiple patients in a hospital emergency room or other hospital department. A battlefield or other military setting may also include multiple patients and thus multiple patient care environments. Due to practical limitations in staffing and equipment availability, the caregivers, medical devices, and/or computing devices may need to move between patients.

In order to provide effective, timely, and accurate medical care, it is important that a body of patient information collected for a patient is readily available for use by caregivers and by medical and computing devices. These devices use this information to inform treatment and intervention parameters, caregiver guidance, and protocol selection, to name a few examples. The ability to transfer or share patient information amongst the various devices in the patient care environment facilitates that effective, timely, and accurate medical care.

### SUMMARY

The foregoing general description of the illustrative implementations and the following detailed description thereof are merely exemplary aspects of the teachings of this disclosure and are not restrictive.

An example of a computer-implemented method of sharing data between medical devices associated with a patient includes forming a decentralized network by a plurality of nodes associated with the patient, the plurality of nodes including a plurality of medical devices, wherein each medical device of the plurality of medical devices includes a communications interface including a signal transmitter and a network communications driver, at least one patient interface device configured to gather patient data from the patient, and a processor, memory, and associated circuitry, the processor of each of the plurality of medical devices being configured to generate a network message including device capabilities of a respective medical device. The method further includes publishing the network message to the decentralized network by each medical device, ranking the device capabilities of two or more first medical devices of the plurality of medical devices in the decentralized network by a second medical device of the plurality of medical devices in the decentralized network to identify one or more candidate data sources, selecting a desired source for the patient data from the one or more candidate data sources based on the ranking, publishing a request for the patient data indicating the desired source by the second medical device, subscribing to the desired source by the second medical device, publishing the patient data to the decentralized network by the desired source in response to the request, routing the patient data from the desired source to the second medical device by the decentralized network, and consuming the patient data from the desired source by the second medical device.

Implementations of such a method may include one or more of the following features. The plurality of medical devices may include at least one of a patient monitor, patient sensor hub, a patient monitor and/or defibrillator, a portable ventilation device, an automated external defibrillator (AED), an automated compression device, a wearable cardio-defibrillator (WCD), a trauma kit, a drug delivery device, and an ultrasound imaging device. In one or more examples, the device capabilities of the respective medical device comprise one or more: types of patient data available from the respective medical device; a patient data sampling frequency associated with one or more types of patient data; a patient data resolution associated with one or more types of patient data, data transmission parameters for the respective medical device including a current signal strength, an indication of an active wired communicative coupling, or an indication of an active wireless communicative coupling; and a decentralized network routing protocol supported by the respective medical device.

In one or more examples, the publishing may include one or more of: broadcasting; multicasting; transmitting using a one-to-many transmission method; posting to a data source accessible to the plurality of medical devices; or transmitting to each of the plurality of medical devices. In one or more examples, the publishing of the patient data includes selecting the patient data indicated in the request from a plurality of available patient data or selecting the patient data indicated in the request in a format indicated in the request and publishing the selected data or publishing the selected data in the format indicated in the request. In one or more examples, the ranking of the device capabilities is based on predetermined data subscription criteria, such as data subscription criteria of the second medical device. In one or more examples, the act of consuming may include the medical device that requested the data receiving the data through the decentralized network and utilizing that data as needed, such as for a medical or patient related purpose.

In one or more examples, at least a subset of the plurality of medical devices are communicatively coupled with a respective physiological sensor for acquiring one or more measurements of the patient for the medical device with which the respective physiological sensor is associated for generating the patient data.

The method may include providing an indication at one or more nodes of associations between data consumers and data suppliers in the decentralized network. The method may include joining the decentralized network by a mobile computing device and displaying the indication on a display screen of the mobile computing device. The method may include displaying the indication on the display screen as part of a caregiver decision support user interface. The method may include providing the indication at each of the data suppliers in response to a query at a caregiver decision support screen or another decentralized network device to determine which data supplier is providing data to a particular data consumer. The device capabilities of the respective medical device may include one or more types of patient data available from the respective medical device. The method may include determining a desired type of patient data by the second medical device, identifying the two or more first medical devices as potential sources for the desired type of patient data, and selecting the desired source from the potential sources. The desired type of patient data may be one of a plurality of types of patient data available from the desired source. The method may include publishing the desired type of patient data to the decentralized network by the desired source without publishing other types of patient data available from the desired source. The device capabilities of the respective medical device may include one or more of a patient data sampling frequency or a patient data resolution associated with each of the one or more types of patient data. The method may include selecting the desired source based at least in part on one or more of the patient data sampling frequency or the patient data resolution. The method may include determining a desired data compression for a patient data type based at least in part on one or more of the patient data sampling frequency or the patient data resolution, publishing the request for the patient data with an indication of the desired data compression, compressing the patient data by the desired source in response to the published request, and publishing the compressed patient data to the decentralized network by the desired source. The method may include receiving an indication by the second medical device that the desired source has exited the decentralized network, identifying a substitute source for the desired type of patient data in the decentralized network by the second medical device, and re-publishing the request for the patient data by the second medical device with an indication of the substitute source. The two or more first medical devices may be previously identified sources for the desired type of patient data. The method may include joining the decentralized network by one or more third medical devices, wherein at least one of the one or more third medical devices is capable of providing the desired type of patient data, publishing network messages of the one or more third medical devices to the decentralized network by the one or more third medical devices, ranking the device capabilities of the two or more first medical devices with the one or more third medical devices, and selecting an updated desired source. The device capabilities of the respective medical device may include data transmission parameters for the respective medical device. The data transmission parameters may include one or more of a signal strength, an indication of a wired communicative coupling, or an indication of a wireless communicative coupling. The data transmission parameters may include a decentralized network routing protocol for the respective medical device. The method may include assigning a patient identification code to the patient, generating a patient-specific network access code from the patient identification code by a first medical device of the plurality of medical devices or by a mobile computing device, receiving join requests at the first medical device from a remainder of the plurality of medical devices or at the mobile computing device from the plurality of medical devices, providing the patient-specific network access code to the remainder of the plurality of medical devices by the first medical device or to the plurality of medical devices by the mobile computing device, and forming the decentralized network based on the patient-specific network access code. The method may include assigning the patient identification code by a computer aided dispatch (CAD) service and sending the patient identification code from the CAD service to the first medical device or the mobile computing device. The method may include affixing a quick response (QR) code or bar code including the patient identification code to the patient by a caregiver to assign the patient identification code and scanning the QR code or bar code by the first medical device or the mobile computing device to generate the patient-specific network access code. The mobile computing device may include a patient charting device, a dispatch interface device, or a combination thereof. The method may include enabling an exchange of handshake information amongst a collection of medical devices and computing devices prior to an arrival at a patient scene of any of the collection of medical devices and computing devices to pre-configure the collection of medical devices and computing devices for decentralized network entry, generating an inventory list, and storing the inventory list in a storage location accessible to the collection of medical devices. The collection of medical devices and computing devices may include the plurality of medical devices and the mobile computing device and is associated with at least one of an EMS agency, an EMS transport vehicle, or an EMS crew. The storage location may be a central registration service, a peer-to-peer storage, a blockchain ledger, or a combination thereof. The method may include joining the decentralized network by at least one additional medical device including the network communications driver, and permanently exiting the decentralized network by at least one of the plurality of medical devices in the decentralized network. The plurality of medical devices may be associated with a basic life support (BLS) emergency medical services (EMS) crew and the at least one additional medical device may be associated with an advanced life support (ALS) EMS crew. The plurality of medical devices may include an automated external defibrillator and a trauma kit. The plurality of medical devices may be associated with an EMS agency, and the at least one additional medical device may be associated with a hospital. The plurality of medical devices may include a physiological sensor coupled to the patient. The method may include forming the decentralized network with nodes including a patient charting device, receiving physiological parameter values by the patient charting device from the physiological sensor via the decentralized network, monitoring the physiological parameter values for the patient by the BLS EMS crew at the patient charting device, joining the decentralized network by at least one ALS medical device, permanently exiting the decentralized network by the patient charting device, and receiving the physiological parameter values by the at least one ALS medical device from the physiological sensor via the decentralized network, wherein the at least one ALS medical device may receive the physiological parameter values without a communicative coupling between the at least one ALS medical device and the physiological sensor other than through the decentralized network. The at least one ALS medical device may include one or more of an ALS patient-monitor, an ALS patient-monitor/defibrillator, or a critical care ventilator. The physiological sensor may include an SpO2 sensor. The plurality of medical devices may include a BLS respiratory distress management (RDM) system coupled to the patient via a ventilation hose and managing respiratory distress based on a set of RDM parameters. The method may include joining the decentralized network by an ALS critical care ventilator, receiving the set of RDM parameters by the ALS critical care ventilator via the decentralized network from the BLS RDM system, setting operational parameters for the ALS critical care ventilator to the set of RDM parameters, moving a connection port of the ventilation hose coupled to the patient from the BLS RDM system to the ALS critical care ventilator during a single inter-breath time interval, exiting the decentralized network by the BLS RDM system, and providing ventilation support for the patient with the ALS critical care ventilator based on the set of RDM parameters. The ALS critical care ventilator may receive the set of RDM parameters from the BLS RDM system without a communicative coupling between the BLS RDM system and the ALS critical care ventilator other than through the decentralized network. The method may include receiving the set of RDM parameters by the ALS critical care ventilator via the decentralized network from the BLS RDM system, setting the operational parameters for the ALS critical care ventilator to the set of RDM parameters, and moving the connection port of ventilation hose coupled to the patient from the BLS RDM system to the ALS critical care ventilator, all during a single inter-breath delivery time interval. The plurality of medical devices may include a first medical device and a first sensor. The method may include initiating closed loop control of the first medical device based on first patient data from the first sensor by a node in the decentralized network, and in response to the initiating, publishing first patient data to the decentralized network by the first sensor, and subscribing to the first patient data by the first medical device, receiving the first patient data at the first medical device via the decentralized network, and operating the first medical device in closed loop control based on the first patient data. The first medical device may include a portable ventilator and the first sensor includes an oxygen saturation sensor. The method may include permanently exiting the decentralized network by the node in the decentralized network and maintaining the closed loop control of the first medical device in without the exited node based on a continued provision of the first patient data from the first sensor to the first medical device via the decentralized network. The method may include entering the decentralized network by a new medical device or a new sensor, publishing the network message to the decentralized network by the new medical device or the new sensor, wherein the network message indicates new patient data available from the new medical device or the new sensor, determining by the first medical device that the new patient data is interchangeable with the first patient data, subscribing to the new patient data by the first medical device, receiving the new patient data via the decentralized network, and modifying the closed loop control of the first medical device to operate based on the new patient data instead of the first patient data. The method may include forming the decentralized network with nodes including a mobile computing device, wherein the node initiating the closed loop control is the mobile computing device. The method may include initiating the closed loop control by another medical device of the plurality of medical devices. The first medical device may include a portable ventilator, the first sensor includes an oxygen saturation sensor, and the another medical device includes a patient-monitor/defibrillator. The method may include forming the decentralized network by the plurality of nodes including at least one device configured to calculate a patient evaluation score, receiving constituent information for the patient evaluation score, calculating the patient evaluation score based on the constituent information, publishing the patient evaluation score to the decentralized network, and utilizing the patient evaluation score at a node in the decentralized network other than the at least one device configured to calculate the patient evaluation score. The utilizing may include displaying the patient evaluation score, incorporating the patient evaluation score into a patient data analysis, or a combination thereof. The constituent information may include caregiver observation input. The method may include forming the decentralized network by the plurality of nodes, the plurality of nodes including at least one device configured to receive caregiver observation input, receiving the caregiver observation input at the at least one device configured to receive caregiver observation input, calculating the patient evaluation score based at least in part on the caregiver observation input by the at least one device configured to calculate the patient evaluation score, publishing the patient evaluation score to the decentralized network by the at least one device configured to calculate the patient evaluation score, and displaying the patient evaluation score at the node in the decentralized network other than the at least one device configured to calculate the patient evaluation score. The constituent information may include physiological sensor measurements. The method may include collecting one or more physiological sensor measurements at one or more respective patient interface devices, publishing the one or more physiological sensor measurements to the decentralized network, receiving the one or more physiological sensor measurements via the decentralized network at the at least one device configured to calculate the patient evaluation score, and calculating the patient evaluation score based on the one or more physiological sensor measurements. The patient evaluation score may include a modified early warning score (MEWS). The method may include collecting a blood pressure (BP) measurement, a temperature measurement, a heart rate (HR) measurement, and a respiratory rate (RR) measurement at a BP sensor, a temperature sensor, a HR sensor, and a RR sensor, respectively, wherein the BP sensor, the temperature sensor, the HR sensor, and the RR sensor are all nodes in the decentralized network, publishing the BP measurement, the temperature measurement, the HR measurement, and the RR measurement to the decentralized network, receiving caregiver evaluations of alertness, voice, pain, and unresponsive (AVPU) scale at the at least one device configured to receive the caregiver observation input, publishing the AVPU scale to the decentralized network, receiving the BP measurement, the temperature measurement, the HR measurement, the RR measurement, and the AVPU scale at the at least one device configured to calculate the MEWS, calculating the MEWS at the at least one device configured to calculate the patient evaluation score, and publishing the MEWS to the decentralized network. The method may include receiving the patient evaluation score at a decentralized network node including a single parameter physiological sensor configured to measure a single physiological parameter, receiving the patient evaluation score via the decentralized network, and displaying the patient evaluation score and the single physiological parameter at the single parameter physiological sensor. The patient evaluation score may be a Glasgow coma score (GCS) score and the single parameter physiological sensor may be a blood glucose monitor. The single parameter physiological sensor may be a disposable sensor. The at least one device configured to calculate the patient evaluation score may include a compensatory reserve index (CRI) sensor configured to calculate a CRI for the patient, and the plurality of nodes may include an ultrasound imaging device. The method may include publishing the CRI to the decentralized network by the CRI sensor and displaying the CRI at the ultrasound imaging device. The method may include including generating an alert at one or more nodes of the decentralized network based on the patient evaluation score, wherein the calculating of the patient evaluation score requires information from at least two different nodes in the decentralized network. The method may include forming the decentralized network in a military environment, wherein each of the plurality of medical devices is configured to be carried in a backpack, worn by military personnel, or a combination thereof. The plurality of medical devices may include at least one of a ruggedized patient-monitor weighing less than 5 kg, a ruggedized patient-monitor/defibrillator weighing less than 6 kg, an impedance threshold device coupled to a facemask or a mouthpiece, a ruggedized portable ventilator weighing less than 5 kg, or a portable and self-contained suction device. The ruggedized patient-monitor, the ruggedized patient-monitor/defibrillator, and the ruggedized portable ventilator may be configured to withstand a shock g-force of 65-85 g. The plurality of medical devices may include at least one of a tourniquet, an intravenous delivery system, an airway management device, a diagnostic device including at least one of a blood pressure cuff, a pulse oximeter, a stethoscope, or a thermometer, a medication kit, a stretcher, or a surgical kit. The plurality of medical devices may be associated with one or more military caregivers, and a total weight of one or more medical devices associated with any one military caregiver may be less than 45 kg. The method may include forming the decentralized network in the military environment without Internet access for the decentralized network. At least one of the plurality of medical devices may include a Wi-Fi chip. The method may include disabling Wi-Fi frequency transmissions for the at least one of the plurality of medical devices that includes the Wi-Fi chip. The method may include collecting a first physiological parameter at a first node of the plurality of nodes and a second physiological parameter at a second node of the plurality of nodes, and publishing the first physiological parameter and the second physiological parameter to the decentralized network by the first node and the second node, respectively, and monitoring the first physiological parameter and the second physiological parameter by a third node different from the first node and the second node. At least two of the plurality of medical devices may be disposed at different locations on a military caregiver. All of the plurality of medical devices may be pocket-sized, wearable, or a combination thereof. The plurality of medical devices may be distributed amongst two or more military caregivers. The third node may include a ruggedized patient-monitor, a ruggedized patient-monitor/defibrillator, or a ruggedized portable ventilator. The plurality of nodes may include a computer tablet or smartphone and the third node may be one of the computer tablet or the smartphone. The method may include exiting the decentralized network by the third node, entering the decentralized network by a fourth node, and monitoring the first physiological parameter and the second physiological parameter by the fourth node. The method may include forming the decentralized network by the plurality of nodes including at least one caregiver guidance device configured to dynamically determine caregiver guidance, receiving the patient data at the at least one caregiver guidance device, dynamically determining caregiver guidance by the at least one caregiver guidance device based on the patient data and the plurality of medical devices in the decentralized network, providing a caregiver guidance user interface (UI) at one or more nodes in the decentralized network, and providing the caregiver guidance at the caregiver guidance UI by the at least one caregiver guidance device. The plurality of medical devices may include the at least one caregiver guidance device. The at least one caregiver guidance device may be a mobile computing device. Providing the caregiver guidance UI may include providing the caregiver guidance UI at the at least one caregiver guidance device. Providing the caregiver guidance UI may include providing a distributed UI at two or more nodes of the decentralized network. Dynamically determining the caregiver guidance may include determining the caregiver guidance based on the plurality of medical devices in the decentralized network, exiting the decentralized network by at least one medical device of the plurality of medical devices, and modifying the caregiver guidance in response to the exiting, and providing the modified caregiver guidance at the caregiver guidance UI. Dynamically determining the caregiver guidance may include determining the caregiver guidance based on the plurality of medical devices in the decentralized network, entering the decentralized network by at least one new medical device, and modifying the caregiver guidance in response to the entering, and providing the modified caregiver guidance at the caregiver guidance UI. The method may include provisioning at least one services library with EMS medical device information, forming the decentralized network by the plurality of nodes including the at least one services library, tagging a subset of the EMS medical device information as medical device information corresponding to the plurality of medical devices based on the published network messages, and providing at least a portion of the tagged subset of the EMS medical device information to the at least one caregiver guidance device. The tagged subset of the EMS medical device information may include at least one of medical device drivers, medical device operation and assembly instructions, medical device operation specifications, or medical device identification information. The method may include providing a graphical representation of at least one of the plurality of medical devices at the caregiver guidance UI. The method may include providing a user control associated with the graphical representation, receiving a selection of the user control, receiving, via the decentralized network, operational interface information from the graphically represented at least one medical device, and displaying the operational interface information at the caregiver guidance UI. The method may include joining the decentralized network by at least one mobile computing device having the network communications driver, subscribing to the first patient data by the at least one mobile computing device, and publishing second patient data to the decentralized network by the at least one mobile computing device. The at least one mobile computing device may include a patient charting device. The method may include receiving the first patient data at the patient charting device, mapping the first patient data to one or more data fields in an electronic patient care record (ePCR), and storing the first patient data in the ePCR. At least one of the plurality of medical devices may include at least one of a patient-monitor, a patient-monitor/defibrillator, a portable ventilation device, an automated external defibrillator (AED), or an automated compression device. The method may include receiving the second patient data including one or more of patient demographic data or symptom-allergy-medication-pertinent history (SAMPLE) data and publishing at least one of the patient demographic data or the SAMPLE data to the decentralized network. The at least one mobile computing device may include an in-vehicle computer aided dispatch (CAD) and navigation interface (CAD-NAV) device configured to communicate with a CAD service and with a positioning system, such as the global positioning system (GPS) or other geolocation system. The method may include receiving the second patient data at the patient charting device from the CAD service via the CAD-NAV device and the decentralized network without a communicative coupling between the patient charting device and a remote patient charting server. The method may include receiving location information at the patient charting device from the CAD-NAV device, the location information including a current EMS vehicle GPS location and a patient transport destination, determining a travel time from the current EMS vehicle GPS location to the patient transport destination, generating a caregiver recommendation by the patient charting device based at least in part on the location information, and providing the caregiver recommendation via a user interface of the patient charting device. Generating the caregiver recommendation may include sorting patient care protocol tasks into on-scene tasks prior to transport and in-ambulance tasks during transport. The method may include generating a caregiver alert to begin patient transport and transition from the on-scene tasks to the in-ambulance tasks. The caregiver recommendation may include one of a fluid delivery rate or volume or a medication dosage based on the travel time to the patient transport destination. The method may include documenting at least one patient condition in the ePCR, providing the at least one patient condition to the CAD-NAV device, modifying the patient transport destination by the CAD-NAV device based on the at least one patient condition, and reporting the modified patient transport destination to the CAD. The method may include documenting at least one patient condition in the ePCR, providing the at least one patient condition to the CAD-NAV device, determining closest appropriate facility information for the at least one patient condition by the CAD-NAV device based on the location information, providing the closest appropriate facility information to the patient charting device, and automatically recording the closest appropriate facility information in the ePCR. The plurality of medical devices may include a patient sensor hub. The method may include forming the decentralized network with one or more of at least one edge server or a patient-dedicated node. The method may include forming the decentralized network without any nodes having a communicative coupling to the Internet or a remote server. The method may include forming the decentralized network without any nodes using a communicative coupling to the Internet or a remote server to form the decentralized network. At least one of the plurality of medical devices may be non-wearable and configured to be supported by a surface in a patient care environment separate from the patient. The plurality of medical devices may be external medical devices. The plurality of medical devices may be mobile medical devices. The plurality of medical devices may include two or more of a patient-monitor, a patient-monitor/defibrillator, an automated external defibrillator, a portable ventilator, and an automated compression device. The network communications driver includes a network interconnection protocol stack wherein at least one layer of the network interconnection protocol stack for each of the plurality of medical devices is a same layer as each other of the plurality of medical devices. Each of the plurality of medical devices may have a same data link layer, network layer, transport layer, session layer, and/or security layer as each other of the plurality of medical devices. The plurality of nodes may include at least one computing device including the network communications driver provisioned with a network interconnection protocol stack having at least one same layer as each of the plurality of medical devices. The network communications driver may be a first network communications driver associated with a first medical device manufacturer. The method may include requesting to join the decentralized network by at least one medical device associated with a second medical device manufacturer, utilizing a second network communications driver that allows the at least one medical device associated with the second medical device manufacturer to participate in the decentralized network as a data supplier and disallows participation as a data consumer, and entering the decentralized network by the at least one medical device associated with the second medical device manufacturer based on the second network communications driver. The method may include retrieving the second network communications driver from a cloud server. The communications interface of at least one of the plurality of medical devices may include a short-range wireless interface configured to join the decentralized network via a dongle on the at least one of the plurality of medical devices, via an edge server, or via an intermediary medical device of the plurality of medical devices. Forming the decentralized network may include forming a mesh network. The method may include forming a mesh network with a partial mesh topology. The method may include forming a mesh network with a full mesh topology. The decentralized network may include a first subset of devices with an application layer configured to enable publish and subscribe functions and a second subset of devices with an application layer configured to enable publish functions without subscribe functions. The signal transmitter may be configured for wired and/or wireless communications. Ranking the device capabilities may include comparing the device capabilities for the two or more first medical devices to data subscription criteria for the second medical device, identifying one first medical device as a singular data source, and identifying the singular data source as the candidate data source. The data subscription criteria may be default data subscription criteria or customized data subscription criteria. Ranking the device capabilities may include comparing the device capabilities for the two or more first medical devices to data subscription criteria for the second medical device and identifying one or more of the first medical devices as non-candidate data sources. The data subscription criteria may be default data subscription criteria or customized data subscription criteria. Ranking the device capabilities may include comparing the device capabilities for the two or more first medical devices to data subscription criteria for the second medical device, identifying two or more of the first medical devices as candidate data sources, generating a compatibility score for each candidate data source, and ranking the candidate data sources based on the compatibility scores. The data subscription criteria may be default data subscription criteria or customized data subscription criteria. Generating the compatibility score may include generating the compatibility score based on one or more of an evaluation of device capabilities of each candidate data source, the data subscription criteria, or a data accuracy based on a comparison of data from the candidate data sources. The data subscription criteria may include one or more of data types, sampling rates, type of a communicative coupling, data tolerances, bandwidth of the communicative coupling, or reliability of connectivity of the communicative coupling. The method may include identifying exchangeable and nonexchangeable data sources. The method may include detecting a degradation in data provision from the desired source, selecting an exchangeable data source as a new desired data source, publishing a request for patient data indicating the new desired data source, and subscribing to the new desired data source by the second medical device. The method may include comparing the degradation in data provision from the desired source to a threshold degradation, and publishing the request for patient data indicating the new desired data source when the degradation reaches the threshold degradation. The plurality of medical devices may include two or more of a patient-monitor/defibrillator, an automated external defibrillator (AED), or a wearable cardio-defibrillator (WCD), a trauma kit, an automated compression device, a portable ventilation device, a drug delivery device, and an ultrasound imaging device. The network communications driver may include an application layer configured to implement a first wired subscription paradigm and a second wireless subscription paradigm. The method may include implementing the second wireless subscription paradigm, receiving a radio-silence request, and switching to the first wired subscription paradigm.

An example of system for transferring data between medical devices associated with a patient includes a plurality of medical devices communicatively coupled via a decentralized network, wherein each of the plurality of medical devices is a node in the decentralized network, and wherein each medical device of the plurality of medical devices includes a communications interface comprising a signal transmitter and a network communications driver, at least one patient interface device configured to gather patient data from the patient, and a processor, memory, and associated circuitry. At least one first medical device of the plurality of medical devices is configured to receive network messages comprising device capabilities of other medical devices of the plurality of medical device, rank the device capabilities of two or more of the other medical devices in the decentralized network to identify one or more candidate data sources in the decentralized network, select a desired source for the patient data from the one or more candidate data sources based on the ranking, publish a request for the patient data indicating the desired source by the at least one first medical device, subscribe to the desired source, receive the patient data from the desired source via the decentralized network, and consume the patient data from the desired source by the at least one first medical device.

Implementations of such system may include one or more of the following features. The plurality of medical devices may include two or more of a patient-monitor/defibrillator, an automated external defibrillator (AED), or a wearable cardio-defibrillator (WCD), a trauma kit, an automated compression device, a portable ventilation device, a drug delivery device, and an ultrasound imaging device. The decentralized network may include one or more of at least one edge server or a patient-dedicated node. The nodes of the decentralized network may not be communicatively coupled to the Internet or a remote server. At least one of the plurality of medical devices may be non-wearable and configured to be supported by a surface in a patient care environment separate from the patient. The plurality of medical devices may be external medical devices. The plurality of medical devices may be mobile medical devices. The plurality of medical devices may each include a network communications driver that may include a network interconnection protocol stack wherein at least one layer of the network interconnection protocol stack for each of the plurality of medical devices may be a same layer as each other of the plurality of medical devices. Each of the plurality of medical devices may have a same data link layer, network layer, transport layer, session layer and/or security layer as each other of the plurality of medical devices. The plurality of nodes may include at least one computing device comprising the network communications driver provisioned with a network interconnection protocol stack having at least one same layer as each of the plurality of medical devices. The network communications driver may be a first network communications driver associated with a first medical device manufacturer. The decentralized network may include at least one medical device associated with a second medical device manufacturer and the at least one medical device associated with the second medical device manufacturer may be configured to participate in the decentralized network as a data publisher without data subscription. The communications interface of at least one of the plurality of medical devices may include a short-range wireless interface configured to join the decentralized network via a dongle on the at least one of the plurality of medical devices, via an edge server, or via an intermediary medical device of the plurality of medical devices. The decentralized network may be a mesh network. The mesh network may have a partial mesh topology. The mesh network may have a full mesh topology. The decentralized network may include a first subset of devices with an application layer configured to enable publish and subscribe functions and a second subset of devices with an application layer configured to enable publish functions without subscribe functions. The signal transmitter may be configured for wired and/or wireless communications. To rank the device capabilities, the at least one first medical device may be configured to compare the device capabilities for the two or more other medical devices to data subscription criteria for the at least one first medical device, identify one of the other medical devices as a singular data source, and identify the singular data source as the candidate data source. The data subscription criteria may be default data subscription criteria or customized data subscription criteria. To rank the device capabilities, the at least one first medical device may be configured to compare the device capabilities for the two or more other medical devices to data subscription criteria for the at least one first medical device and identify one or more of the other medical devices as non-candidate data sources. The data subscription criteria may be default data subscription criteria or customized data subscription criteria. To rank the device capabilities, the at least one first medical device may be configured to compare the device capabilities for the two or more other medical devices to data subscription criteria for the at least one first medical device, identify two or more of the other medical devices as candidate data sources, generate a compatibility score for each candidate data source, and rank the candidate data sources based on the compatibility scores. The data subscription criteria may be default data subscription criteria or customized data subscription criteria. To generate the compatibility score, the at least one first medical device may be configured to generate the compatibility score based on one or more of an evaluation of device capabilities of each candidate data source, the data subscription criteria, or a data accuracy based on a comparison of data from the candidate data sources. The data subscription criteria may include one or more of data types, sampling rates, type of a communicative coupling, data tolerances, bandwidth of the communicative coupling, or reliability of connectivity of the communicative coupling. The at least one first medical device may be configured to identify exchangeable and nonexchangeable data sources. The at least one first medical device may be configured to detect a degradation in data provision from the desired source, select an exchangeable data source as a new desired data source, publish a request for patient data indicating the new desired data source, and subscribe to the new desired data source by the at least one first medical device. The at least one first medical device may be configured to compare the degradation in data provision from the desired source to a threshold degradation and publish the request for patient data indicating the new desired data source when the degradation reaches the threshold degradation. The decentralized network may include at least one mobile computing device. The at least one mobile computing device may be configured to display a caregiver decision support user interface with an indication of associations between data consumers and data suppliers within the decentralized network. The device capabilities may include one or more types of patient data available from the respective medical device, and one or more of a patient data sampling frequency, data transmission parameters, or a patient data resolution associated with each of the one or more types of patient data. The data transmission parameters may include one or more of a signal strength, an indication of a wired communicative coupling, an indication of a wireless communicative coupling, or routing protocol. At least one node of the decentralized network may be configured to receive a patient identification code for the patient, generate a patient-specific network access code from the patient identification code, receive join requests from one or more of the plurality of medical devices, and provide the patient-specific network access code to the remainder of the plurality of medical devices by the first medical device or to the plurality of medical devices by the mobile computing device to form the decentralized network based on the patient-specific network access code. The patient identification code may be a code assigned by a computer aided dispatch service or a quick response code affixed to the patient. The plurality of medical devices may be configured to exchange handshake information to generate an inventory list for the decentralized network. The inventory list may be available to the decentralized network as a central registration service, a peer-to-peer storage, a blockchain ledger, or a combination thereof. Each of the plurality of medical devices may be configured to be carried in a backpack, worn by military personnel, or a combination thereof. The plurality of medical devices may include at least one of a ruggedized patient-monitor weighing less than 5 kg, a ruggedized patient-monitor/defibrillator weighing less than 6 kg, an impedance threshold device coupled to a facemask or a mouthpiece, a ruggedized portable ventilator weighing less than 5 kg, or a portable and self-contained suction device. The ruggedized patient-monitor, the ruggedized patient-monitor/defibrillator, and the ruggedized portable ventilator are configured to withstand a shock g-force of 65-85 g. The plurality of medical devices may include at least one of a tourniquet, an intravenous delivery system, an airway management device, a diagnostic device comprising at least one of a blood pressure cuff, a pulse oximeter, a stethoscope, or a thermometer, a medication kit, a stretcher, or a surgical kit. The plurality of medical devices may be associated with one or more military caregivers, and a total weight of one or more medical devices associated with any one military caregiver is less than 45 kg. The decentralized network in the military environment may lack Internet access. The decentralized network may include one or more of at least one caregiver guidance device configured to dynamically determine caregiver guidance, a patient charting device configured to generate an ePCR, an in-vehicle computer aided dispatch (CAD) and navigation interface (CAD-NAV) device configured to communicate with a CAD service and with a global positioning system (GPS), and a patient sensor hub.

According to a further aspect we provide a system for transferring data between medical devices associated with a patient, the system comprising: a plurality of medical devices communicatively coupled via a network, wherein each of the plurality of medical devices is a node in the network, and wherein each medical device of the plurality of medical devices comprises: a communications interface comprising a signal transmitter for use in publishing messages to the network and network communications driver, at least one patient interface device configured to gather patient data from the patient, and a processor, memory, and associated circuitry, and wherein at least one first medical device of the plurality of medical devices is configured to execute an application that uses the network communications driver to communicate with other medical devices of the plurality of medical devices and is configured to cause a respective processor to: receive network messages comprising device capabilities of the other medical devices of the plurality of medical devices; rank, based on predetermined criteria, device capabilities of two or more of the other medical devices in the network to identify one or more candidate data sources in the network; select a desired source for the patient data from the one or more candidate data sources based on the ranking; publish a request for the patient data indicating the desired source by the at least one first medical device in order to subscribe to the desired source; receive the patient data from the desired source via the network; and consume the patient data from the desired source by the at least one first medical device.

According to a further aspect we provide a medical device, wherein the medical device comprises: a network communication driver configured to enable the medical device to join a network, such as a decentralized network, with a plurality of other medical devices such that the medical device and the plurality of other medical devices are communicatively coupled via the network; a communications interface comprising a signal transmitter for use in publishing messages to the network and a signal receiver; at least one patient interface device configured to gather patient data from the patient; and a processor, memory, and associated circuitry, and wherein the medical device is configured to cause a respective processor to: receive network messages comprising device capabilities of the other medical devices: rank, based on predetermined criteria, device capabilities of two or more of the other medical devices in the network to identify one or more candidate data sources in the network; select a desired source for the patient data from the one or more candidate data sources based on the ranking; publish a request for the patient data indicating the desired source by the medical device in order to subscribe to the desired source; receive the patient data from the desired source via the network; and consume the patient data from the desired source by the medical device. In one or more examples, the medical device is part of a system including the plurality of other medical devices. In one or more examples of this or any other aspect, the medical device may be configured to publish, automatically, to the network, for receipt by the other medical devices, its device capabilities in response to one or more of: joining the network; a request from one or more of the other medical devices; if the device capabilities include types of patient data, based on the patient data becoming available to the medical device; and if the device capabilities include an acquisition rate of patient data, based on the patient data being acquired at the acquisition rate. In one or more examples, the connection of a patient interface device, such as a sensor, may make the patient data or information on which the patient data is based available to the medical device.

Other capabilities may be provided and not every implementation according to the disclosure must provide any, let alone all, of the capabilities discussed. Further, it may be possible for an effect noted above to be achieved by means other than that noted, and a noted item/technique may not necessarily yield the noted effect.

### BRIEF DESCRIPTION OF THE DRAWINGS

The accompanying drawings, which are incorporated in and constitute a part of the specification, illustrate one or more embodiments and, together with the description, explain these embodiments. The accompanying drawings have not necessarily been drawn to scale. Any values and/or dimensions illustrated in the accompanying graphs and figures are for illustration purposes only and may or may not represent actual or preferred values or dimensions. Where applicable, some or all features may not be illustrated to assist in the description of underlying features.
FIG. 1 shows examples of emergency environments in which a decentralized medical device network may be deployed.
FIG. 2A shows an example of medical devices and computing devices as decentralized network nodes.
FIGS. 2B and 2C shows examples of decentralized network nodes specific to a particular patient condition.
FIG. 2D shows an example of variations in node capabilities within a decentralized network.
FIG. 2E shows examples of patient interface devices.
FIG. 3A shows an example of nodes exiting and entering a decentralized network.
FIG. 3B shows an example of nodes participating in a decentralized network as enabled by a decentralized network communication driver.
FIG. 4A shows an example of a method of hierarchically ranking sources of patient data for consumption by medical devices in a decentralized network.
FIG. 4B shows an example of a ranking process for data subscription in a decentralized network.
FIG. 4C shows an example of a method of interchanging data sources due to data degradation.
FIG. 5 shows a schematic diagram of one medical device ranking device capabilities of at least two other medical devices.
FIG. 6 shows a schematic diagram of a medical device re-ranking possible data sources in response to changes in the decentralized network.
FIG. 7 shows an example of a method of patient-centric decentralized network formation.
FIG. 8 shows examples of sources of a patient identification code.
FIG. 9 shows an example of a device system for initiating the decentralized network based on the patient-specific decentralized network access code.
FIGS. 10 and 11 show an example of a change in medical and/or computing devices in a decentralized network due to a BLS/ALS transition.
FIG. 12 shows a schematic example of a decentralized network enabling a rapid change in respiratory support.
FIGS. 13 and 14 show an example of closed-loop control as facilitated by a decentralized network.
FIG. 15 shows an example of patient evaluation score determination and distribution using a decentralized network.
FIG. 16 shows an example of MEWS score determination and distribution using a decentralized network.
FIG. 17 shows an example of a Glasgow coma score determination and distribution using a decentralized network.
FIG. 18 shows an example of compensatory reserve index distribution using a decentralized network.
FIG. 19 shows an example of distributed system of medical care devices in a military medical care environment.
FIG. 20 shows an example of a method of determining and modifying caregiver guidance based on a decentralized network device inventory.
FIG. 21 shows an example of a caregiver guidance UI.
FIGS. 22A, 22B, and 23 show examples of information provided at a caregiver guidance user interface area.
FIG. 24 an example of computing and medical device nodes within a transport vehicle
FIG. 25 shows examples of components of various devices discussed herein.

### DETAILED DESCRIPTION

The description set forth below in connection with the appended drawings is intended to be a description of various, illustrative embodiments or implementations of the disclosed subject matter. Specific features and functionalities are described in connection with each illustrative embodiment or implementation; however, it will be apparent to those skilled in the art that the disclosed embodiments and implementations may be practiced without each of those specific features and functionalities.

Reference throughout the specification to "one embodiment," "an embodiment," or "an implementation" means that a particular feature, structure, or characteristic described in connection with an embodiment or implementation is included in at least one embodiment or implementation of the subject matter disclosed. Thus, the appearance of the phrases "in one embodiment" or "in an embodiment" or "in an implementation" in various places throughout the specification is not necessarily referring to the same embodiment or implementation. Further, the particular features, structures or characteristics may be combined in any suitable manner in one or more embodiments or implementations. Further, it is intended that embodiments and implementations of the disclosed subject matter cover modifications and variations thereof.

It must be noted that, as used in the specification and the appended claims, the singular forms "a," "an," and "the" include plural referents unless the context expressly dictates otherwise. That is, unless expressly specified otherwise, as used herein the words "a," "an," "the," and the like carry the meaning of "one or more." Additionally, it is to be understood that terms such as "left," "right," "top," "bottom," "front," "rear," "side," "height," "length," "width," "upper," "lower," "interior," "exterior," "inner," "outer," and the like that may be used herein merely describe points of reference and do not necessarily limit embodiments of the present disclosure to any particular orientation or configuration. Furthermore, terms such as "first," "second," "third," etc., merely identify one of a number of portions, components, steps, operations, functions, and/or points of reference as disclosed herein, and likewise do not necessarily limit embodiments of the present disclosure to any particular configuration or orientation.

Furthermore, the terms "approximately," "about," "proximate," "minor variation," and similar terms generally refer to ranges that include the identified value within a margin of 20%, 10% or preferably 5% in certain embodiments, and any values there between.

All of the functionalities described in connection with one embodiment or implementation are intended to be applicable to the additional embodiments and implementations described below except where expressly stated or where the feature or function is incompatible with the additional embodiments and implementations. For example, where a given feature or function is expressly described in connection with one embodiment or implementation but not expressly mentioned in connection with an alternative embodiment or implementation, it should be understood that that feature or function may be deployed, utilized or implemented in connection with the alternative embodiment or implementation unless the feature or function is incompatible with the alternative embodiment or implementation.

Aspects of the present disclosure are directed to systems and methods for creating and utilizing a decentralized medical device network to provide emergency medical care and caregiver guidance. The decentralized medical device network includes multiple medical and computing devices that constitute a patient-centric ecosystem because all of the devices are engaged in the provision of care for a particular patient. The devices in the patient ecosystem can communicatively couple via a decentralized network where each or most of the devices form a node in the decentralized network.

Decentralized network communications for a patient ecosystem of devices provides multiple benefits and advantages. In a decentralized network, no single device is critical to the continuity of data transfer as is true where communications flow through a central administrative device. In the case of a centralized system where one device acts as a central node through which the other devices communicate, if that one device needs to leave the system there is a gap in data transmission. If you locate the "brain" or central hub of a communication network with a particular device, either a computing device or a medical device, it may be the case that such a device would have to remain with the patient ecosystem in order for communications to continue. Such permanency is often impossible or impractical for devices deployed in an EMS incident. In accordance with embodiments illustrated herein, the decentralized network beneficially ensures that no single device is critical to the circulation of information necessary for providing patient treatments and interventions. Furthermore, the decentralized network beneficially protects against a single point of failure. This may be of particular importance in a medical device ecosystem for a patient where the continuity of care requires information from multiple sensors and/or therapies delivered with multiple devices.

For example, if a patient is being treated with a defibrillator and a ventilator, these devices may need to share information in order to provide coordinated care. In a centralized system, a tablet computer may form a central communications link between the defibrillator and the ventilator. The tablet computer may control the exchange of information and/or synchronization of care between the defibrillator and the ventilator. However, if the tablet computer is removed from the system (e.g., the owner of the tablet computer may have to leave the patient to attend to another patient, the tablet computer may fail operationally, etc.), then the coordination of care between the defibrillator and ventilator is lost. However, if the three devices form a network, then the tablet computer may initialize the coordinated care and then may leave the system without disruption of care because the ventilator and defibrillator can carry on as nodes in the network. Therefore, with the decentralized network a single device may not be critical to care in the same manner as with a centralized network where the central device cannot be removed without detrimentally affecting care. Thus with the decentralized network, therapy could be initiated on a device and if the communications fail the therapy may continue uninterrupted, separating the care from the needs of communications.

As other examples, in a mass casualty situation there may be one or a few medics treating multiple patients. These medics may have multiple therapy delivery devices, such as defibrillators or ventilators, so that each patient is associated with his/her own therapy delivery devices. However, there may be fewer medics than there are patients. As such, the monitoring and coordination of care by these devices may be controlled by a tablet that is carried by a medic and has to come in and out decentralized medical device networks associated with each of the patients as the medic moves around the casualty scene. Such a situation might also arise in critical care air transport where there are multiple patients, each with their own suite of therapy delivery devices, but only one medic with a tablet for monitoring and coordination. As a further example, in a hospital or at an emergency scene, a second crew of caregivers may arrive and need to communicatively join their devices with those already engaged in treatment without disruption.

For example, if a mobile device acts as a central node for the set of BLS devices, rather than as a member of a decentralized network, then when the caregiver associated with that mobile device needs to leave the scene and the patient ecosystem to attend to a different patient, there is a gap in data exchange as a new device is initiated as a central node. Additionally, the mobile device may need to transfer some or all of its accumulated data to the new central node or to a server from which the new central node may retrieve the data. In the decentralized network, the mobile device may exit the decentralized network and the patient ecosystem at any time and any devices that are configured or otherwise able to be associated with the decentralized network joining the patient ecosystem can receive needed information without impact from the exit. Furthermore, the decentralized network communication could be established in such a way that if aspects of the decentralized network fails, micro sub-networks could remain in communication delivering therapy such that care continues without the entire decentralized network failing.

As another example, a caregiver may need to run back and forth between the patient and a vehicle, or look around a patient's home for medications, or move around to observe other victims on scene. All of these circumstances may impact the ability of a single device like a mobile device associated with the caregiver to act as a central node. Importantly, many emergency rescue scenes or transports lack access to a remote server, either temporarily or for most or all of an EMS response. Thus, using a central node and/or a remote server as a conduit for information, while helpful at an institution such as a hospital, may be unavailable to EMS devices. The devices in an EMS environment are not positioned physically, in one place for any particular length of time, the Internet or cellular connectivity in any given patient environment is unknown, and the types of medical devices needed may change quickly and urgently in the first few minutes of care where the extent of a patient's injury is initially evaluated. Also, in the emergency environment, the EMS crews are limited in terms of the number of people available and therefore tasks assigned to each person may change quickly and frequently. The decentralized network offers fast, flexible, and specific data exchanges without requiring a server and even with frequent and unpredictable changes in the members of the decentralized network. The decentralized network readily supports ad hoc changes to the ecosystem of devices providing patient care.

Additionally, the decentralized network enables devices to share smaller and customized sets of information, potentially distributed over time. Devices do not need to transmit entire data files to a server, for example, for other devices to select desired subset. The data requested and received to in the decentralized network may be tailored to the immediate needs of the devices based on the medical status of the patient. In an implementation, the devices in the decentralized network communicate according to a publish/subscribe messaging paradigm such that the data received and consumed by a given node is tailored to the needs of that node. The server transfers impose a particular burden on data transfers in real-time as needed to stay current with the dynamically changing medical status of a patient. Medical devices and sensors have to almost continuously upload their data to a server for any receiving devices to have access to real-time updates.

For example, unlike a single transfer of information upon arrival of ALS, the transfer of information may be spread over a period of time as crews transition devices. This enables a transfer of information without an interruption or other impact on deployment of various devices. As each medical device is ready and introduced, it may receive information as needed. The information exchange is tailored to the needs of each medical and/or computing device through the publish and subscribe functions. Thus, devices do not need to burden or be hampered by bandwidth or other transmission channel limitations that may affect the transmission of a large file that contains all of the data gathered for a patient. The data provided to the decentralized network by devices like a pulse oximetry sensor is immediately available to devices joining the decentralized network enabling these devices to utilize this data for determining care without delays that may be introduced if a device had to access a server or connect with an individual device.

Each device forming a node of decentralized network joins the network according to a handshake protocol. This handshake protocol makes each device in the network aware of the presence of every other device in the network. Because the devices in the decentralized network are known to one another by virtue of the handshake protocol, the new devices joining the decentralized network immediately have visibility to the available data and to the ecosystem of care underway for the patient. In this manner, the medical and/or computing devices are able to function cooperatively and with less redundancy or conflict. Furthermore, because all of the devices are communicating within a single decentralized network, the time stamps for data are all synchronized to that same communications network. In accordance with embodiments illustrated herein, as a benefit of the decentralized network hierarchical ranking and selective data subscriptions, the data communications at the time of care may be limited to data necessary for care with a significant reduction or elimination of unnecessary information transmission. As a result, the time synchronization of data for consolidation into single case files where the data is associated with but not critical to care can be postponed until there is access to a cloud server or larger bandwidth communication network than at the point of care without a diminishment of accuracy in the record or in the efficacy of care.

As an advantage to medical device manufacturers, provisioning devices with the necessary communications drivers that enable decentralized network communications allows groups of devices to be configurable to fit situations with particular needs. For example, as discussed below, devices deployed in a military environment may be limited to a physically distributed collection of sensors that in the aggregate function as would a centralized patient-monitor device. The decentralized network enables these devices to operate as a unified system despite the physical distribution.

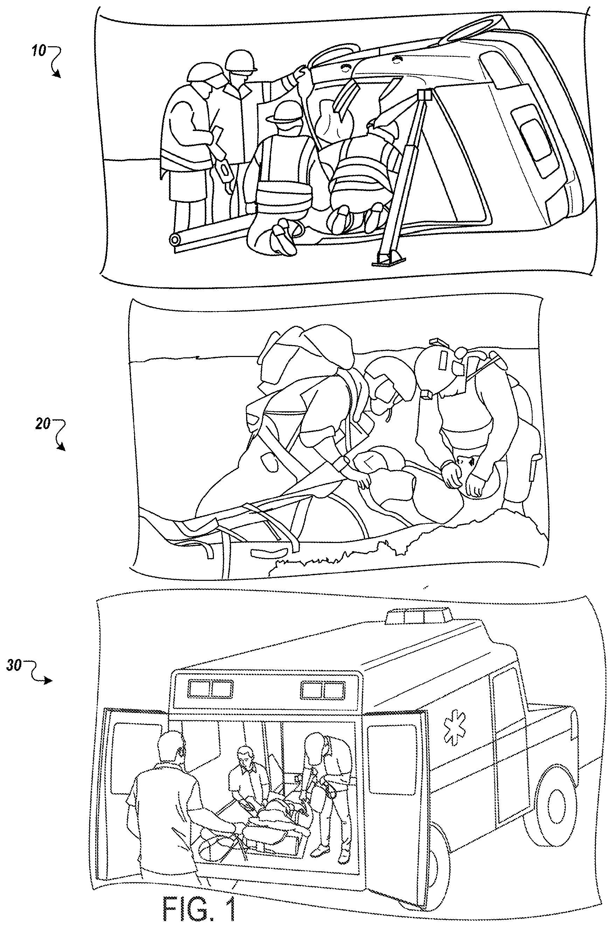

Referring to FIG. 1, examples of patient care environments in which a decentralized medical device network may be deployed are shown. Such an environment may include a rescue scene, a military scene, or a transport vehicle scene, to name a few examples. The rescue scene 10 may be a scene of a car crash or another on-site trauma rescue scene such as a sporting event field, a blast site, a scene of a fall, etc. The military medical scene 20 may be on or near a battlefield, in a bay of a field hospital, in a military triage area, etc. The transport vehicle scene 30 may be inside an ambulance, as shown, or in a helicopter or other transport vehicle. The patient care environment is not limited to the physical scenes shown and may also include a hospital, an urgent care clinic, a rural field hospital, an emergency medical tent, etc. In some physical locations, a lack of long-range network connectivity (e.g., connectivity to a computer network such as the Internet or a cellular communication network) may prevent access to remotely located caregivers and central computing systems such as cloud server. For example, a rural or military location, a parking garage, an interior location, or a moving vehicle may have little or no Internet or cellular network access or variable communication signal strength. In a military environment an absence of radio signals, often referred to as radio silence, is necessary for troop safety. Therefore, a decentralized medical device network needs to dynamically and adaptively function with or without long-range network connectivity.

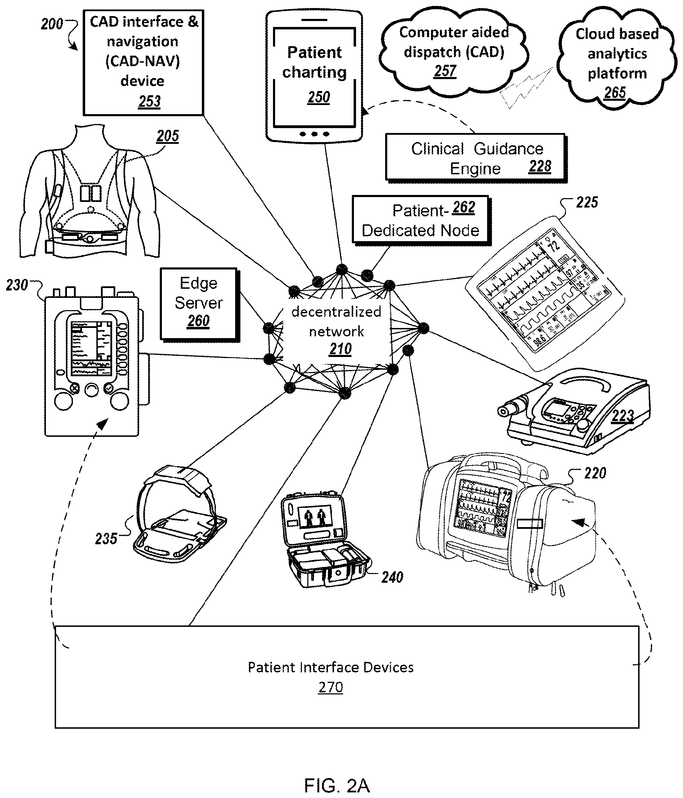

Referring to FIG. 2A, an example of medical devices and computing devices as decentralized network nodes is shown. A quantity of each component in FIG. 2A is an example only and other quantities of each, or any, component could be used. The system 200 includes the decentralized network 210 and a variety of potential medical devices and computing devices forming nodes of the decentralized network 210.

The decentralized network 210 is an ad hoc network that may include wireless communications (e.g., according to one or more wireless communications standards such as, for example, but not limited to, IEEE 802.11 commonly referred to as Wi-Fi or 2.4GHz wireless communications commonly referred to as Bluetooth^{®}), wired communications, or a combination thereof. The decentralized network 210 may be a local area network (LAN) without any communicative couplings to any remote computing devices and/or may be a wide area network (WAN) that includes a LAN communicatively coupled to one or more remote computing devices, for example a 4G or 5G protocol network. The decentralized network 210 described herein may be accomplished through a variety of network architectures. In various implementations, these architectures may include mesh networks and peer-to-peer networks. In some instances, the device communications described herein may be accomplished via multipoint Bluetooth^{®} communications. In various implementations, the devices in the decentralized network 210 may communicate according to a publish/subscribe messaging paradigm. The publish/subscribe messaging paradigm may utilize a distributed database, or ledger. For example, the distributed database, or ledger, may be a blockchain ledger. The blockchain ledger provides advantages in terms of cybersecurity and reliability, or fidelity, of medical information exchanged via the decentralized network 210. Thus, devices exchanging medical information via the network 210 can reliably trust that the information has not been corrupted during the communications process and can safely base treatment and intervention parameters on this information. Further, devices exchanging medical information via the network 210 may reliably trust that synchronization between devices has not been compromised to a degree that medical parameters, such as vital signs or other physiological indicators, forming the basis for clinical guidance are not obsolete or misrepresented as synchronous.

The decentralized network 210 may include two or more nodes where each node is associated with a medical device or a computing device. Depending on the types of devices forming the nodes, the decentralized network 210 may be a distributed network or a pseudo-distributed network. In the distributed network, all of the nodes may have both publish and subscribe capabilities. In the pseudo-distributed network, some of the nodes may only publish while others may publish and subscribe. For example, one or more of the patient interface devices 270 may only publish to the decentralized network 210 whereas one or more of the devices 260, 230, 235, 240, 220, 223, and 225 may both publish and subscribe. In general, simpler devices like physiological sensors may only publish whereas more sophisticated devices with more processing capability may both publish and subscribe. In a mesh network, for example, devices that publish only may be referred to as end nodes whereas devices that publish and subscribe may be referred to as mesh nodes.

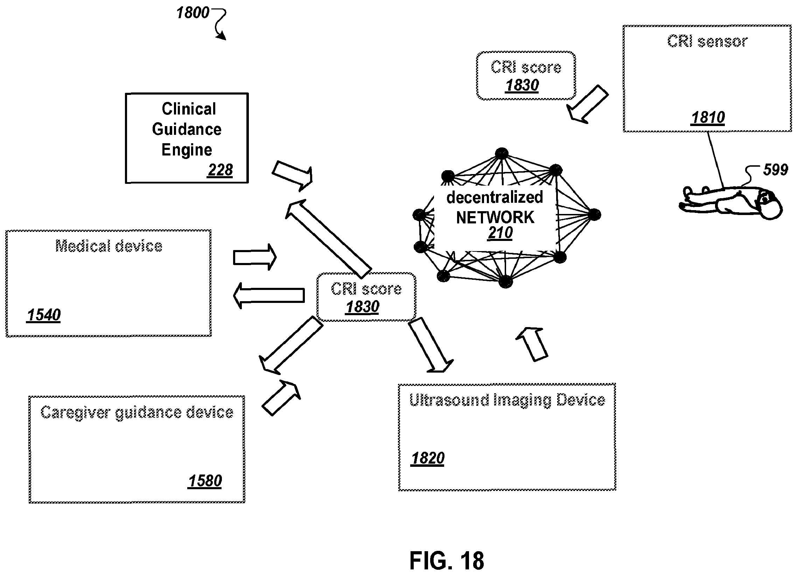

The medical devices in the decentralized network for a patient ecosystem of devices as all are employed in the care and treatment of a single patient. The medical devices in the decentralized network 210 are those employed by an EMS crew or military medic and may include external medical devices or insertable medical devices. In various implementations, the medical devices in the decentralized network 210 may include implanted medical devices or may exclude implanted medical devices. In an implementation, the decentralized network 210 may include a combination of external and implanted medical devices. The insertable medical devices are insertable into a patient on-scene by an EMS caregiver and a portion of the device is accessible from the outside of the patient's body (e.g., an invasive blood pressure device, an intravenous device, an intubation device, etc.). This is contrasted with an implanted medical device that is surgically implanted and only removable by a surgeon or other hospital personnel. The medical devices in the decentralized network 210 are mobile devices configured to be carried by hand, or in a backpack to the site of patient. The medical devices may be external wearable devices, either by caregivers or by patients and/or may include non-wearable devices, like a patient-monitor/defibrillator, designed to rest on a floor or a table but not supported by the patient's body. The medical device may be a therapy delivery device, a sensor device, a monitoring device, or a combination thereof. For example, the medical device forming a decentralized network node may be one or more external defibrillators (e.g., a patient-monitor/defibrillator 220, an automated external defibrillator (AED) 223, or a wearable cardio-defibrillator 205), a trauma kit 240, an automated compression device 235, or a portable ventilation device 230. The decentralized network nodes may also include a drug delivery device 272 or an ultrasound imaging device (e.g., the device 1820 as shown in FIG. 18).

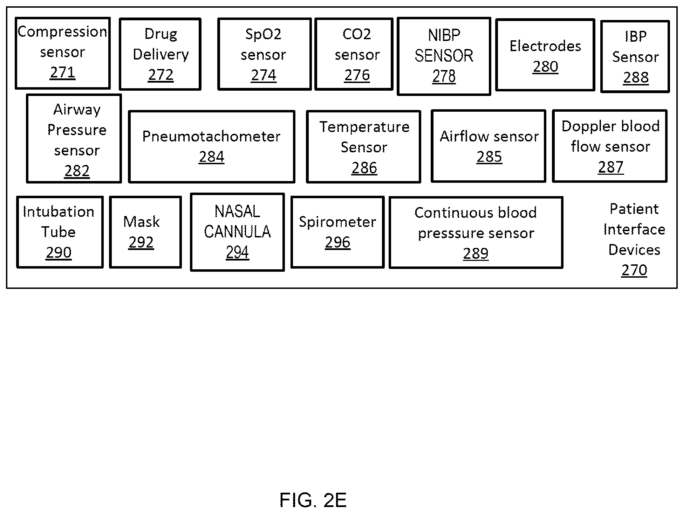

Referring to FIG. 2E. the patient interface devices 270 may include one or more devices that may form one or more respective nodes in the decentralized network 270. For example, the patient interface device forming a decentralized network node may be a compression sensor 271, a SpO2 sensor 274, a CO2 sensor 276, a non-invasive blood pressure (NIBP) sensor 278, electrodes 280 (e.g., sensing electrodes and/or therapy electrodes), an airway pressure sensor 282, a pneumotachometer 284, an airflow sensor 285, a temperature sensor 286, a Doppler blood flow sensor 287, an invasive blood pressure (IBP) sensor 288, a continuous blood pressure sensor 289, an intubation tube 290, a mask 292, a nasal cannula 294, or a spirometer 296. The decentralized network nodes may also include a drug delivery device 272 or an ultrasound imaging device (e.g., the device 1820 as shown in FIG. 18). The devices shown collectively in FIG. 2A as patient interface devices 270 may each form their own node in the decentralized network 210 as stand-alone devices or may be incorporated into a larger medical device that forms the node in the decentralized network 210. In an implementation, the medical devices may include a patient sensor hub configured couple to one or more physiological sensors and may be designed to travel with a patient to enable substantially continuous monitoring of the patient with the one or more physiological sensors. The patient sensor hub has its own processor, memory, and power supply and may removably attach to a larger item of medical equipment like a patient-monitor/defibrillator or portable ventilator. The patient sensor hub may couple to the physiological sensors and/or the larger item of medical equipment through a wired or wireless connection as well as forming a node in the decentralized network 210. The patient sensor hub may further include a display screen.

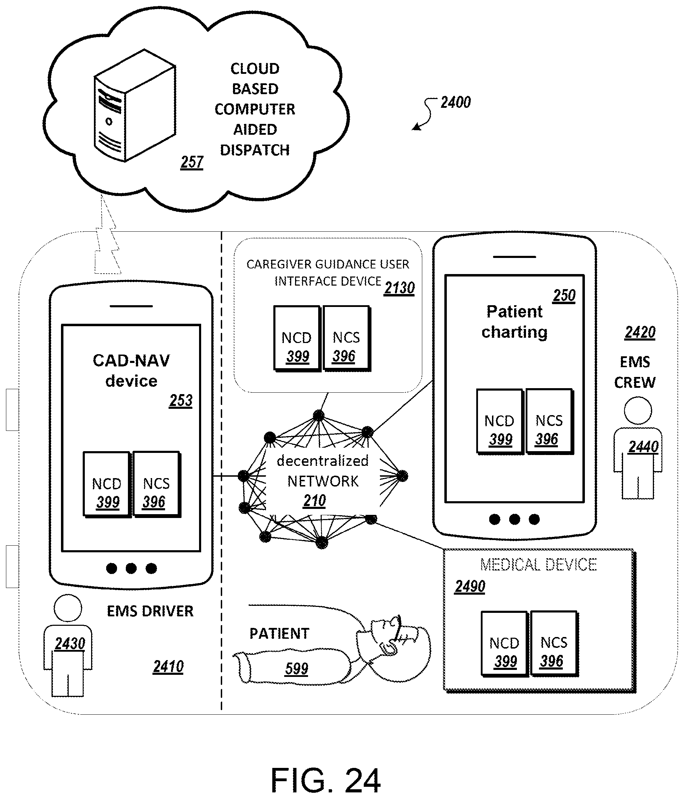

Referring again to FIG. 2A, the decentralized network nodes may further include computing devices, for example, a mobile computing devices such as a tablet or a smartphone. Each of these computing devices may provide one or more applications such as a patient charting application (e.g., on the patient charting device 250) or an in-vehicle computer aided dispatch (CAD) interface and navigation (CAD-NAV) application (e.g., on the CAD-NAV device 253). These applications are described in more detail below in regard to FIG. 24.

In an implementation, one or more of the decentralized network nodes may additionally provide a clinical guidance engine 228. The clinical guidance engine 228 may provide caregiver guidance upon dispatch, en route to a patient, at the scene of the patient, and during transport of the patient from the scene to a destination health care facility.

Caregiver guidance provided upon dispatch and/or en route to a patient may improve the care provided to the victim by optimizing a response prior to arrival and enabling immediate deployment of that response upon arrival. For example, the clinical guidance engine 228 may evaluate an inventory of the medical equipment available to the caregivers and determine if the inventory includes recommended medical equipment based on care protocols available to the clinical guidance engine 228. The clinical guidance engine 228 may provide instructions on preparing this equipment for deployment in the emergency environment and/or provide instruction for use. If the inventory does not include recommended medical equipment, then the clinical guidance engine 228 may adjust the protocol recommendation based on what item(s) are available or otherwise useful for carrying out the protocol recommendation and/or may provide a request to an emergency agency to deliver the recommended equipment to the emergency environment. As another example, the clinical guidance engine 228 may provide caregiver instructions to prepare for patient treatment by preparing equipment, reviewing equipment assembly and operational instructions, protocol instructions, etc.

At least one of the devices 225 and/or 250 may be a caregiver interface device and enable the caregiver to interact with the clinical guidance engine 228. For example, the devices 225 and/or 250 may enable the caregiver to provide input to the clinical guidance engine 228 such as spoken input or input entered at a touchscreen. The devices 225 and/or 250 may enable the caregiver to receive output from the clinical guidance engine 228 such as prompts, instructions, reminders, etc. as visible, audible, and/or tactile output.

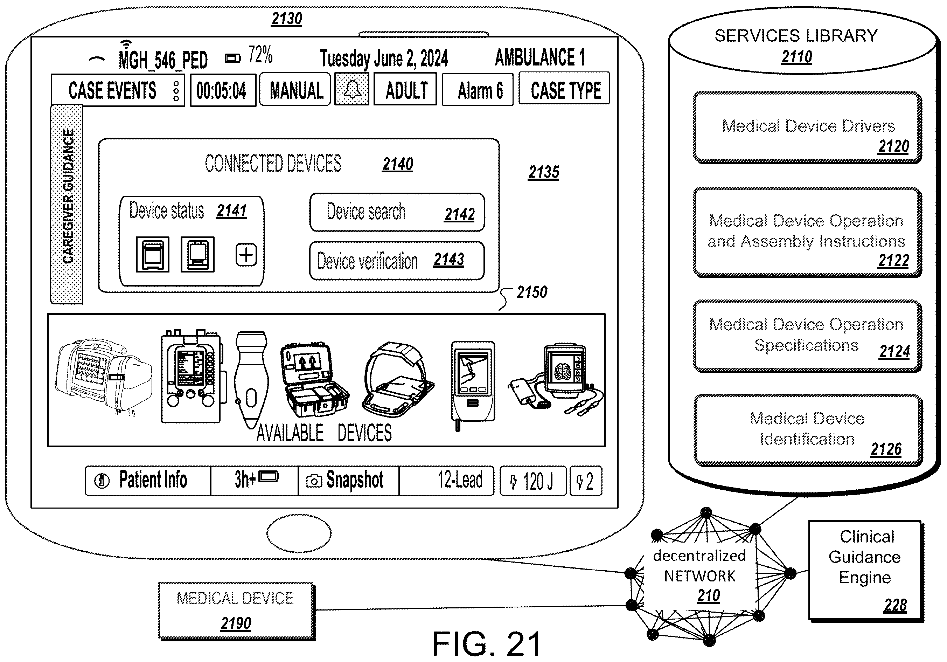

The patient device ecosystem may also include at least one edge server 260 that forms a node in the decentralized network 210, The edge server 260 may be a standalone computing device or may reside on another computing device or medical device. The edge server 260 may be configured to execute processor intensive operations that are sometimes involved when executing operations of the clinical guidance engine 228. Some implementations of the edge server 260 include, for example, one or more GPUs that are capable of efficiently executing matrix operations and substantial cache or other high-speed memory to service the GPUs. In some examples, the edge server 260 is a separate, ruggedized physical device that travels with EMS personnel in the field. In some examples, the edge server 260 is incorporated into other EMS field equipment such as a medical device and/or may be located in the EMS vehicle and/or may be located within a carrying case for a medical device. In an implementation, the edge server 260 may have a relatively small physical profile and/or be incorporated into another portable device such as a portable medical device or mobile computing device. In some implementations, the edge server 260 may be a rack server. The rack server may be mounted in a cabinet that may be portable, a transport vehicle such as an ambulance. The rack server may be a scalable computing platform. For example, individual server units may have a footprint of less than one square meter and multiple units may be combined to increase computing capacity. In an implementation, the computing device 250, 225, and/or 253 may provide the edge server if the processing capability of this device is sufficient to provide computing services associated with an external edge server. The edge server moves more computing capability into the local environment so that computation intensive clinical guidance can run accurately and efficiently even in the absence of a connection with the remote cloud-based analytics platform 265. In an implementation, the edge server 260 may include the clinical guidance engine 228 and/or the services library 2110 (as shown in FIG. 21). The edge server 260 may enable the clinical guidance engine 228 to run without any cloud connection by providing more substantial processing and computational resources than those available on the mobile devices 225 and/or 250 and/or the medical equipment. These resources may be necessary for the functioning of machine learning and artificial intelligence models used by the clinical guidance engine 228 to determine and guide patient treatment. These models may also enable the use of natural language processing and computer vision for automated data entry and information exchange. The edge server 260 may monitor for communication connections with a cloud platform 265 and may connect and synchronize with this server when the communication connection is available. The edge server 260 may also provide some security functions as a computing entity that sits in between the mobile devices 225 and/or 250 and the cloud platform 265. Where remote server connections are available, the edge server 260 may serve as an access device to the cloud platform 265 to enable remote providers (e.g., hospital staff, physicians, telemedicine providers, and teleguidance providers.

In an implementation, the network 210 may include a compact and portable computing device furnished with significant storage (e.g., at least ten gigabytes of memory) that may serve as a patient-dedicated node. For example, this portable computing device may be a Raspberry Pi^{®} or Arduino^{®} computer or another similar device. Unlike some of the other nodes in the decentralized network, in an implementation the patient-dedicated node may remain with a single and same patient irrespective of transfers of the patient between medical personnel teams, medical transport vehicles, and/or medical facilities or sites. Such a patient-dedicated node may host a patient ledger that may include medical information (for example, one or more of clinical information, patient history, care record history) and device synchronization information (for example, message timing, device identifiers, etc.) that enables post-case reconstruction of a care record that includes information from all of the medical and/or computing devices that participated in the decentralized network. The patient-dedicated node may be a non-medical device as it may not be associated with any monitoring or therapy functions.

Optionally, any or all of the nodes in the decentralized network may communicatively couple to a remote cloud-based server providing, for example, a cloud-based analytics platform 265 or cloud-based computer aided dispatch (CAD) 257. However, some applications, a cloud connection is either unavailable (e.g., in a rural area, an urban canyon, a parking garage, etc.) or undesirable or disallowed (e.g., in a radio-silence military setting). Therefore, the decentralized network 210 may form without any nodes having a communicative coupling to the Internet or other remote server or long-range computer or cellular network.

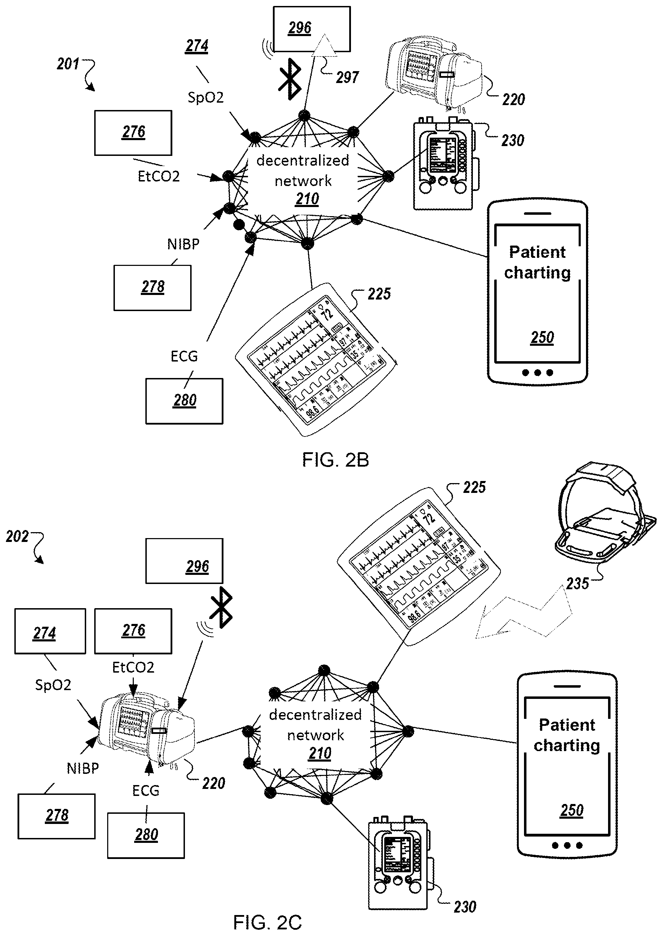

Referring to FIGS. 2B and 2C, examples of decentralized network nodes specific to a particular patient condition are shown. A quantity of each component in FIG. 2B and FIG. 2C is an example only and other quantities of each, or any, component could be used.

The collection of devices shown in FIGS. 2B and 2C is a subset of those shown in FIG. 2A. The systems 201 and 202 represent examples of devices that may be used by EMS to treat a victim of respiratory distress (RD). As discussed herein, the decentralized network 210 enables an exchange of information between these devices that is centered on a single patient or victim being treated with the devices shown. The devices forming the decentralized network 210 may be particular to a patient condition or treatment and may change dynamically as the patient condition or treatment changes and evolves. For example, as illustrated in FIG. 2C, if the patient being treated for RD goes into cardiac arrest, the EMS crew may introduce an automated compression device and this device may join the decentralized network 210 as a node.

In an implementation, the connections to the decentralized network 210 may be individual as illustrated in FIG. 2B where each device forms its own node in the decentralized network 210. Alternatively, the connections to the decentralized network 210 may be combined. For example, the sensors 274, 276, 278, and 280 may be coupled to a patient-monitor/defibrillator 220 via wired or wireless connections. The patient-monitor/defibrillator may then form a node on the decentralized network 210 and provide patient data gathered from the patient interface devices (e.g., the sensors 274, 276, 278, and 280). Devices, such as the spirometer 296, that are configured to communicate via a short-range wireless coupling, such as Bluetooth^{®}, may form their own node on the decentralized network 210 as shown in FIG. 2B or may send their signal to another device in the decentralized network 210 (e.g., a medical device or a computing device) via the short range wireless connection and then that device may form a node on the decentralized network 210. For example, as shown in FIG. 2C, the spirometer 296 may send its data to the patient-monitor/defibrillator 220 which in tum may form a node on the decentralized network 210. In an embodiment, a device configured for short-range wireless communications, like Bluetooth^{®} may be further configured to couple to a dongle 297. The dongle may enable the device to function as a stand-alone node in the decentralized network 210.

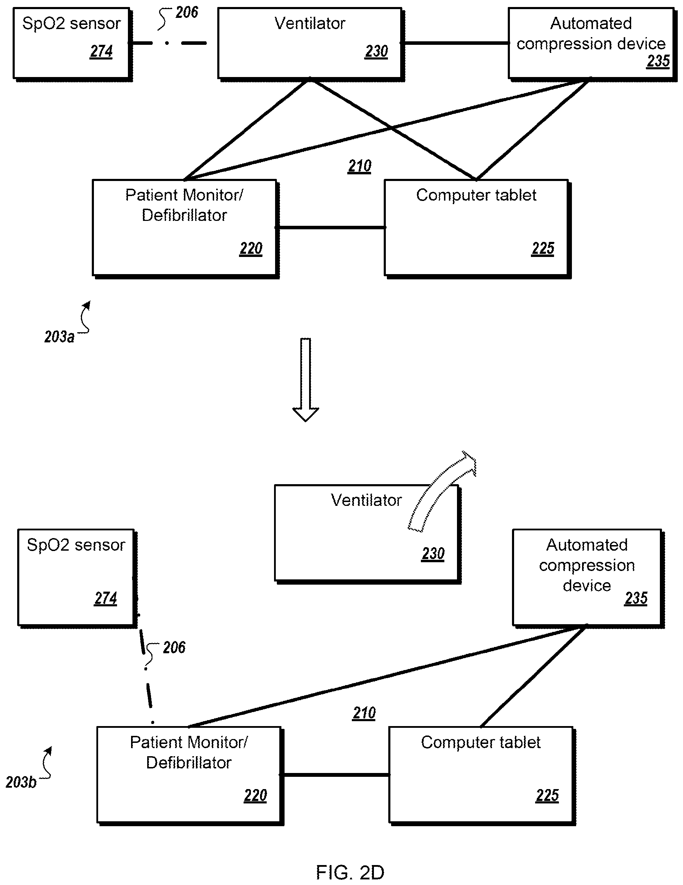

Referring to FIG. 2D, an example of various node capabilities within a decentralized network is shown. A quantity of each component in FIG. 2D is an example only and other quantities of each, or any, component could be used.

In configuration 203a, the decentralized network 210 includes a ventilator 230, an automated compression device 235, a patient-monitor/defibrillator 220, and a computer tablet 225. For example, these devices may be coupled in a mesh network topology. The SpO2 sensor 274 may be lack the capability to join the decentralized network 210 and may only be able to couple to one device at a time, for example, by a Bluetooth^{®} connection 206. However, because the other devices 230, 235, 220, 225 are networked, the existence of the SpO2 sensor may be broadcast to the network by the individual device that the SpO2 sensor is connected to. Thus, for example, the ventilator 230 may include the existence of the SpO2 sensor, the connection of the SpO2 to a patient, and its device capabilities in a network message provided to the decentralized network 210 by the ventilator 230. As a result if the ventilator needs to leave the network 210 (e.g., because the ventilator is no longer needed for patient care), as shown schematically in configuration 203b, then one of the other devices in the network 210 will already have the requisite knowledge of the SpO2 sensor and can transfer the connection 206. For example, the patient-monitor/defibrillator 220 can connect to the SpO2 sensor and the ventilator 230 may exit. If the one or more of the other devices in the network, such as the automated compression device 235 and/or the computer tablet 225 was receiving data from the SpO2 sensor 274 via the ventilator 230 and the network 210, the transfer of the connection 206 to the patient-monitor/defibrillator 220 may provide uninterrupted access to the SpO2 sensor data for the entire network 210.

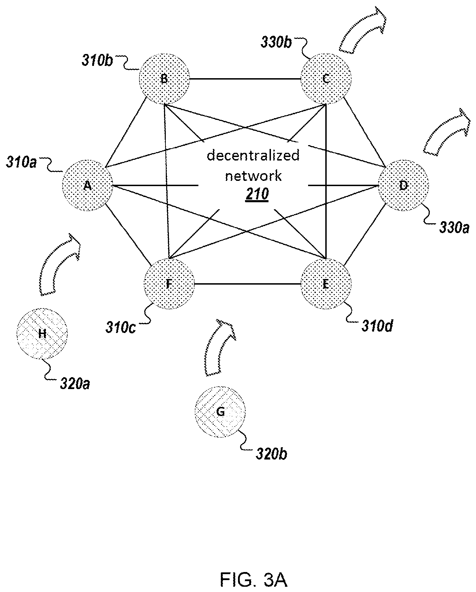

Referring to FIG. 3A, an example of nodes exiting and entering a decentralized network is shown. A quantity of each component in FIG. 3A is an example only and other quantities of each, or any, component could be used.

As noted in regard to the automated compression device in FIG. 2C, devices may enter and exit the decentralized network 210 as they enter and exit the ecosystem of devices providing treatment and care for a single patient. This may occur dynamically due to changes in the patient condition and/or patient treatment as well as due to changes in personnel treating a patient. As discussed in examples below, personnel changes are inherent during transitions from basic life support (BLS) crews to advanced life support (ALS) crews and also during transitions from EMS to hospital. Transitions may also occur in military settings where, for example, a particular medic may need to treat multiple casualties or be injured and unable to provide care. Computing devices and medical devices may be associated with a particular crew or medic and the crew or medic may need to take those devices away from a patient when the crew or medic discontinues their care for a patient. However, the patient stays constant. Therefore, entering and exiting the decentralized network 210 enables a seamless exchange of information as devices necessarily exit and enter the care scene and the patient ecosystem.

As illustrated in FIG. 3A, the decentralized network 210 may start with the nodes 310a, 310b, 310c, 310c, 310d, 330a, and 330b. At some point in time, the nodes 330a and 330b may exit the decentralized network 210 and the nodes 320a and 320b may join the decentralized network 210.

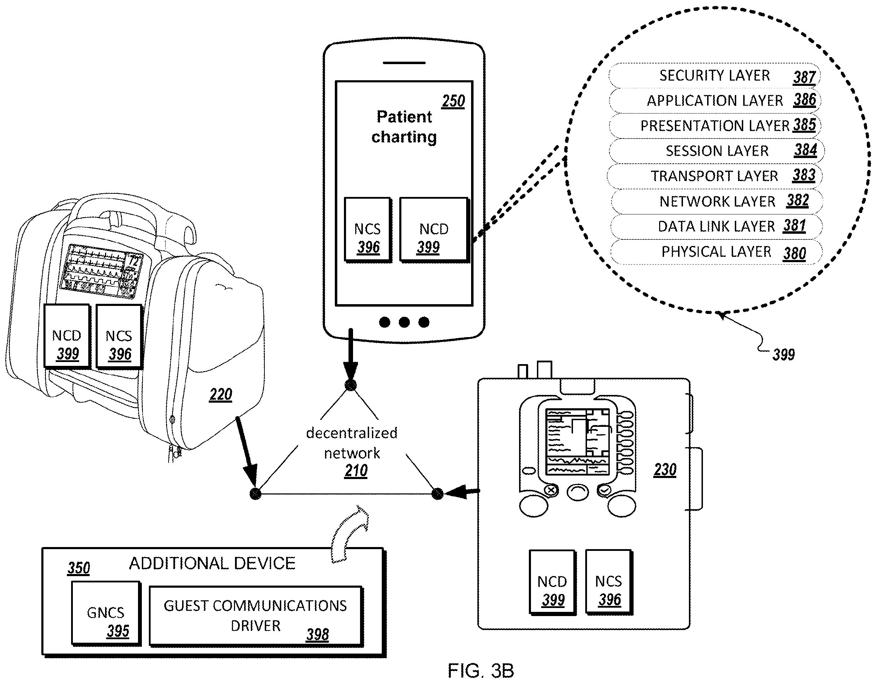

Referring to FIG. 3B, an example of nodes participating in a decentralized network as enabled by a network communication driver is shown. A quantity of each component in FIG. 3B is an example only and other quantities of each, or any, component could be used. Each of the devices forming a node in the decentralized network 210 may be provisioned with a network communications driver (NCD) 399. Thus each of the devices forming a node in the decentralized network 210 may serve as an NCD host device. In the example shown in FIG. 3B, the patient charting device 250, the portable ventilator 230, and the patient-monitor/defibrillator 220 are all provisioned with the network communications driver 399.

The network communications driver 399 may include a network interconnection protocol stack, for example, according to an Open Systems Interconnection (OSI) model. The network interconnection protocol stack may include, for example, multiple layers such as a physical layer 380 including the radio hardware, a data link layer 381, a network layer 382, a transport layer 383, a session layer 384, a presentation layer 385, an application layer 386, and a security layer 387. Some devices may have more complex stacks than that shown in FIG. 3B. Each of the devices forming a node in the decentralized network may additionally be provisioned with network communications software (NCS) 396. The NCS 396 may communicate with the NCD 399, for example with the application layer 386, to manage bidirectional communications and information transfer between software and applications residing on the NCD host device and the NCD 399. The NCS 396 may provide information received via the decentralized network 210 to application(s) on the NCD host devices as described herein such as, but not limited to, a patient charting application, a computer-aided dispatch application, a navigation application, a medical device data storage application (e.g., to generate a medical device case file), medical device control software (e.g., to control medical device parameters and settings for monitoring and/or delivering therapy to a patient),a closed loop control application, a caregiver guidance, clinical guidance, and/or clinical decision support application, a patient evaluation scoring application, telemedicine and/or remote monitoring applications, etc. Additionally, the NCS 396 may receive information from one or more of these applications and/or may receive information via a user interface on the host device. In this way, the various devices and applications described herein may incorporate, utilize and/or store information received via the decentralized network 210 and/or may select information to provide to other devices in the decentralized network 210.

In order to participate in the decentralized network 210, the medical devices and computing devices are provisioned with at least one substantially similar or identical layer. In an example, each of the medical devices and computing devices has a same data link layer 381, network layer 382, transport layer 383, session layer 384, and security layer 387 as each other of the medical devices and computing devices. In an example, at least one of a data link layer 381, network layer 382, transport layer 383, session layer 384, or security layer 387 is a same layer for all of the medical devices and computing devices. For example, a medical device manufacturer may provision devices with the hardware and/or software that make up the network communications driver 399 to provide network compatibility and interoperability between nodes on the decentralized network 210. The publish and subscribe functions described herein may be functions of the application layer 386. At least the application layer 386 may vary between devices to tailor the particular the publish and subscribe paradigm to each device. These layers diversify the capabilities of various devices so that some publish, some subscribe, some publish and subscribe, and devices may be able to select between these capabilities. For example, for some devices the application layer 386 may limit these devices to publish only. In a mesh network, the publish only devices function as end nodes. For other devices, the application layer 386 may enable publish and subscribe capabilities. In a mesh network, these devices will function as mesh nodes. The security layer 387 may enable and ensure data encryption so that only allowed devices are able to publish/subscribe to the network 210, the devices subscribed to the network 210 are all associated with a same patient, and/or cybersecurity protections and protocols are enforced and provided at all active nodes of the network 210.

As another example, a mobile device or other computing device may interoperate with the medical devices in the decentralized network 210 via a software development kit (SDK) provided by the medical device manufacturer. The SDK may provide at least an application layer that enables these devices to subscribe to and read data from the medical devices provisioned with the network communications driver 399. The SDK may enable a translation mapping of the communication protocol of the computing device to that of the decentralized network 210. As a further example, the medical device manufacturer that provides the network communications driver 399 may provide a portion of the driver 399 as a secure kiosk application for the mobile or other computing devices.

Additional device(s) 350 attempting to join the decentralized network may be provisioned with the network communications driver 399. In an implementation, the additional device(s) 350 may be from a different medical device manufacturer. In this case, one of the devices already in the decentralized network 210 may be provisioned with a guest network communications driver 398 or may retrieve guest network communications driver 398 from a cloud server. The additional device(s) may additionally be provisioned with guest network communications software (GNCS) 395. The GNCS 395 may communicate with the guest network communications driver 398, for example with an application layer of the driver 398, to manage bidirectional communications and information transfer between software and applications residing on the additional device(s) and the guest network communications driver 398. The guest network communications driver 398, which may interoperate with the decentralized network 210 via an SDK, may enable variable levels of interoperability between medical devices from disparate manufacturers. The devices from the various manufacturers may have a common and compatible physical layer, configured for communications via a Bluetooth^{®} protocol, an IEEE 802.11 protocol, or a more specific network standard for the decentralized medical device network 210 described herein. However, the application layer 386 may distinguish devices from different manufacturers from one another and enable control of information exchange based on the manufacturer of the device.

In an implementation, the additional device(s) 350 may implement the application layer as a licensed application layer from another medical device manufacturer in order to provide the additional device(s) 350 with the ability to function as publish and subscribe nodes. In an implementation, the additional device(s) 350 with a guest network communications driver 398 may only subscribe, or only publish, have diminished publish and/or subscribe capabilities as compared to the devices with the driver 399 or only be known to the decentralized network 210 as being part of patient care but be unavailable for data exchange. In an implementation, the guest network communications driver 398 may enable the medical device associated with the second medical device manufacturer to participate in the decentralized network as a data supplier and disallow participation as a data consumer.

Unlike a point-to-point transmission network, the decentralized network 210 is an ad hoc, self-configuring, and self-healing network. Each node can publish data packets to the decentralized network 210 and some nodes can subscribe and receive data from the decentralized network 210. The presentation and application layers may differ, for example, between a more intelligent device like a patient-monitor/defibrillator and a more passive device like a SpO2 sensor. Thus, the patient ecosystem medical and/or computing devices in the decentralized network 210 may provide different degrees of data exchange capability.

In a mesh network, a node also functions as a router and can relay messages for its neighbors. Through the relaying process, a packet of wireless data will find its way to its destination, passing through intermediate nodes with reliable communication links. The mesh topology may enhance the overall reliability of the network by offering multiple redundant communications paths throughout the network. In various implementations, the decentralized network 210 may be mesh network with a partial mesh network topology or a full mesh network topology.

In a mesh network, if one link fails for any reason (including the introduction of strong RF interference), the network automatically routes messages through alternate paths. The reach of a decentralized network may be extended, redundancy added, and general reliability improved simply by adding more nodes. The decentralized network 210 described herein does not require a system administrator or central authority to tell it how to get a message to its destination or to track the participants in the network. Additionally, because the decentralized network 210 is self-organizing it does not require manual configuration. Because of this, adding new devices to the decentralized network 210 may be achieved simply by powering on the devices. Furthermore, a decentralized network 210 is self-healing because human intervention is not necessary for re-routing of messages. Because the operation of decentralized network does not depend on a central control point, it is convenient to add multiple data collection points or gateways.

In a mesh network implementation, the degree of redundancy is essentially a function of node density. A mesh network is scalable and can be deliberately over-designed for reliability simply by adding extra nodes, so each device has two or more paths for sending data. This may be a simpler way of obtaining redundancy than is possible in other types of systems. Further, having multiple data transport paths available to a device may enable that device to select between the paths based on communication connection parameters such as bandwidth (e.g., capacity and/or transfer rate) and reliability of connectivity. As examples, a device may choose a higher bandwidth path based on an amount of data or time-sensitivity of data or may choose a wired path over a wireless path to leverage a higher reliability of connectivity for high priority data or time sensitive data.

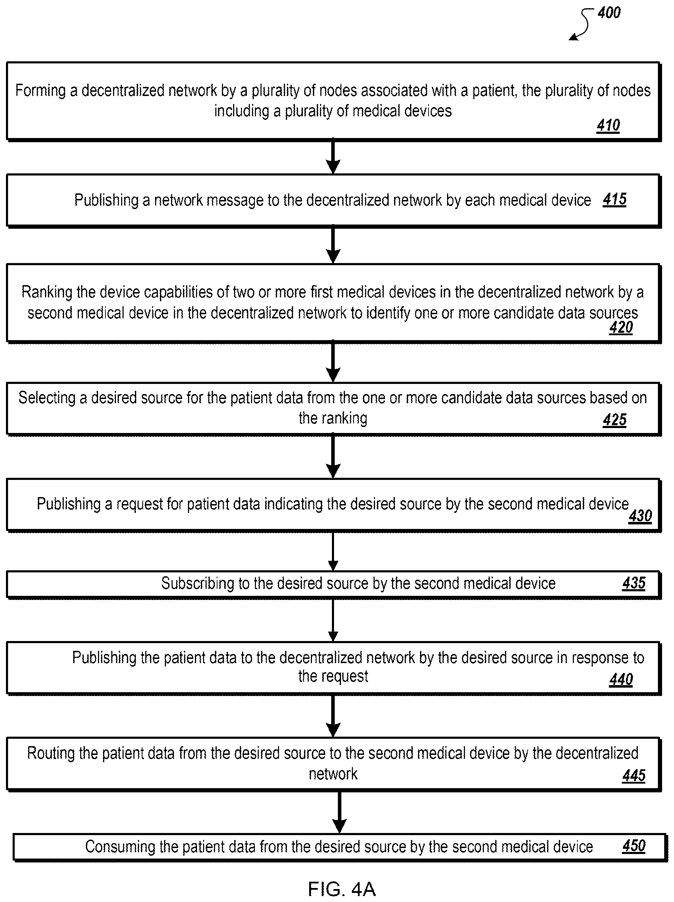

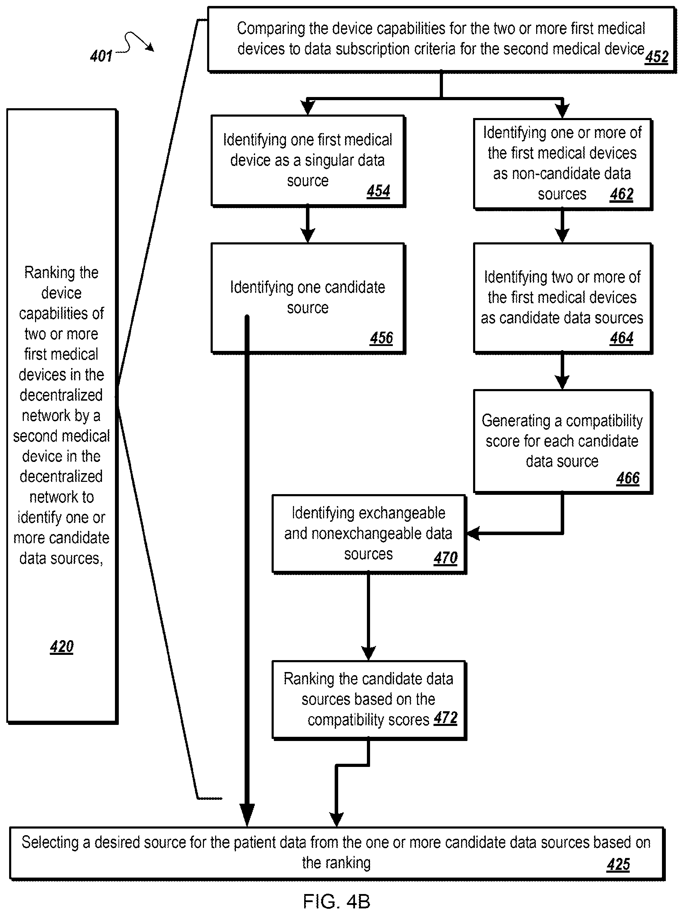

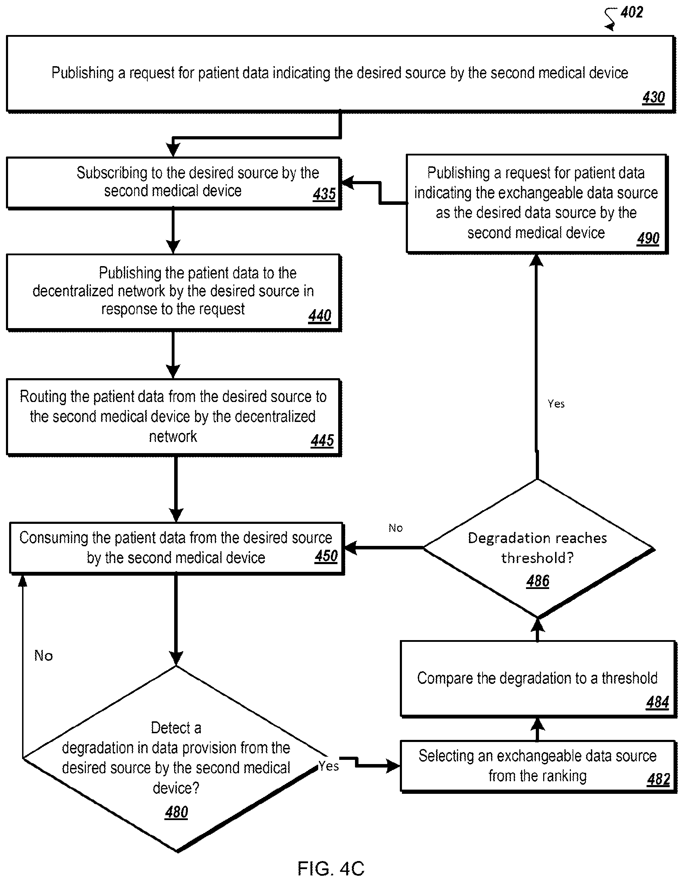

Referring to FIG. 4, an example of a method of hierarchically ranking sources of patient data for consumption by medical devices in a decentralized network is shown. For example, at least one of the NCD 399 or the NCS 396 of the medical devices in the decentralized network may perform one or more stages of the method 400. The method 400 is, however, an example only and not limiting. The method 400 can be altered, e.g., by having stages added, removed, rearranged, combined, and/or performed concurrently.

A networked group of medical devices used to treat a patient may include multiple source nodes for a particular data type and/or multiple consumer nodes for that data type. As an example of multiple source nodes, in the systems 201 and 202 (as shown in FIGS. 2B and 2C), both the patient-monitor/defibrillator 220 and the portable ventilator 230 may provide capnography sensors. Each of these sensor sources may generate capnography data for the patient. However, between these sources there may be differences for the collected capnography data with regard to the signal quality, the signal strength, the signal resolution, the sampling frequency, etc. along with differences in the transmission characteristics for transmission of this data to other devices. Additionally, the capnography data may be used for different purposes by different data consumers. For instance, the patient-monitor/defibrillator 220 may use this data to monitor the patient's respiratory state, the caregiver guidance application on the tablet 225 may use this data to recommend treatment or determine a diagnosis, the patient charting application on the patient charting device 250 may record this data, and the portable ventilator 230 may use capnography data for closed loop control. The various sources of capnography data may be better suited for one or the other of these consumption needs. As another example, pulse rate may be available from one or more of a pulse oximetry sensor, an invasive blood pressure sensor, or a non-invasive blood pressure sensor and heart rate may be available from electrocardiogram (ECG) data. As with the capnography example, the various consumers of heart rate or pulse rate data in the decentralized network 210 may have different needs in terms of data characteristics. In an implementation, a medical and/or computing device consuming heart rate data may subscribe to the pulse rate data and use this data interchangeably with the heart rate if the ECG data is unavailable or unreliable. However, once the ECG data is available reliably, then the medical device may subscribe to the ECG heart rate data as a more direct measurement and unsubscribe from the pulse rate data. As yet another example, medical and/or computing devices in the decentralized network 210 in need of respiration rate data may select amongst EtCO2 data, ECG data, impedance data, ventilator data, filtered SpO2 data, and flow sensor data in making a data subscription selection.

Each consumer node may sort, select, and/or rank sources nodes based on the data needs for the consumer node and the characteristics of the source node. For instance, a node may evaluate and determine a preference for a type of communication (e.g., Bluetooth^{®}, wired, wireless, Wi-Fi, etc.), for a communication specification such as data rates, for a sampling rate by the source node, for a bandwidth for the source node, consumer node, and/or the network, for a source operationally connected to the patient (e.g., as opposed to a source that is designed to generate a particular type of data but is not operationally active), for a local source versus a networked source, etc. The local source may be a sensor incorporated into a node. For example, a patient-monitor/defibrillator may have an integrated heart rate sensor and there may also be a heart rate sensor that forms another node that makes data available to the patient-monitor/defibrillator through the network.

In determining a preferred source node, a consumer node may not choose to subscribe to the one that optimizes every aspect of the ranking or sorting. Alternatively, a consumer node may optimize towards one aspect or a subset. For instance, if a particular consumer node needs SpO2 data every minute it may not subscribe to a high data rate source node in order to reduce the impact on messaging bandwidth. Alternatively, the consumer node may subscribe to the high data rate source but only request a data point from that subscription periodically rather than continuously. Additionally, the sources and consumers of data typically change over the course of patient treatment. Therefore, it is necessary for devices to dynamically evaluate and select a source of a particular type of data. The method 400 provides an example of such a dynamic process for the transfer and selection of data by nodes in the decentralized network 210.

Redundant sources of same or similar data and the ability to compare data sources may enable a consumer node to use these redundant sources to cross-check signal quality. Returning to the example of pulse rate from a pulse oximeter and heart rate from an ECG monitor, a consumer node may confirm that a pulse rate provided by a pulse oximetry sensor is accurate by comparing the pulse rate to the heart rate from the ECG monitor. ECG is a more direct and reliable source of heart rate data than the pulse measured by a pulse oximeter however in some instances it may be preferable for the consumer node to receive the pulse rate from a pulse oximeter. For example, the pulse oximeter may provide a wired connection, or it may be easier to keep the pulse oximeter connected to the patient during transport or the ECG sensors may be connected to a medical device critically needed for another patient. However, with the two sources available, the consumer node may verify that the pulse rate is a same rate as the heart rate from the ECG monitor before selecting to the pulse oximeter as the preferred source of data.

As yet a further example of a benefit of redundancy, multiple subscribers needing a same or similar parameter may benefit from having a choice of source. For example, for closed loop control, a ventilation system may prioritize and select a respiratory rate available from a first source whereas a patient-monitor may prioritize and select a respiratory rate available from a second source for patient-monitor algorithms. These priorities may be determined, for example, by differences in schema between the first and second sources. However, the preference may only be a priority if the data source is reliable. Thus the subscriber can balance a preference for a particular data source against the reliability of the data source based on a comparison of data from the candidate sources. Where the data from the candidate sources should be the same or approximately the same, the subscriber can evaluate these sources to verify that that data is the same or approximately the same and not divergent. This comparison may gate the prioritization. In the example above, if the patient-monitor prefers the second source based on schema, it may subscribe to the second source if the data from the second source substantially matches the data from the first source but subscribe to the first source if the second source diverges.

At the stage 410, the method includes forming a decentralized network by a plurality of decentralized network nodes associated with the patient. For example, two or more of the medical devices shown in FIG. 2A may form the nodes of the decentralized network 210. In some examples, computing devices also shown in FIG. 2A may form the nodes of the decentralized network 210 or a mix of computing devices and medical devices may form the nodes of the decentralized network 210. As discussed above with regard to FIG. 3B, each medical device includes a communications interface comprising a signal transmitter and a network communications driver. The signal transmitter provides the physical layer of the network communications driver. The signal transmitter may be a radio transmitter for wireless communications and/or a signal transmitter configured for wired transmission (e.g., twisted pair cable, Ethernet cable, coaxial cable, optical fiber, radio frequency, etc.). The medical devices in the decentralized network either constitute a patient interface device (e.g., as a standalone sensor) or include a patient interface device (e.g., a sensor couple to the medical device). The patient interface devices gather patient data from the patient for use in monitoring and treating the patient. The medical devices also include a processor, memory, and associated circuitry.

At the stage 415, the method includes publishing a network message to the decentralized network by each medical device. For example, each medical device forming a node in the decentralized network 210 publishes a network message that indicates its own device capabilities. The device capabilities may include the one or more data types that are collected by the medical device, a data sampling frequency, a data resolution associated with each of the one or more types of data, and/or data communications characteristics like signal strength, data formatting, data encryption, transmission protocol, an indication of a wired communicative coupling, an indication of a wireless communicative coupling, etc. The network communications driver 399 is configured to publish the network message as a device discovery message upon entry into the decentralized network 210. The network message is published as part of any decentralized network entry handshake. The network message may indicate the types of patient data available from a respective medical device. In an implementation, the network message may be data structures or one or more blockchain ledger entries. The network message may include metadata that every member of the decentralized network 210 may receive. Each receiving device configured for subscription capability will then determine a data subscription to a particular node based on the network message published by that node. Each receiving device configured for publish only will have knowledge of the published network message and therefore will be aware of and have stored information about the publishing device as a member of the decentralized network 210. If a medical device is a single parameter sensor, then there will be one type of patient data available. For example, as shown in FIG. 2B, a blood pressure sensor 278 may provide blood pressure data, a pulse oximetry sensor 274 can provide SpO2 data, a carbon dioxide sensor 276 can provide capnography data, and electrodes 280 can provide ECG data, etc. If a medical device is a multi-parameter sensor or if the medical device supports multiple connected sensors, then there will be multiple types of patient data available. For example, as shown in FIG. 2C, the patient-monitor/defibrillator 220 may be connected to multiple patient interface devices including a pulse oximetry sensor, a carbon dioxide sensor, electrodes, and blood pressure sensor. In this case, the patient-monitor/defibrillator 220 may receive data from these sensors. The network message from the patient-monitor/defibrillator may indicate the ability to provide all of these types of sensor data along with parameters that the patient-monitor/defibrillator may derive from the sensor data. For example, the patient-monitor/defibrillator may receive ECG data from electrodes and determine a heart rate. The patient-monitor/defibrillator may then make both the ECG data and the heart rate data available to a subscriber. In an implementation, computing devices in the decentralized network may also publish network messages indicating device capabilities based the types of data entered into, received at, and/or stored at the various computing devices and/or data sources available to the computing devices.

In addition to data types, the network message may indicate various medical device capabilities such as a therapy delivery capability, a monitoring capability, an imaging capability, a sensor attachment capability, a mode of operation, battery life, etc. In some examples, the network message may also indicate a make, model or other identification information for the medical device. In some examples the capabilities may include a method of computation (e.g., an algorithm identification and/or assessment), an ability to scan a bar code and/or a QR code, an ability to parse text, and/or other computational or processing capabilities. The network message may further include the device capabilities such as data sampling frequency or a data resolution associated with each of the one or more types of data and/or transmission characteristics like signal strength, data formatting, data encryption, transmission protocol, an indication of a wired communicative coupling, an indication of a wireless communicative coupling, etc. In some implementations, a first device, such as a sensor, may couple via a wired coupling to a second device, such as another sensor and/or a more complex device (e.g., one of the devices 205, 230, 235, 240, 220, 223, 225) and the more complex device may provide the sensor data to the decentralized network 210. In various implementations, data format for data from a particular medical device may be formatted according to a Health Level Seven (HL7) standard or Fast Healthcare Interoperability Resource (FHIR) standard as indicated in the network message. The network message may further include a routing protocol for each device. In an implementation, the routing protocol may be the same for two or more devices or may be a global network characteristic with a same routing protocol for all the devices in the decentralized network 210. In various implementations, the network message may indicate one or more of the capabilities described herein for a non-medical computing device.

At the stage 420, the method includes ranking the device capabilities of two or more first medical devices in the decentralized network by a second medical device in the decentralized network to identify one or more candidate data sources. For example, the NCS 396 of at least one of the medical devices shown in FIG. 2A may rank the device capabilities of two or more other medical devices in FIG. 2A in the decentralized network 210. The two or more other medical devices may be two or more first medical devices that publish their network messages and the at least one medical device may be a second medical device that receives these network messages. The second medical device may rank based on its own data needs and the published capabilities of the sources. In an implementation, a medical device or a computing device may rank one or more computing devices as sources or a combination of computing devices and medical devices.

In an implementation, the second medical device may receive network messages from the other medical devices in the network and identify one or more first medical devices as candidate sources for a desired type of patient data. If there is only one source of a particular data type, then the second medical device may identify only one candidate device, rank it as the first choice of only one choice, and subscribe to that medical device. However, if the second medical device identifies more than one source for the desired data type, then the second medical device may rank these identified sources in order to select a desired source.

Referring to FIG. 4B, an example of a ranking process for data subscription in a decentralized network is shown. The method 401 is, however, an example only and not limiting. The method 401 can be altered, e.g., by having stages added, removed, rearranged, combined, and/or performed concurrently. For example, the NCS 396 may perform the stages of the method 401. Based on the network message received at the stage 415, the second medical device may rank the two or more first medical devices in order to identify a device to which the second medical device will subscribe for data. At the stage 452, the ranking includes comparing the device capabilities for the two or more first medical devices to data subscription criteria for the second medical device. In various implementations, the subscription criteria may include data types, desired sampling rates, type of communicative coupling (e.g., wireless, wired, Bluetooth^{®}, Wi-Fi, etc.), data accuracy tolerances, etc. In various implementations, the criteria may be rules based, algorithm based, protocol based, or combinations thereof. These criteria may be based on rules encoded in the application layer of the second medical device. The rules may prioritize or may mandate a particular attribute. For example, the rules may prioritize a wired connection over a wireless connection or may mandate wired connections only. The rules may vary based on the particular data type. For example, the rules for ECG data may be different than the rules for SpO2 data. The rules may be based on algorithms executed by the second medical device in order to process, derive, and/or evaluate patient data. The criteria may be based on a care protocol where the second medical device needs to evaluate certain data types to determine device settings, closed loop control values, thresholds, alarms, caregiver guidance, protocol selections, etc. The criteria may also specify a verification that the first medical device is connected to a patient and not merely capable of collecting a data type without actually being connected to the patient.

At the stage 454, the second medical device may identify one first medical device as a singular data source. In this case, the second medical device may identify this singular source as the only candidate at the stage 456 and proceed to select this device as the data source at the stage 425. The identification of the singular data source may be based on the data type available or may be based on a rule that eliminates all of the other first medical devices as candidates (e.g., the rule mandates a wired connection and there is only one wired connection). The identification of the singular data source may also be based on only one device being connected to the patient. If there is not a singular data source, then the method 401 may skip the stages 454 and 456.

At the stage 462, the second medical device may identify one or more of the first medical devices as non-candidate devices. If any of the first devices do not satisfy any of the data subscription criteria, then these devices will be removed from the ranking. This may occur, for example, if the device capabilities are incompatible and/or if the device capabilities do not provide a desired data type. If none of the first devices are non-candidates, then the method 401 may skip the stage 462.

At the stage 464, the second medical device may identify two or more of the first medical devices as candidate data sources. In this case, there are multiple devices that are not eliminated as candidates based on a rules-based subscription criterion and/or that satisfy at least a portion of the subscription criteria. At a minimum, a candidate device would need to be able to collect, calculate, derive, or otherwise generate a data type that is needed or desired by the second medical device.

At the stage 466, the second medical device may generate a compatibility score for each candidate data source. The compatibility score may indicate a degree to which the device capabilities match the data subscription criteria where the various criteria are weighted. Thus a high compatibility score does not necessarily indicate a one-to-one exact match between the device capabilities of the publishing device and the data subscription criteria. Rather a high compatibility score indicates a better fit between the device capabilities of the publishing device and the data subscription criteria relative to a lower compatibility score.

At the stage 470, the second medical device may identify exchangeable and nonexchangeable data sources. For example, based on the compatibility scores, the second medical device may flag a particular first device as being uniquely suited to provide a particular type of data and therefore nonexchangeable with any other source. If there is any issue with that particular first device, such as signal degradation or other degradation of data, then the second medical device would not have any other options for obtaining that data from within the decentralized network 210. Additionally or alternatively, based on the compatibility scores, the second medical device may flag two or more first devices as exchangeable data sources. In this case, if the second medical device selects a first exchangeable device as a data source but subsequently the data degrades, then the second medical device may switch over to a second exchangeable device as a substitute. This exchange is described in further detail below with regard to FIG. 4C.

The ranking may be of particular importance for a derived parameter where a large number of devices in a patient ecosystem may be able to generate the derived parameter. As one example, respiration rate is a derived parameter that may be derived from data collected by at least a CO2 sensor, a ventilator, a flow sensor, ECG/defibrillation pads, ECG leads, an SpO2 sensor, a continuous blood pressure sensor, an accelerometer, manual input by a caregiver, or a CPR sensor. Thus where a medical device wants to subscribe to respiration rate data, there may be many candidate sources.

As an additional consideration, the subscribing device may not want to oversubscribe. In other words, the subscribing device may not want to receive more data than it needs execute a therapy or intervention, an algorithm, a protocol, etc. Therefore, the subscription criteria may also include an ability of the publishing device to limit the data to a type, publication frequency, refresh rate, accuracy, etc. as dictated by the needs of the subscribing device.

At the stage 472, the second medical device may rank the candidate data sources based on the compatibility scores. Once ranked from highest to lowest compatibility, the method may return to the selection stage 425 in FIG. 4A.

Referring again to FIG. 4A, at the stage 425, the method includes selecting a desired source for the patient data from the one or more candidate devices based on the ranking. For example, the NCS 396 of one of the medical devices shown in FIG. 2A may select another one of the medical devices shown in FIG. 2A as a desired source for patient data. In an implementation, a medical device or computing device may select a computing device as a desired source for the patient data. In an implementation, the second medical device may select multiple desired data sources for various needs of the second medical device.

At the stage 430, the method includes publishing a request for patient data indicating the desired source by the second medical device. The second medical device, or data consumer, may publish a request for the patient data indicating the desired data source by the data consumer. In an implementation, a computing device may publish a request for data from a medical device or other computing device and/or the medical device may publish a request for data from a computing device.

At the stage 435, the method includes subscribing to the desired data source by the second medical device. The second medical device (i.e., the data consumer device) may subscribe to the desired data source of another medical device or a computing device. In an implementation, a computing device may subscribe to a desired data source which may be a medical device or a computing device. When the consumer device or node subscribes to data from another node that can send or publish the data, the subscribing device is requesting that the publishing node send it requested information. The publishing device records the subscription request and sends the requested data to the subscribing device on either a periodic schedule (e.g., at regular time intervals) or a dynamic schedule (e.g., whenever the publishing device has new or updated data). With a subscription, a communication channel is established between the source node and the requestor node for a particular data type along with subscription parameters. For example, the subscription parameters may include a frequency of transmission. Frequency of transmission may include transmission at a specified time interval (e.g., send every N seconds), transmission at a specified collection interval (e.g., send every Nth data sample), data streamed substantially continuously in real-time upon availability at the source, or a transmission at the time of or at a particular interval from response to a request, or ping, from the requestor node. For example, the transmission may be to send data the next time it is available to the source node after the request from the requestor node.

At the stage 440, the method includes publishing the patient data to the decentralized network by the desired source in response to the request. The desired source publishes the patient data requested by the data consumer to the decentralized network 210. In an implementation, the desired source has multiple types of patient data available in addition to the data requested by the data consumer. Thus, the desired type of patient data may be one of multiple types of patient data available from the desired source. However, the desired source may only publish the type or types of data requested by the data consumer. In this way, the desired source need only transmit data that the consuming device requested and needs, and the consuming device need only receive the requested data. Thus, the publish and subscribe data request and provision from the decentralized network 210 provide improved efficiency over a system where a device publishes all of its data from a patient encounter or portion thereof to a server and then a receiving device has to access the server and parse the data or exchange communications with the server to determine what is available. In this server exchange situation, the first device has to send the server all of its data in order for the second device to be able to pick and choose various subsets. If the data exchange needs to happen in real time with dynamically changing data, then the first device is tasked with an almost continuous burden of uploading data to the server. In contrast, the decentralized network exchange provides a faster exchange with less network overhead in terms of data transmissions and message exchanges than a server-based exchange. In the decentralized network examples described herein, each device knows what every other device has available, but each device need only transmit and receive the amount and type of data needed.

In an implementation, the data consumer (i.e., either the second medical device referred to above or a computing device) may determine a desired or acceptable data compression for a patient data type based at least in part on one or more of the patient data sampling frequency or the patient data resolution available from the desired data source. In this case, the data consumer may publish the request for the patient data with an indication of the desired data compression and the data source may compress the patient data in response to and according to the published request. The data source may then publish the compressed data to the decentralized network 210.

At the stage 445, the method includes routing the patient data from the desired source to the second medical device by the decentralized network. For example, the decentralized network 210 routes the patient data from the desired source to the second medical device according to a routing protocol for the decentralized network.

At the stage 450, the method includes consuming the patient data from the desired source by the second medical device. For example, the medical device that requested the data receives the data through the decentralized network and utilizes that data as needed.

In an implementation, the medical devices and/or computing devices forming the nodes of the decentralized network 210 may be configured to provide an indication of associations based on data exchange between devices in the decentralized network. The associations are those between data consumers and data suppliers in the decentralized network. For example, the medical devices and/or computing devices (e.g., the devices shown in FIG. 2A) may include a light or sound indicator that displays a same color light or light emission pattern (e.g., blinking, steady, or a combination thereof) and/or a same sound at devices associated with one another as a data consumer-supplier pair. In an implementation, the decentralized network 210 may include one or more mobile computing devices with a display screen, such as the tablet 225, the patient charting device 250, or the edge server 260. The display screen may include a listing or graphic representation of patient ecosystem devices in the decentralized network 210 and indicate on the listing or graphic representation devices providing or consuming data along with which data is provided. This display of the indication of associations between data consumers and data suppliers in the decentralized network 210 would serve as a data exchange map for member nodes of the decentralized network 210. The data exchange map may be available on more than one display screen, including a medical device display screen, and may update itself dynamically as data exchange associations within the decentralized network 210 change. In an implementation, the clinical guidance engine 228 may cause a display of the data exchange map at a caregiver decision support user interface (e.g., the caregiver guidance UI 2135 on the caregiver guidance interface device 2130 as shown in FIG. 21). In an implementation, the caregiver guidance UI 2135 may enable a user to enter a quern, to request an indication of a source or a set of possible sources for a particular data type. For example, the connected devices window 2140 and/or the graphic display 2150 of available devices in the decentralized network may enable such a query and provide an indication and/or the data exchange map in response to the query. Thus a caregiver may receive an indication of which data supplier is providing data to a particular data consumer along with the type of data provided as part of caregiver guidance. In an implementation, the connected devices window 2140 and/or the graphic display 2150 of available devices in the decentralized network may enable a user to enter a manual request for a source of information. For example, the caregiver may edit the indication of which data supplier is providing data to a particular data consumer to manually change the source. In an implementation, clicking on or hovering over the indication of data supplier or selecting a manual entry control may provide an editing field and/or a source menu from which the user may select a source of information.

Referring to FIG. 4C, a method of interchanging data sources due to data degradation is shown. For example, at least one of the NCD 399 and/or the NCS 396 of the medical devices in the decentralized network may perform one or more stages of the method 402. The method 402 is, however, an example only and not limiting. The method 402 can be altered, e.g., by having stages added, removed, rearranged, combined, and/or performed concurrently.

As discussed above, in regard to FIG. 4B, at the stage 470, the second medical device may identify exchangeable sources of data. At the stage 480 of the method 402, the second medical device may detect a degradation in the data coming from a source to which the second medical device subscribed at the stage 435. Such a degradation may occur due to degradation in data transmission from the desired source to the decentralized network 210 or may occur due to some sort of disturbance at the desired source device that is causing the data received at the second medical device to be of a lower quality than that originally consumed.

If no degradation is detected, then the second medical device may continue to consume the data at the stage 450. However, if such a degradation is detected then the second medical device may select an exchangeable data source from the ranking at the stage 482. As part of this stage, the second medical device may repeat the ranking if there are multiple options for exchange. At the stage 484, the second medical device may compare the degradation to a threshold and determine at the stage 486 whether the degradation has reached the threshold. The threshold may correspond to amount of degradation at which the integrity of the data may be questionable and switching to a different data source may be beneficial. The threshold may refer to parameters of the data itself (e.g., signal to noise, data publishing frequency, missing packets, etc.) or may refer to a refreshed compatibility score based on the detected changes in the received data. Either way, when the degradation reaches the threshold, the second medical device may publish a request for patient data indicating the exchangeable data source as a new desired data source at the stage 490 and return to the stage 435 to subscribe and receive data. In order to avoid hopping back and forth between two devices which may detrimentally affect the signal quality and the data received by the second medical device and thus potentially disrupt medical care, the second medical device may allow degradation to reach the threshold before switching by returning to the consuming of data at stage 450 until the threshold is reached. Additionally or alternatively, the second medical device may impose time limit or data volume restrictions on the switching so that it doesn't occur too often so as to detrimentally affect the data collection.

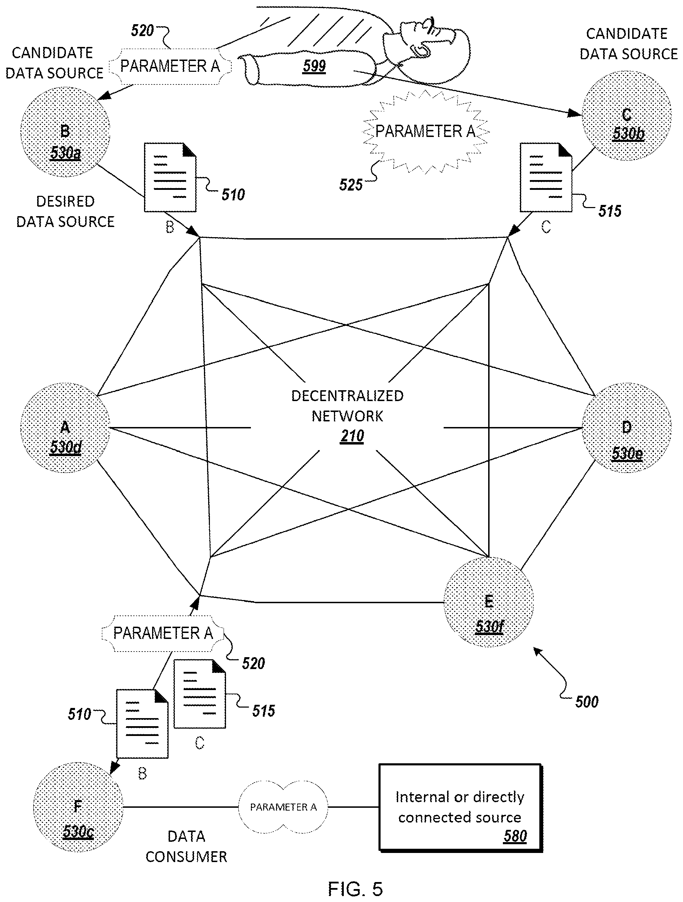

Referring to FIG. 5, a schematic diagram of one medical device ranking device capabilities of at least two other medical devices is shown. A quantity of each component in FIG. 5 is an example only and other quantities of each, or any, component could be used. In an implementation, the devices forming the nodes in the system 500 of FIG. 5 may be medical devices, computing devices, or combinations thereof. At least one of the NCD 399 or the NCS 396 associated with each device forming a node may implement the medical device ranking capabilities. The decentralized network 210 may include nodes A 530d, B 530a, C 530b, D 530e, E 530f and F 530c. In the example of the system 500, two first medical devices form node B 530a and node C 530b in the decentralized network 210. A second medical device forms node F 530c. When a device joins the decentralized network 210, the device will publish a network message. For example, the first devices at nodes B and C publish network messages 510 and 515 respectively. In an implementation, the network message (e.g., the messages 510 and 515) may be data structures or one or more blockchain ledger entries. The network messages may include metadata that every member of the decentralized network 210 may receive. For example, the second device at node F receives these network messages. In response to receiving the network messages including the metadata, a node device may request further metadata and/or or may subscribe to data from the node publishing the metadata. The data subscription may be for one or more types of data available from the publishing node. As illustrated schematically in FIG. 5, the medical devices at nodes B and C are coupled to the patient 599 and both devices have the capability to collect and provide data for parameter A. The medical device at node B receives first parameter A data 520 and the medical device at node C receives second parameter A data 525. Although both devices receive the same type of data, the characteristics of this data may differ based on the device capabilities discussed above in regard to the stage 415. The medical device at node F, having received network messages from nodes B and C may rank the device capabilities of two or more first medical devices in the decentralized network based on its own data needs. Additionally, the medical device at node F 530c may have its own internal or directly connected source 580 for parameter A. The medical device at node F 530c may rank this internal or directly connected source 580 along with those available through the decentralized network 210. The internal or directly connected source 580 may couple to the medical device at node F 530c by a wired or wireless connection independent from the decentralized network 210.

For example, parameter A may be SpO2 data available from a ventilator at node B and a pulse oximetry clip sensor at node C. Node B The medical device at node F may be a patient-monitor/defibrillator. In an implementation, the patient-monitor/defibrillator may lack its own connected SpO2 monitor and then may rank the two sources, node B or node C in order to select a device from which to receive this data. For example, the patient-monitor/defibrillator may rank these sources based on characteristics such as, for example, but not limiting of the disclosure, data rate, signal-to-noise ratio, communicative coupling type (e.g., Wi-Fi, Bluetooth^{®}, etc.).In various implementations, the ranking may be based on a care protocol specification (e.g., a type of data, a frequency of data, a data tolerance, etc.), or may be rule-based (e.g., always pick wired over wireless where wired is available, always pick a local sensor over a remote sensor where available, etc.).

As another example, computing devices may form nodes B and C. For example, a patient charting device 250 may form node B, the CAD-NAV device 253 may form node C, and the edge server 260 may form node D. Each of these devices may have patient age or other demographic information. For instance, the patient charting device 250 may include demographic information self-reported by the patient 599. the CAD-NAV device 253 may include demographic information provided to the CAD by a bystander, and the edge server 260 may include a patient health record obtained from a health provider. If a device at node F needs patient age information, this device might prioritize health record information over self-reported or bystander information.

In some examples, a mix of computing devices and medical devices may form the nodes of the decentralized network 210. These devices may also have similar information. For example, a patient chart may include pulse information based on a caregiver palpation and a medical device may include pulse information determined from a pulse oximetry sensor.

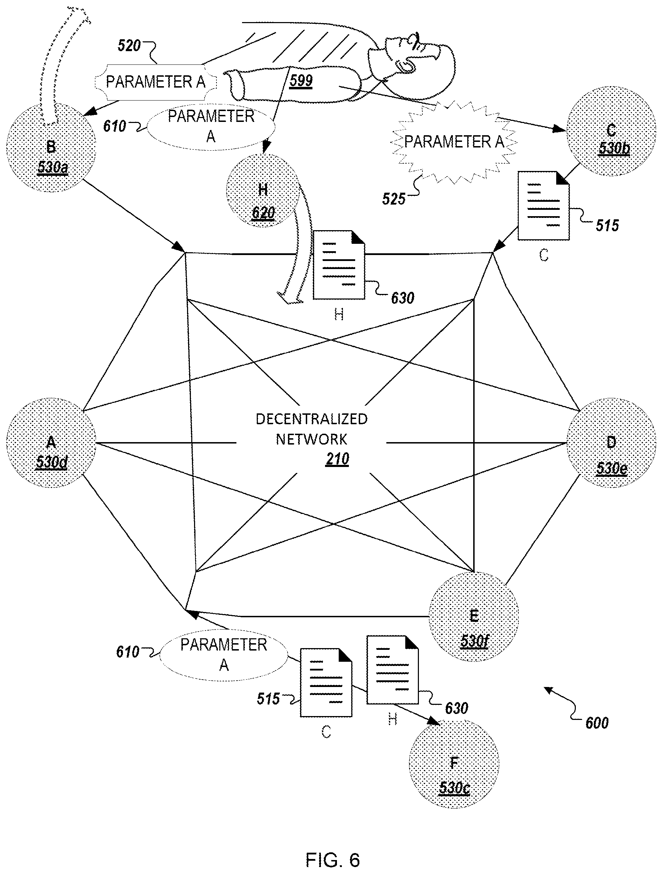

Referring to FIG. 6, a schematic diagram of a medical device re-ranking possible data sources in response to changes in the decentralized network is shown. A quantity of each component in FIG. 6 is an example only and other quantities of each, or any, component could be used. In the example of the system 600 of FIG. 6, the devices forming the nodes in the system 600 may be medical devices, computing devices, or combinations thereof. At least one of the NCD 399 or the NCS 396 associated with each device forming a node may implement the medical device ranking capabilities. In the example of FIG. 6, the device at node B 530a is exiting the decentralized network 210 and a new device forming node H 620 is entering the decentralized network 210. The device at node F 530c may receive an indication that the device at node B 530a, which was the desired source for parameter A, has exited the decentralized network 210. When a device joins the decentralized network 210, the handshake would include broadcasting a message to the rest of the network of what the new device has to offer. This broadcast effectively pings every node element and requests a return broadcast as part of the handshake. The return broadcast may include an updated list of all the devices currently in the network (e.g., nodes, data available, specifications, etc. for each device). In a blockchain implementation, this broadcast would constitute publishing a new block in the chain containing this updated list. Once that broadcast exchange is complete as part of the handshake, anytime the composition of the decentralized network 210 changes (i.e., the devices forming nodes change) every node gets pinged with the new updated network contents list. The list may be a library or a database, for example. In some implementations, this may occur when a node is added or removed and/or when a node itself changes. For example, the node itself may change if an accessory is connected to a device. This would change the data sources available from the node.

The device at node F 530c may revert to receiving parameter A 525 from node C 530b. Alternatively, a new device may enter the decentralized network 210 at node H 620. This device may also be able to provide parameter A 610. The new device at node H 620 may provide the network message 630. The device at node F 530c may receive the network message 630 and rank the devices at nodes C 530b and H 620 to identify a substitute source in the decentralized network for the desired type of patient data previously received from node B 530a. Once identified, the device at node F 530c may republish a request for data from the substitute and updated source and subscribe to that updated source for the desired data. As shown in the example in FIG. 6, the device at node F 530c ranked the devices at nodes C 530b and H 620 and selected the device at node H 620 as the desired source for parameter A 610.

The data subscription criteria used in the ranking processes and methods described herein, for example, in conjunction with FIGS. 4A-6, may be default criteria programmed into the node devices, for example, as programmed into the NCS 396 when the NCS 396 is provided to a particular device. This may enable a manufacturer to cause an entire fleet of devices associated with a particular organization or all of the devices of a particular type from the manufacturer to operate subject to the same or similar ranking criteria. In an implementation, default criteria programmed via the NCS 396 may enable updates and/or changes in default criteria via a software change or upgrade. The default criteria may be non-customizable parameters (i.e., unavailable as user selectable settings). Alternatively, in an implementation, the NCS 396 generate, provide, or otherwise enable a user interface ranking control that allows a user of the medical device to specify customized data subscription criteria. For example, the ranking control may be available in device mode that allows a supervisor or medical director to customize the data subscription criteria for an individual device. In turn, the ranking processes and methods may be customized for each medical device. This may be helpful when the decision criteria may vary based on a particular patient care environment. For example, the particular patient care environment may determine a preference between wired or wireless connections and/or between sources of particular physiologic data. For instance, the selection described in other examples between using a pulse rate from a pulse oximeter or a heart rate from ECG data may depend on the circumstances of a particular patient care environment.

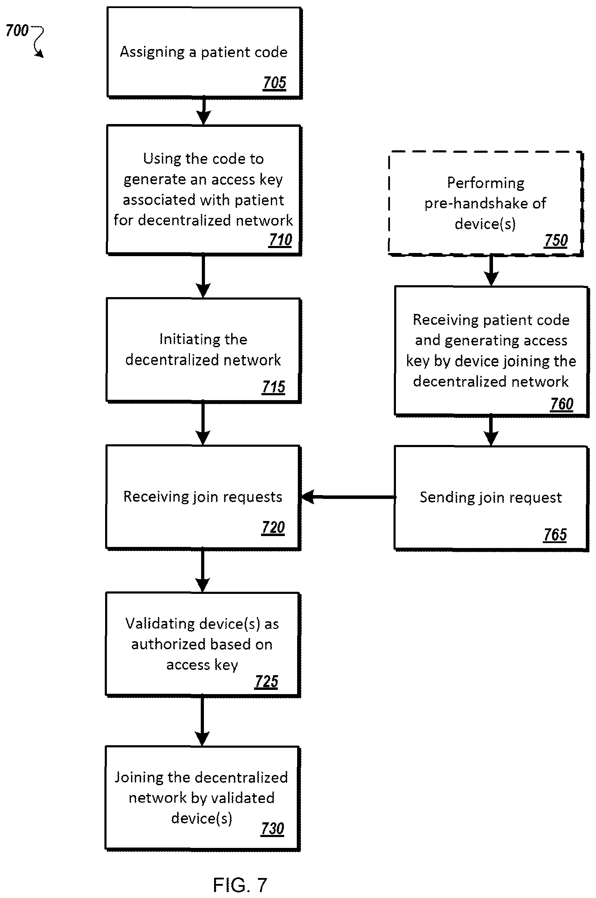

Referring to FIG. 7, an example of a method of patient-centric decentralized network formation is shown. At least one of the NCD 399 or the NCS 396 associated with each device forming a node may perform the stages of the method 700. The method 700 is, however, an example only and not limiting. The method 700 can be altered, e.g., by having stages added, removed, rearranged, combined, and/or performed concurrently.

At the stage 705, the method 700 includes assigning a patient code to the patient. The patient code may associate the decentralized network 210 with a patient-specific network access key. The decentralized network 210 may communicatively couple multiple devices that are all associated with a single patient. In this way, the patient information that the devices share with one another via the decentralized network is all for the same patient. Additionally, by associating the decentralized network 210 with a single and specific patient, medical devices and computing devices may enter and exit the network as the personnel associated with these devices and/or the patient's needs change, but the information shared through the decentralized network 210 is always associated with the patient. In an implementation, the decentralized network 210 may associate with one patient based on a network access code assigned to the patient. The patient-specific network access key may ensure that all devices in the decentralized network are associated with a same patient. In other words, all of the devices in the decentralized network are part of a same patient ecosystem.

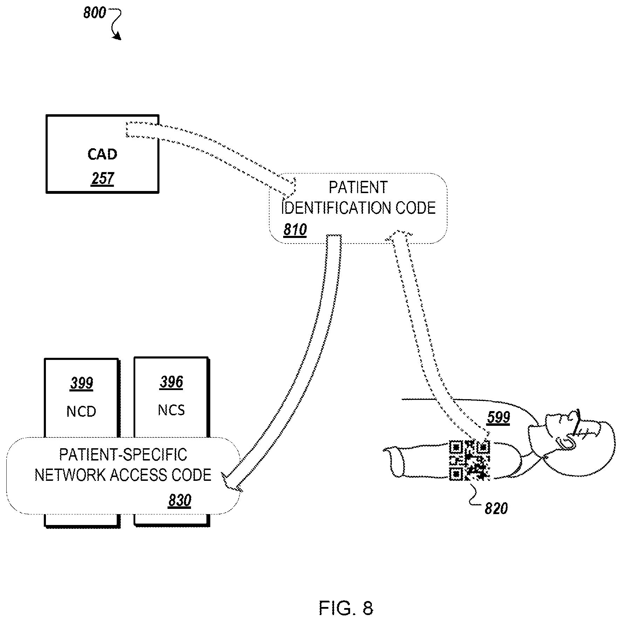

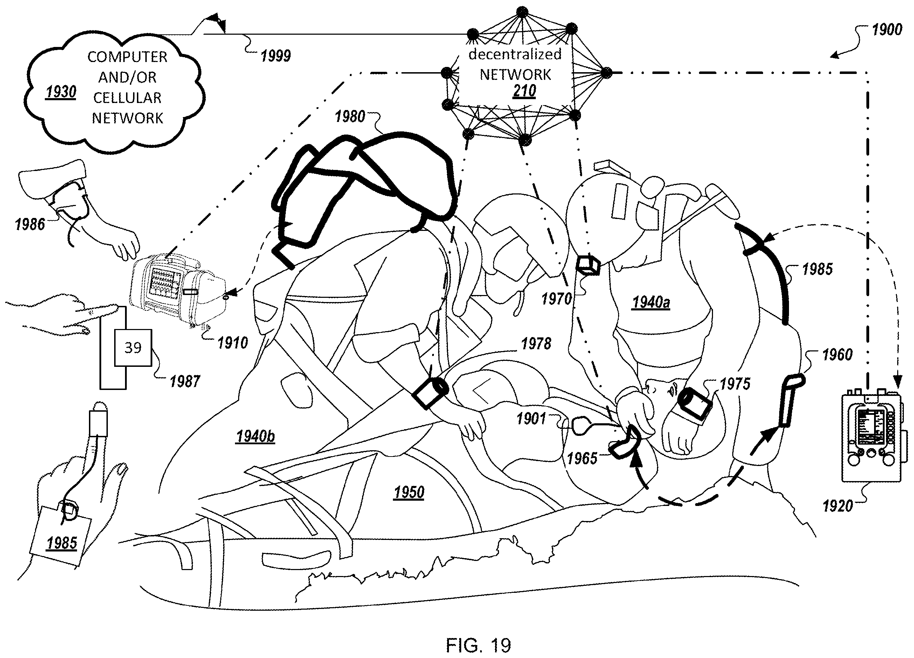

Referring to FIG. 8, examples of sources of a patient identification code are shown. A quantity of each component in FIG. 8 is an example only and other quantities of each, or any, component could be used. In an implementation, the CAD service 257 in the system 800 may assign a patient identification code 810. For example, the CAD service 257 may assign the patient identification code 810 as part of an initiation of a transport or emergency response by EMS when the CAD receives patient demographic information for the response. Alternatively, a caregiver may affix a quick response (QR) code or bar code that includes a patient identification code to the patient. The QR or bar code may be on a sticker, may be on a medical device such as defibrillation electrodes, a tourniquet or bandage, military identification tags, or dog tags, (e.g., the dog tag 1901 shown in FIG. 19), or other device that,is attached to and stays with the patient, or may be on a bracelet or anklet or other item worn by the patient. In some implementations, a device may acquire the code via near field communications, manual entry and/or via a patient biometric measurement.

In an implementation, when a device enters the decentralized network 210, the device may receive the patient code as part of the handshake protocol. Alternatively, the device may generate their own patient code which may be synchronized with or associated other patient codes. The network 210 may hold this list of patient codes as a reference list of codes that all refer to the same patient by virtue of entry into the network. Where the network 210 includes an active communications link with a cloud server, the common reference link between various patient codes may be stored together with patient data. In some implementations the patient code may serve as a gateway to entry. In other implementations, the patient code may associate the data from a device with a particular patient along with other codes for devices in the network 210 without serving the gateway function. Where the patient code serves the gateway function, the joining device may acquire the code as described above and the acquisition of the code may provide credentials that enable the device to join the network. Alternatively, a joining device may ping the network and the network may provide the patient code in order to admit the joining device.

The network communications driver 399 of at least one medical device or computing device may receive the patient identification code 810 and use identification code to generate the patient-specific network access code 830. In an implementation, the NCS 396 may receive the code 810 from a user of the at least one medical device or computing device and/or may receive the code via other software or applications implemented on the at least one medical device or computing device. The NCS 396 may provide the code to the NCD 399. For example, in an implementation, the software or applications may generate a user interface control to request input of the patient identification code 810 by a user and then store the code 810 in memory for retrieval and use by the NCS 396. As another example, in an implementation, the at least one medical device or computing device may receive the patient identification code 810 from another device and store the code 810 in memory for retrieval and use by the NCS 396. n some implementations, the patient-specific network access code 830 may be different at each or one or more of the devices in the decentralized network 210. However, the devices may link these codes such that they all refer to the same patient. In an implementation, when a code is associated with a patient that code may be stored as part of the patient data. If additional codes are associated with the patient, they may also be stored as part of the patient data. When queried for a code for patient association, the data repository of the codes may search all codes that have been stored for the patient.

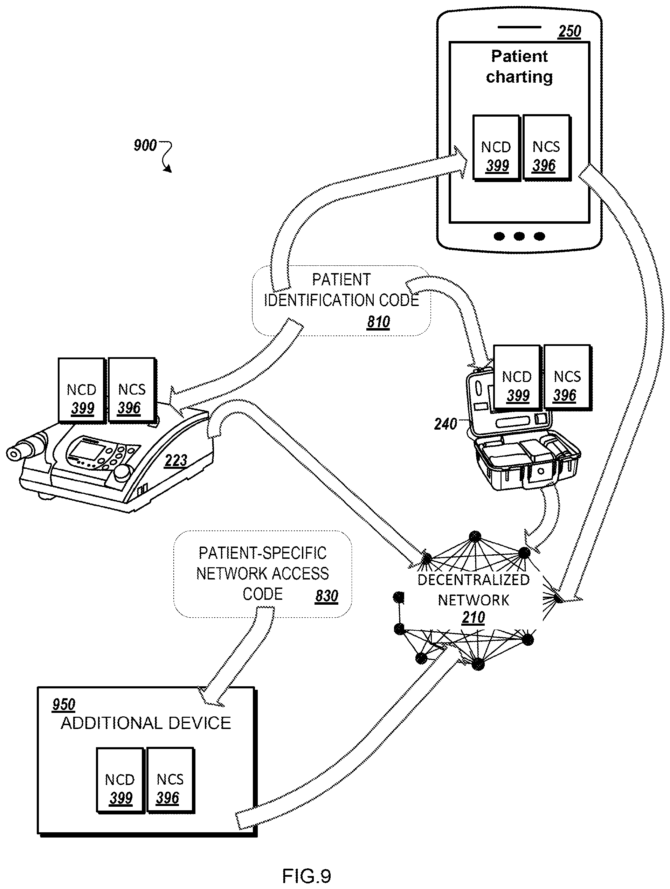

Referring to FIG. 9, an example of a device system for initiating the decentralized network based on the patient-specific network access code is shown. A quantity of each component in FIG. 9 is an example only and other quantities of each, or any, component could be used.

In the example of the system 900, a patient charting device 250, an AED 223, and a trauma kit 240 are shown. One or more of these devices may be the first devices deployed for care of emergency victim or for a transport patient. EMS crews, whether BLS or ALS, utilize the patient charting device 250 to generate an ePCR and the AED 223 and/or trauma kit 240 enable provision of BLS services which may be the first level of care provided. In this example, one or more of the patient charting device 250, the AED 223, or the trauma kit 240 may receive a patient identification code 810. Based on the examples shown in FIG. 8, the CAD may generate the patient identification code and provide that code directly to one or more of the patient charting device 250, the AED 223, or the trauma kit 240. Alternatively, the CAD service 257 may provide that code For example, the CAD service 257 may be communicatively coupled to the CAD-NAV device 253 and provide the code to the CAD-NAV device 253 which may in turn provide the code to one or more of the patient charting device 250, the AED 223, or the trauma kit 240. The CAD-NAV device 253 has a communicative coupling to the CAD service 257 at least to initiate an EMS response and therefore, if the CAD service 257 generates and provides the code to the CAD-NAV device 253 during this initiation, the code is available to the rest of the devices even if a connection with the CAD service 257 is lost.

Additionally or alternatively, one or more of the patient charting device 250, AED 223, and trauma kit 240 may scan the QR or bar code 820 affixed to the patient and retrieve the patient identification code.

The devices included in FIG. 9 are examples only. In various implementations, other devices or combinations of devices may receive the patient identification code and generate a patient-specific network access code.

Referring again to FIG. 7, at the stage 710, the method 700 includes generating a patient-specific network access code using the patient identification code by a first medical device or by a mobile computing device. For example, one or more of the patient charting device 250, AED 223, and trauma kit 240, or other device(s) at the patient scene, may use the patient identification code to generate a patient-specific network access code.

At the stage 715, the method 700 may include initiating the decentralized network 210. The first devices at a patient care scene may establish the decentralized network based on the patient-specific network access code.

At the stage 720, the method 700 includes receiving join requests from additional medical devices and/or computing devices. For example, as shown in FIG. 9, the additional device 950 that includes the NCD 399 and NCS 396 may request to join the decentralized network 210. The decentralized network 210 may provide the patient-specific network access code 830. Alternatively, the additional devices may be provisioned with the patient-specific network access code, for example, by receiving the patient code and generating the patient-specific network access code at the stage 760 prior to sending a join request to the decentralized network at the stage 765.

In an implementation, one or more of the additional devices trying to join the decentralized network may be part of a collection of devices associated with at least one of an EMS agency, an EMS transport vehicle, or an EMS crew. At the stage 750. devices in such a collection may exchange handshake information amongst themselves prior to an arrival at a patient scene to pre-configure the medical devices and/or computing devices for decentralized network entry. This pre-configuration may not require the identification of a particular patient; association of a patient with the pre-configured network may occur at the scene. In an example, the devices may exchange handshake information at an EMS agency or on an ambulance. The handshake information may be exchanged via radio signals, near field communications, and/or tap-to-connect capabilities. This pre-configuration may generate an inventory list for the EMS agency, transport, and/or crew. One or more devices may store this inventory list such that it is accessible to other devices upon entry into the decentralized network 210. The storage location for the inventory list may be a central registration service, a peer-to-peer storage, a blockchain ledger, or a combination thereof.

The advantage of the pre-configuration is that rather than requiring a user to potentially spend significant amounts of time in manually configuring the system of each medical and/or computing device to join the decentralized network 210, the medical and/or computing device(s) located at the emergency scene may be pre-configured to dynamically join and/or leave the decentralized network 210, for example, automatically and/or with one or more simple actions (e.g., switch actuation, pressing a button, near field communication connection, radio frequency, location/proximity recognition, gestural code, tap/bump recognition, motion-activated, sound/vibration, voice command/recognition, amongst others) and/or merely by being in close physical proximity to one another such as by a Bluetooth^{®} proximity connection.

In an implementation, two collections of devices may independently pre-configure themselves and form separate decentralized networks that subsequently may combine with one another. For example, a collection of devices on a BLS EMS vehicle may pre-configure to form a decentralized network based on a first handshake. A collection of devices on an ALS EMS vehicle may pre-configure to form a decentralized network on an ALS EMS vehicle. When the ALS EMS vehicle arrives on a scene already populated by BLS EMS devices, the two decentralized networks may merge.

At the stage 725, the method 700 includes validating devices as having the patient-specific network access code as being authorized to join the decentralized network followed by a device joining the decentralized network at the stage 730. The validation step ensures that devices joining the decentralized network 210 are associated with the same patient as all of the other devices already in the decentralized network 210. Additionally, this step verifies a proper standard for communication and an authentication for security and encryption. The validation step may also serve to limit the number of devices and/or the specific devices that have permission to subscribe to data from the decentralized network 210 to control the distribution and dissemination of patient data.

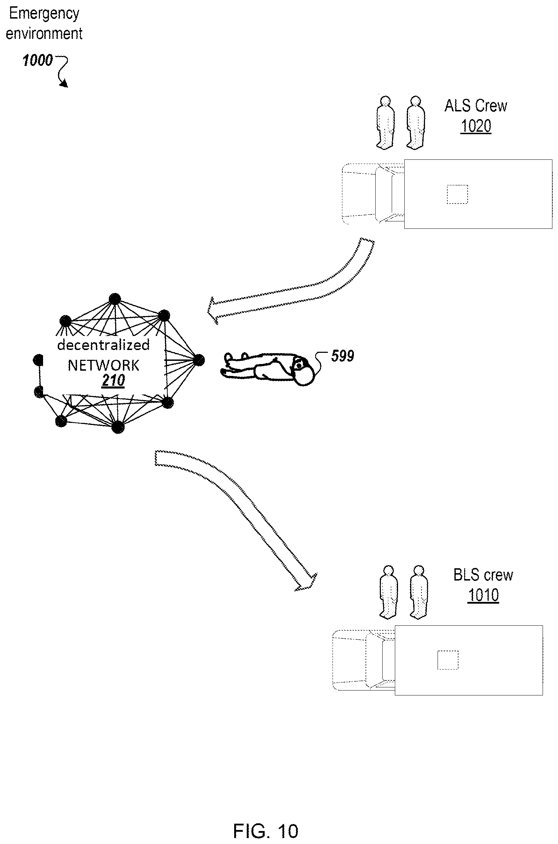

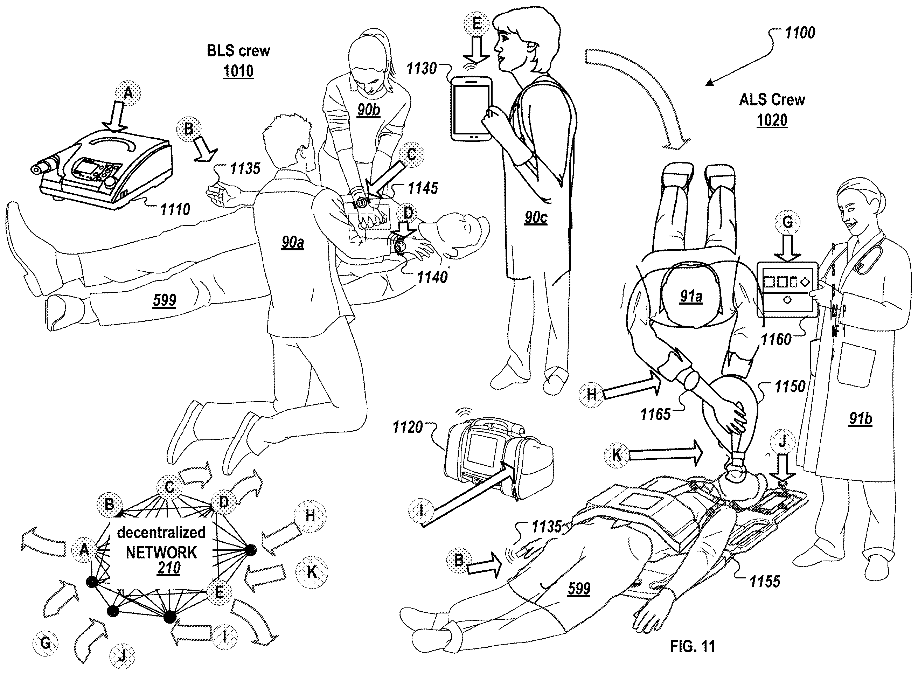

Referring to FIGS. 10 and 11, an example of a change in medical and/or computing devices in a decentralized network due to a BLS/ALS transition is shown. A quantity of each component in FIGS. 10 and 11 is an example only and other quantities of each, or any, component could be used.

As shown schematically in FIG. 10, a patient 599 in an emergency environment 1000 (e.g., the scene of a patient injury or medical emergency) may initially be treated and/or monitored by a BLS crew 1010. The decentralized network 210 may include the BLS devices associated with the BLS crew 1010. At some point in time, an ALS crew 1020 may arrive on scene and that crew may take over care of the patient 599. The ALS crew 1020 may have additional devices, e.g., ALS devices or other devices associated with the ALS crew 1020, that will join the decentralized network 210 and some or all of the BLS devices will exit the decentralized network 210. EMS crews typically bring their own equipment to a scene and the devices used for patient care generally remain with the associated crew rather than with the patient. Therefore, data exchange via the decentralized network 210 provides an efficient and seamless way to pass data to new devices as needed so that the patient data remains with the patient even when the specific devices change. The data exchanges facilitated by the decentralized network 210, as described herein, enable medical and/or computing devices and medical crews attending to the patient for intervals of time within an entire arc of care to view the data from preceding stages of care. As a benefit, this capability may expedite and facilitate an evaluation of patient progression or deterioration to better guide caregivers to detecting changes in patient state and/or evaluating the efficacy of care and/or the accuracy of diagnoses.

As shown in more detail in the system 1100 of FIG. 11, the BLS crew 1010 may be associated with, for example, an AED 1110, a pulse oximetry sensor 1135, first and second wearable computing devices 1140 and 1145 (e.g., wrist-worn devices), and a tablet 1130. The BLS crew 1010 may include three caregivers 90a, 90b, and 90c. These caregivers may provide manual compressions as illustrated in FIG. 11. The BLS crew 1010 may deploy these devices for the care of the patient 599, for example a cardiac arrest victim. These devices may form nodes A, B, C. D, and E of the decentralized network 210.

Upon the arrival of the ALS crew 1020, the BLS crew 1010 may exit the emergency scene and remove their devices. In the example of FIG. 11, the pulse oximetry sensor 1135 may stay with the patient 599. However, the ALS crew 1020, that includes the caregivers 91a and 91b, may replace the AED 1110 with a patient-monitor/defibrillator 1120 (node I), implement use of an automated compression device 1155 (node J) in place of the manual compressions, and may initiate use of a bag valve mask 1150 (node K). Furthermore, the wearable devices 1140 and 1145 may stay with caregivers 90a and 90b and the ALS crew member 91a may have his/her own wearable device 1165 (node H). The mobile computing device 1130 associated with caregiver 90c may be replaced by the mobile computing device 1160 (node G) associated with caregiver 91b. Thus where nodes A, B C, D, and E form an initial decentralized network 210, the decentralized network may transition such that nodes A, C, D, and E permanently exit the decentralized network 210, node B remains, and nodes G, H, I, J, and K enter.

In an implementation, the BLS crew 1010 may monitor the pulse oximetry readings for the patient 599 as provided by the SpO2 sensor 1135 at the patient charting device 1130. The patient charting device 1130 may receive the SpO2 data via the decentralized network 210, as may other devices in the decentralized network 210 such as the wearable devices 1140 and 1145. When the ALS crew 1020 arrives on scene, they may initiate the patient-monitor/defibrillator 1120, for example, and/or other ALS device(s) and these devices may immediately access and monitor the SpO2 data from the sensor 1135. In such a transition, there would be no gap in care and no need for the caregivers to divert attention away from the patient and towards device connections in order to avoid a break in the monitoring of the SpO2 data. They would merely initiate their medical and/or computing device as needed to take care of the patient without any extra steps to cause the medical and/or computing device to access the SpO2 data. The patient charting device 1130 may permanently exit the decentralized network and any of the ALS devices (e.g., the patient-monitor/defibrillator 1120, a patient-monitor, or a critical care ventilator) can receive physiological parameters previously tracked by the patient charting device 1130.

A decentralized network 210 at a transition from BLS to ALS may also facilitate the completion of an ePCR. Without the decentralized network 210, the BLS crew and the ALS crew either have to prepare and submit separate ePCR reports or work on a web interface to a cloud-based ePCR in order to keep all of their information for the patient in one place as the individual charting devices enter and exit the scene. However, with a decentralized network 210, the information entered on a first charting device, like 1130, is immediately available to a second charting device, such as 1160, without any cloud server connection required. Additionally, both devices have access to the data from the medical devices deployed on scene concurrently with the charting data which further may enable the completion of a single document contemporaneously with patient care.

The advantages provided by a decentralized network as described above are particularly beneficial for the BLS/ALS transition illustrated in FIGS. 10 and 11. For example, as each ALS medical device is ready and introduced, it may receive information as needed. The data provided to the decentralized network 210 by devices like a pulse oximetry sensor is immediately available to devices joining the decentralized network 210 enabling these devices to utilize this data for determining care without delays that may be introduced if a device had to access a server or connect with an individual device. If the mobile device 1130 were to function as a central node for the set of BLS devices, rather than as a member of a decentralized network, then when the caregiver 90c needs to leave the scene and remove a device from the patient ecosystem to attend to a different patient, there would be a gap in data exchange as a new device is initiated as a central node. Additionally, the mobile device 1130 may need to transfer all of its accumulated data to the new central node or to a server from which the new central node may retrieve the data. In the decentralized network 210, the mobile device 1130 may exit at any time and any devices joining can receive needed information without impact from the exit. The caregiver 90c may need to run back and forth between the patient 599 and a vehicle, or look around a patient's home for medications, or move while the caregiver 90c evaluates other victims on scene. All of these may impact the ability of a single device like the mobile device 1130 to act as an effective central node. As devices join and exit the network 210, network synchronization timestamps may be saved at one or more of the devices in order to enable post-event compilation and synchronization of patient data.

Although not illustrated in FIG. 11, a similar transition may occur when the patient 599 arrives at a hospital. The ALS crew 1020 will take most if not all of their equipment with them and transition the patient care to one or more medical devices and/or computing devices associated with hospital or other care facility staff. In an implementation, an emergency services crew may add a destination hospital or other healthcare facility to the decentralized network 210 as a cloud node while the patient is on route to the destination. This may enable a transfer of critical information to the receiving care providers before the patient arrives. The receiving care providers may access a user interface to view the information. In an implementation, medical devices at the destination may be added to the decentralized network 210 prior to patient arrival. This may expedite the transfer of the patient to the destination facility and may enable the medical devices to pre-configure for the patient. Furthermore, this connection may facilitate communications between the caregivers at the destination facility and the emergency caregivers on route for purposes of telemedicine support and guidance. As another node or nodes on the decentralized network 210, the receiving caregivers and medical devices would have access to as much information as the devices with the patient with the same ability to rank sources and control data flow through the publish and subscribe capabilities of the decentralized network 210.

An additional transition may occur between various roles of medical crews (e.g., role zero to role one, role one to role two, role two to role three, etc.). Under role zero (i.e., in or potentially in the line of fire), the medics have only what they can carry in a backpack, pocket, or on their person. This crew may provide initial and rudimentary care and then carry or transport a victim to a role one crew. The role one crew is generally responsible for casualty evacuations and may have access to a transport vehicle (e.g., an ambulance, jeep, "Humvee" (i.e., a high mobility multipurpose wheeled vehicle (HMMWV), etc.) that can provide larger devices to stabilize a patient. A role two crew, for example an army forward surgical team) may evacuate a patient, for example, by helicopter, to a combat support hospital for care by a role three crew. Similarly to the role one crew, the role two crew may have access to larger devices associated with the evacuation transport vehicle. The role three crew may provide surgical treatments, x-rays, laboratory services, and other medical support that requires a more stable environment than roles zero, one, or two.

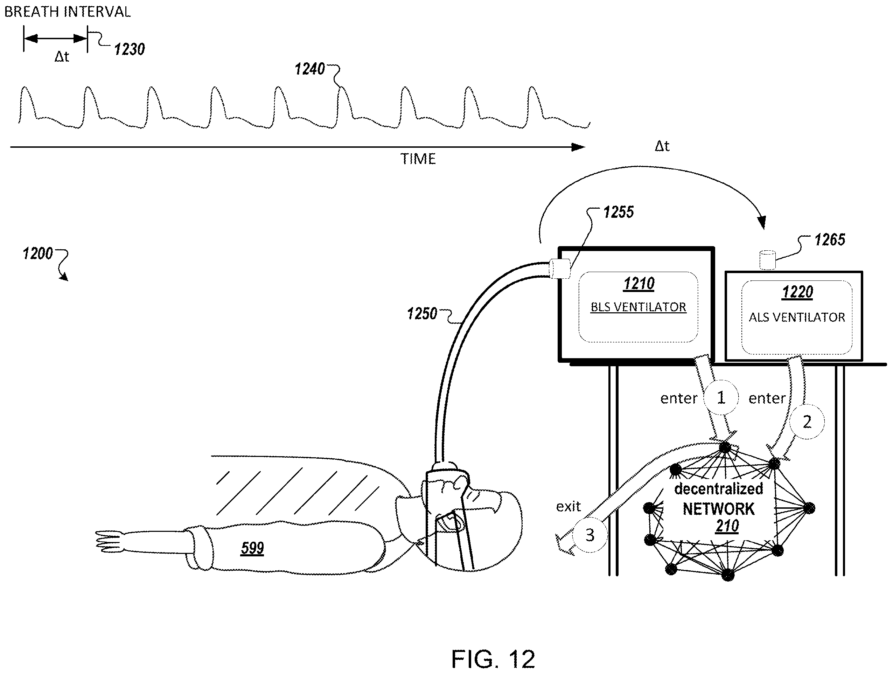

Referring to FIG. 12, an example of a decentralized network enabling a rapid change in respiratory support is shown schematically. A quantity of each component in FIG. 12 is an example only and other quantities of each, or any, component could be used.

In the system 1200 shown in FIG 12, a patient 599 is initially treated with medical devices communicatively coupled in the decentralized network 210 where the medical devices include a portable ventilator that may be configured as a BLS portable ventilator device 1210. However, the patient 599 may need more complex care than that available from the BLS ventilator 1210 and so an ALS crew may move the patient 599 to an ALS ventilator 1220, which may be a critical care ventilator. The patient 599 may receive respiratory support via a ventilation hose 1250, or patient circuit, that couples to a connection port 1255 on the BLS ventilator 1210. In an implementation, the BLS ventilator 1210 may not include SpO2 and/or EtO2 sensors and therefore may rely on other sensors in the decentralized network 210 for this information. If this information is available to the BLS ventilator 1210, this device may be able to operate under closed loop control and/or automatically adjust therapy settings based on measured physiological parameters of the patient. In various implementations, the BLS ventilator 1210 may lack the capability to provide the range of operating parameters available to an ALS ventilator. For example, a limited capacity blower may exclude shorter inspiratory times and higher inspiratory pressures as compared with an ALS ventilator. As a further example, the BLS ventilator 1210 may lack a high-pressure oxygen valve.

Upon arrival at the emergency scene and power on, the ALS ventilator 1220 may receive some or all of the patient specific operating parameters from the BLS ventilator (e.g., tidal volume, gas pressures, etc.) and configure itself to the identical operating parameters. In an implementation, the ALS ventilator 1220 is configured to transpose the BLS ventilator settings such that the effects on the patient from the two ventilators is the same. The devices may exchange these parameters through the decentralized network 210 and would not require any other communicative connection. Upon joining the decentralized network automatically at power-on, the parameters may be exchanged in a short period of time which may be as short as a single inter-breath interval 1230 between individual breaths 1240. This may correspond to a time interval of 2-5 seconds. Once configured, the caregiver need only move the ventilation hose 1250 from port 1255 on the BLS ventilator to the port 1265 on the ALS ventilator to provide the advanced care to the patient 599. Thus with the relatively seamless information exchange available from the decentralized network599, a transition from a BLS ventilation device to an ALS ventilation device to provide ventilation support may be effectuated within one breath cycle.

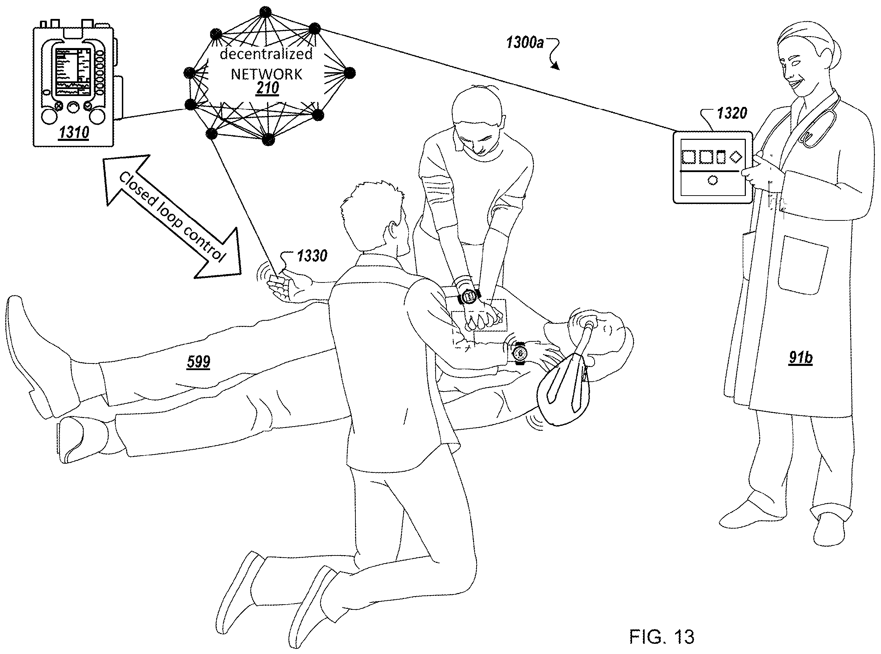

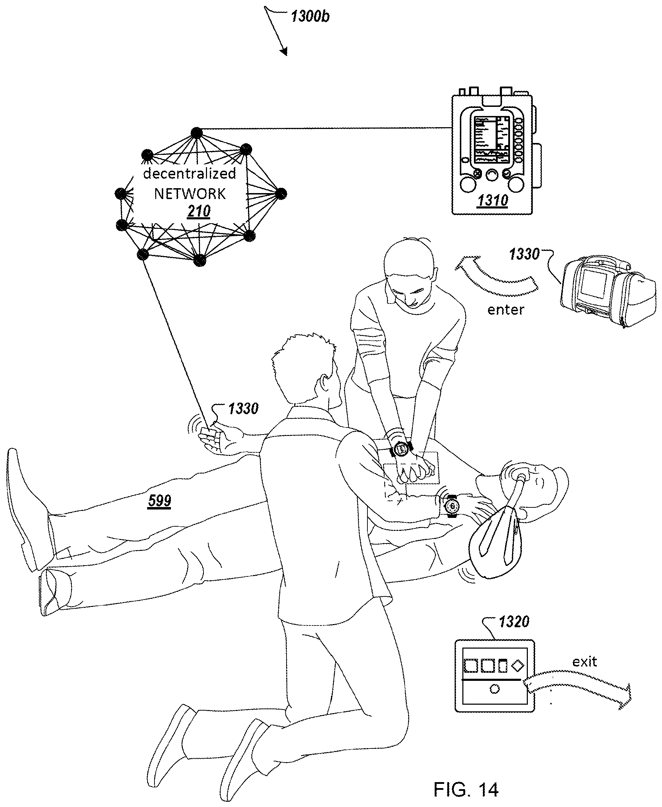

Referring to FIGS. 13 and 14, an example of closed-loop control as facilitated by a decentralized network is shown. A quantity of each component in FIGS. 13 and 14 is an example only and other quantities of each, or any, component could be used.

In the system 1300a as shown schematically in FIG. 13, the mobile computing device 1320. the ventilator 1310, and the pulse oximeter 1330 all form nodes of the decentralized network 210. The mobile computing device 1320 may initiate closed loop control of the ventilator 1310 based on patient data, e.g., SpO2 data, provided from an oxygen saturation sensor such as the pulse oximeter 1330. Once initiated, the pulse oximeter 1330 may publish the SpO2 data to the decentralized network 210 and the ventilator 1310 may subscribe to this data to receive it via the decentralized network 210. The ventilator 1310 may then operate in a closed loop control mode based on this data. Subsequently, as shown schematically in the system 1300b in FIG. 13, the mobile computing device 1320 may exit the decentralized network 210. For example, the caregiver 91b may need to use the mobile computing device 1320 in a different place within the emergency environment, may need to use it to attend to another patient, or may need to leave the scene altogether with the mobile computing device 1320. However, when the mobile computing device 1320 exits, the ventilator 1310 does not lose its access to the pulse oximetry data because the mobile computing device 1320 is not a conduit. Rather, the mobile computing device 1320 just initiates the closed loop control with the decentralized network 210 as the data conduit. Thus, the ventilator 1310 can continue to maintain ventilation support in a closed loop control mode unaffected by the departure of the mobile computing device 1320. The provision of the patient data from the sensor 1330 may continue without interruption due to the exit of the mobile computing device 1320.

In FIGS. 13 and 14 the mobile computing device 1320, the ventilator 1310, and the pulse oximeter 1330 are provided as examples only and not limiting of the disclosure. Transitions in closed loop control for other medical devices and/or based on other physiological sensors and/or data are contemplated. The device initiating the closed loop control is shown as a mobile computing device, but another medical device may also fulfill this function.

In an implementation, a new device 1330, (e.g., a medical device or sensor, shown in FIG. 14 as a patient-monitor/defibrillator as an example only but not limited to this device) may enter the decentralized network and the patient care ecosystem. The new device 1330 may, for example, provide a patient care function that is unavailable from the existing devices in the network 210 and/or be associated with a new team of caregivers associated with the patient. For example, the patient may require defibrillation and the network 210 may not include a defibrillator, the patient may require treatment from an ALS defibrillator that may replace a BLS defibrillator in the network, or an ALS or hospital crew may replace a BLS or other EMS crew and transition the patient to new devices. The new device 1330 may publish a network message to the decentralized network 210 indicating the type(s) of patient data available. The medical device 1310 may determine that the new patient data from the new device 1330 may be substantially equivalent to, interchangeable with, and/or suitable for the same closed loop control. As a result, the medical device 1330 may subscribe to the new patient data available from the new medical device 1330, receive this data via the decentralized network 210, and modify the closed loop control to operate based on the new patient data in place of the data from sensor 1330. This transition may be accomplished without any caregiver interaction with the medical devices and thus not distract the caregivers from their patient focused tasks. The new data may provide one or more improvements over the original patient data such as better resolution, signal strength, signal to noise, frequency of collection, etc. As an example, the new device may be a patient-monitor/defibrillator with a different sensor and/or that also may provide some data analytics that better suit the sensor data to the needs of the device operating in closed loop mode.

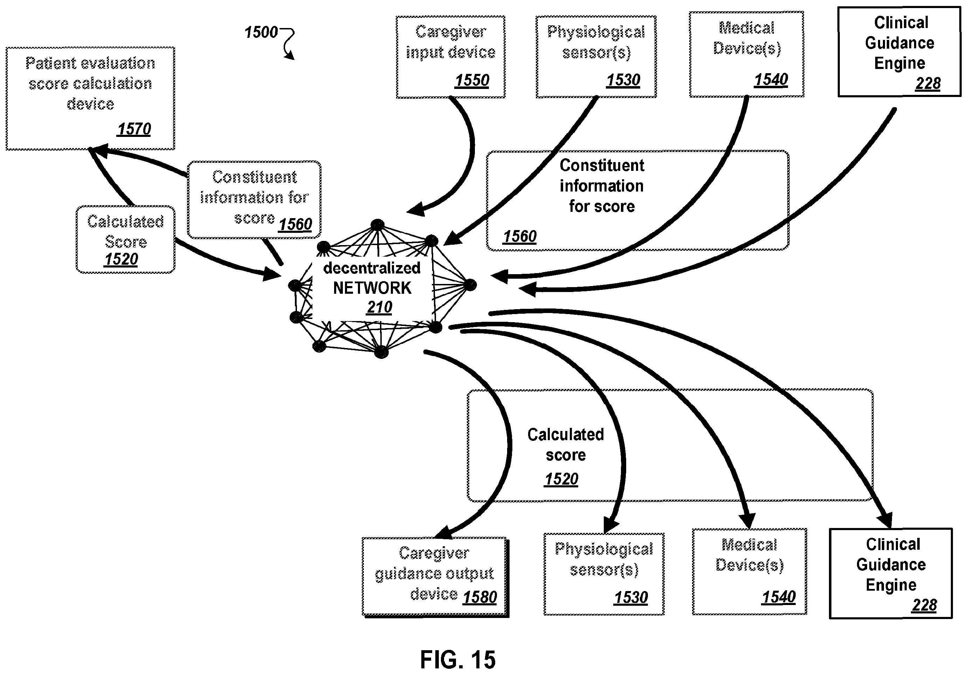

Referring to FIG. 15, an example of patient evaluation score determination and distribution using a decentralized network is shown. A quantity of each component in FIG. 15 is an example only and other quantities of each, or any, component could be used.

In an implementation example of the system 1500, the decentralized network 210 may include at least one device 1570 configured to calculate a patient evaluation score. The patient evaluation score is a quantitative value assigned to one or more physiological parameters, observational parameters, or a combination thereof in order to ascertain a degree of illness or injury in a patient or a likelihood of a particular illness or injury. Such score is often used to triage patients, to determine an initial treatment of a patient, or to monitor a patient for changes that require new or changed treatment. The patient evaluation score may require input from multiple sources, such as multiple physiological sensors and possible input from a caregiver observing and assessing the patient. As examples, the caregiver may note a skin tone or a pupil dilation or may take a temperature or pulse reading or may ask a patient to perform a task. The patient evaluation score calculation device 1570 may be a computing device or a medical device and may be distributed over multiple devices.

The constituent information 1560 for a score may originate from a caregiver input device 1550, physiological sensor(s) 1530, medical device(s) 1540, the clinical guidance engine 228, or a combination thereof. The constituent information may include physiological sensor measurements, caregiver observations input to a caregiver input device 1550 or a combination thereof. Although shown as one device, the caregiver input device 1550 may be multiple devices configured to capture caregiver input. Components of a caregiver observation may be provided at different devices. For example, for a MEWS score as discussed further below, one or more of the alertness, verbal, pain, and unconscious indicators could be provided at different input devices and/or by different caregivers. These devices may publish the constituent information 1560 to the decentralized network 210 and the patient evaluation score calculation device 1570 may subscribe and receive the constituent information 1560. The patient evaluation score calculation device 1570 may calculate the patient evaluation score based on the constituent information and publish the patient evaluation score 1520 to the decentralized network 210. Devices in the decentralized network 210 like a caregiver guidance output device 1580 (which may be a same device as the caregiver input device 1550), physiological sensor(s) 1530, medical device(s) 1540, and the clinical guidance engine 228. One or more of these devices may subscribe to and receive the patient evaluation score 1520 and use this score. For example, a device may use the score by displaying the score, incorporating the score into a patient data analysis, such as a determination of clinical guidance or patient treatment settings, adjustments, or sequences.

In an implementation, a device may also use the score by monitoring the score in order to generate an alarm if the score exceeds a threshold or range. This may be of particular use to caregivers and the patient where calculating the patient evaluation score requires information from at least two nodes in the decentralized network that are different from one or more nodes at which display or knowledge of the score is critical to patient care. In an implementation, the decentralized network 210 may detect a nearby or connected device and recommend that data available from such a device be accessed in order to facilitate a score calculation. For instance, a lactate monitor may facilitate, supplement, or corroborate a sequential organ failure assessment (SOFA) or quick SOFA (qSOFA) score determination based on correlations between blood lactate levels and SOFA known to those of skill in the art.

As another example, a patient score may represent a value derived from measured physiological parameters such as a ratio of oxygen saturation (e.g., oxygen saturation as measured by an SpO2 sensor) to inspired oxygen (e.g., FiO2 as measured by an EtCO2 sensor). This ratio may be calculated by a node on the decentralized network 210 by virtue of access to both sensors via the decentralized network 210. The calculating node may publish the ratio to the decentralized network 210. This ratio may be predictive of mortality risk and thus may inform the care provided by the emergency crew and subsequent crews. Furthermore, any decision support utilized during care may adjust workflows, guidance, and recommendations for treatment, medication, and/or transport based on such a risk evaluation.

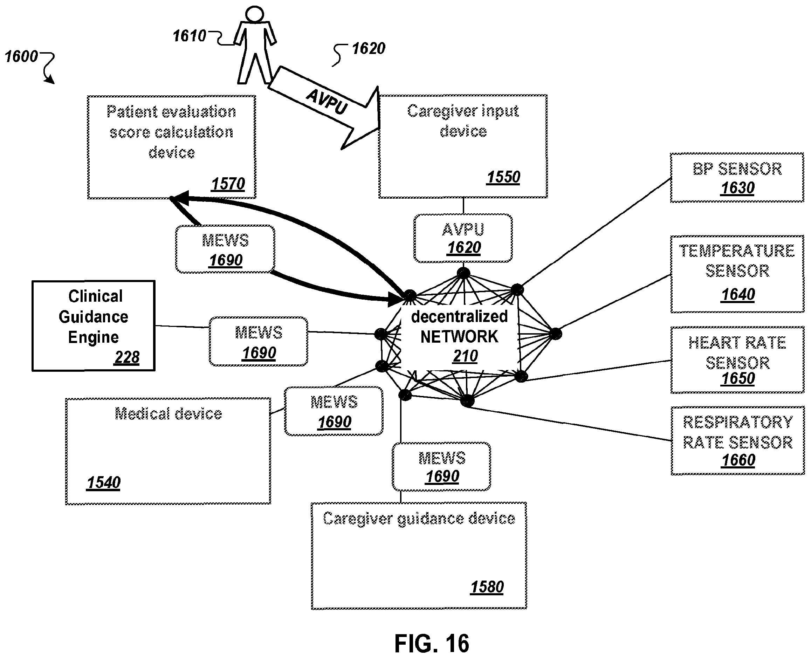

Referring to FIG. 16, an example of MEWS score determination and distribution using a decentralized network is shown. A quantity of each component in FIG. 16 is an example only and other quantities of each, or any, component could be used. In various implementations, as illustrated in the exemplary system 1600, the MEWS score is determined using input from one or more nodes of the network 210 and then distributed to one or more nodes for display and/or use in a treatment determination algorithm or patient care protocol.

In an implementation, the patient evaluation score may be a modified early warning score (MEWS). The constituent information for the MEWS score may include a blood pressure (BP) measurement, a temperature measurement, a heart rate (HR) measurement, and a respiratory rate (RR) measurement. One or more of a BP sensor 1630, a temperature sensor 1640, an HR sensor 1650, and a RR sensor 1660 may be nodes in the decentralized network 210 or one or more of these sensors may be communicatively coupled to a medical device 1540 that may be a node in the decentralized network 210. The BP sensor, the temperature sensor, the HR sensor, the RR sensor and/or the medical device 1540 coupled to one or more of these sensors may publish the BP measurement, the temperature measurement, the HR measurement, and the RR measurement to the decentralized network 210. The patient evaluation score calculation device 1570 may subscribe to this sensor data and receive the data via the decentralized network 210. Additionally, a caregiver 1610 may provide evaluations of an alertness, voice, pain, and unresponsive (AVPU) scale at the at least one device 1550 configured to receive the caregiver observation input. The device 1550 may publish the AVPU scale 1620 to the decentralized network 210 and the device 1570 may subscribe to and receive the AVPU scale 1620. The patient evaluation score calculation device 1570 may use the sensor measurements and AVPU score to calculate the MEWS score 1690 and publish the MEWS score 1690 to the decentralized network 210. One or more of the clinical guidance engine 228, the medical device 1540, and the caregiver guidance output device 1580 may then receive the MEWS score 1690 and use for display, caregiver guidance determination, monitoring, data analysis, treatment determination or adjustment, etc.

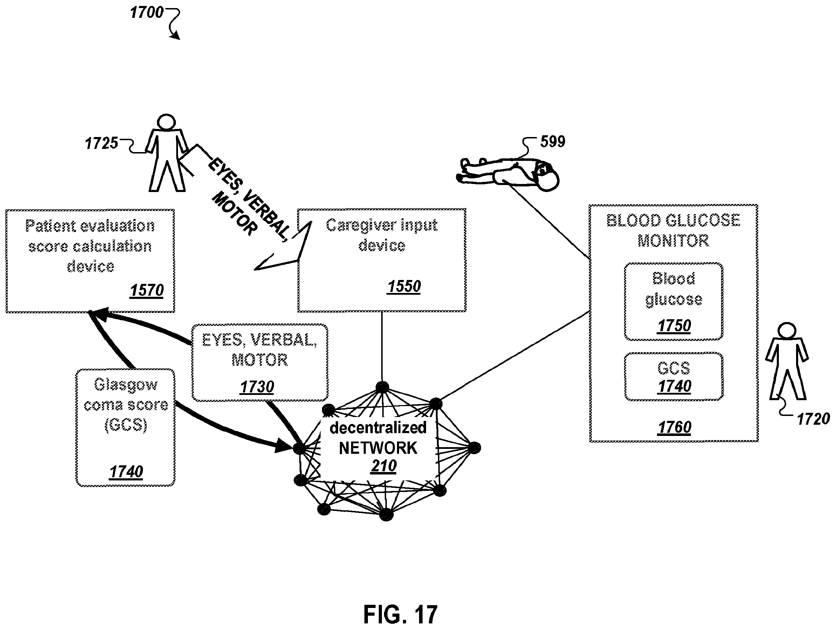

Referring to FIG. 17, an example of a Glasgow coma score determination and distribution using a decentralized network is shown A quantity of each component in FIG. 17 is an example only and other quantities of each, or any, component could be used.

In an implementation, as shown for example in the system 1700, the devices in the decentralized network 210 may include a single parameter physiological sensor configured to measure a single physiological parameter. The single parameter sensor may be a relatively small (e.g., a handheld sensor), portable, inexpensive, and/or disposable sensor with a limited display area primarily designed to display the parameter measured by the sensor. In general usage, such a sensor may have a relatively simple integrated circuit designed to enable measurement and display of the measured parameter. The single parameter sensor may also be configured to transmit a measurement to a larger medical device or publish a measurement to the decentralized network as a stand-alone node. In an implementation, such a single parameter sensor may be configured to receive a patient evaluation score from the decentralized network 210 and display that score along with the measured parameter. The device may be configured to subscribe to score data that is relevant to the measured parameter. The single parameter sensor may display the score received from the decentralized network 210 and/or a warning or advisement associated with the score.

An advantage of displaying the score at the single parameter sensor is that the score may be particularly relevant to this sensor. Therefore, caregivers may only need to know this score or associated warnings when this sensor is deployed. Thus, modifying a display of a larger device, such as a patient-monitor or patient-monitor/defibrillator, may be impractical because the score may not be of general relevance. In other words, there is limited display space on any medical device and generally this display space is relegated to the most commonly relevant physiological parameters. Typically scores, like the MEWS score discussed above or the GCS score discussed below, are available to a patient chart but not at a monitor. Enabling a display of such a score at the physiological sensor(s) most closely related to that score requires only a small alteration of the display on that physiological sensor and does not take away from display space on the patient-monitor. Furthermore, it is often difficult in a patient care space for all caregivers to have ready access to every device. These spaces are often crowded and cramped. Thus a distribution of the information over multiple devices in the space improves caregiver access.

As an example, the single parameter sensor may be a blood glucose monitor 1760 and the single measured parameter may be blood glucose 1750. A caregiver 1725 may provide eyes, verbal, and motor observational evaluations to a caregiver input device 1550. The patient evaluation score calculation device 1570 may subscribe to and receive the eyes, verbal, and motor observation data 1730 published to the decentralized network 210 by the caregiver input device 1550. The device 1570 may then calculate a Glasgow coma score (GCS) 1740 and publish this score to the decentralized network 210. The blood glucose monitor 1760 may subscribe to this score and display the score and/or warnings (e.g., an "altered mental state" warning) or advisements indicated by the score at the blood glucose monitor 1760. The caregiver 1720 may then have ready access to the GCS which may inform or contribute to altered care decisions for the patient 599. This point of care display of a pertinent patient score is particularly helpful in an EMS crew where there is a necessary division of labor and often enough noise and chaos to hamper easy communication between caregivers. The combination of the GCS 1740 with the blood glucose 1750 provides the impact of the blood glucose level on the patient. Such a transfer of information may be critical where an original device that measures the GCS leaves the patient ecosystem. The sensor, by retaining this value, may continue to provide important care information with regard to the parameter it is monitoring because of the easy access to the data through the decentralized network 210. For example, EMS medical devices and care guidance systems use scores like GCS to evaluate a patient's condition and make care decisions based on these assessment scores. For example, a transport priority, destination, etc. may be made based on one or more assessment scores. Having the score immediately available allows the caregiver more time to observe and treat the patient. Additionally, having a score calculated on an ongoing basis may allow caregivers to quickly observe decline or improvement in the patient's condition.

Referring to FIG. 18, an example of compensatory reserve index distribution using a decentralized network is shown. A quantity of each component in FIG. 18 is an example only and other quantities of each, or any, component could be used.

In an implementation, as shown for example in the system 1800, the patient medical device ecosystem may include a compensatory reserve index (CRI) sensor 1810. The CRI sensor applies an algorithm to pulse oximetry data that may quantitatively assess a person's capacity to compensate for blood loss (i.e. the person's compensatory reserve). The CRI may provide early detection of impending circulatory shock in trauma patients. When the CRI algorithm is applied, waveforms available from pulse oximetry (e.g., through photoplethysmography signals) may reveal how a person's physiological system is compensating for inadequate tissue oxygenation (i.e., shock) due to blood loss.

As shown in FIG. 18, the CRI sensor 1810 coupled to the patient 599 may form a node in the decentralized network 210 (or may transmit data to a larger medical device that forms a node on the decentralized network 210). The CRI is an indicator of shock and/or conditions leading to shock. For example, hypoperfusion (i.e., a lack of blood perfusion) as indicated by a CRI sensor reading may be a precursor to shock. Knowledge of this condition may be used to instruct the caregiver to continue scanning for additional areas of blood loss. CRI values indicative of blood loss may also indicate a tourniquet has not been applied correctly and blood loss is continuing The CRI sensor 1810 may publish the CRI score 1830 to the decentralized network 210 thus making that score available to other devices in the patient ecosystem forming nodes in the decentralized network 210. These may include the clinical guidance engine 228, the medical device 1540, a caregiver guidance device 1580, and an ultrasound imaging device 1820. In an implementation, the ultrasound imaging device 1820 may display the CRI score 1830. Such a display at the point of care for ultrasound imaging may provide a critical guide or alarm for a caregiver in determining the scope of an ultrasound scan. For example, this score may alert a caregiver to life-threatening internal or external bleeding. Based on a mechanism of injury, this information may accelerate a caregiver's ability to locate the life-threatening wound. Early location of a life-threatening condition may enable prehospital interventions like a needle decompression which in turn may save lives that may otherwise be lost in transit if such interventions not performed prehospital.

Referring to FIG. 19, an example of distributed system of medical care devices in a military medical care environment is shown. A quantity of each component in FIG. 19 is an example only and other quantities of each, or any, component could be used. For example, the system in FIG. 19 may be most likely associated with a role zero, role one, or role two crew but in some instances with a role three medical crew depending on the particular circumstances of a care environment.

Medical care provided in a military medical care environment presents several particular technical problems that are readily addressed with a decentralized network 210. In an implementation the decentralized network 210 may be a mesh network. As one issue, in many instances a military medical care environment requires radio silence. As a result, devices may be limited to wired communications that do not require radio wave transmissions. Additionally, this requirement may prohibit devices in a patient ecosystem from connecting to a remote server and utilizing such a server to facilitate the exchange of information or data analysis. As illustrated schematically in FIG. 19, the devices in the patient ecosystem and decentralized network 210 may need to operate in the absence of a connection to a computer and/or cellular network 1930. In an implementation, the decentralized network 210 may operate with or without such a connection as illustrated schematically by the configurable coupling 1999. Therefore, forming the decentralized network in the military environment may include forming without any Internet access for the decentralized network. For example, at least one medical devices used for patient care may include a Wi-Fi chip and may disable Wi-Fi frequency transmissions for all or a portion of a patient care event.

In an implementation, the decentralized network 210 may also determine the critical devices in the network for temporary radio silence as may be needed during military situations where wired communication may be recommended for a subset of devices in the network until such a time where radios can be used for communication. For example, the application layer of the devices in the decentralized network 210 and as used in a military application may downgrade the selection ranking of wireless sources such that no devices in the network 210 subscribe to data from these wireless sources. As such, the application layers may hold a first subscription paradigm and a second subscription paradigm. The first subscription paradigm may include subscriptions to wired and wireless publishing nodes. The second subscription paradigm may include subscriptions to wired nodes only. When wireless communications may be undesirable, the network 210 may enter a halt state where all nodes are notified of a temporary communications disruption and switch and hold on to the second data subscription paradigms engaged to avoid subscriptions to wireless sources until networks are resumed. When wireless data communications resume, the devices can revert back to the first paradigm and relatively seamlessly reconnect and revert to pre-interruption data flows.

As another consideration, the medical devices utilized in the military medical care environment may be specifically designed as portable, ruggedized, and light weight devices. For example, a ruggedized patient-monitor, patient-monitor/defibrillator, or portable ventilator may weigh less than 5 kg, less than 6 kg, or less than 4-7 kg. As ruggedized devices, these devices may be configured to withstand a particular shock g-force. For example, they may be configured to withstand a shock g-force of 65-85 g. The threshold g-force may correspond to a particular military specification. Often, military medical care is provided in a make-shift environment at the scene of injury for a soldier or civilian. Therefore, the equipment used to care for the patient must all be able to fit in a backpack or a pocket or be designed as a wearable device. This enables military medics to carry these devices over rugged and unpredictable terrain and potentially for long distances (e.g., more than a few feet for example). Military medical devices may also include night vision settings.

As shown for example in the system 1900 of FIG. 19, the ruggedized medical devices such as a ruggedized patient-monitor/defibrillator 1910 or a ruggedized portable ventilator 1920 are configured to fit inside of a backpack 1980 or 1985. Such devices are 1500-2000 cubic centimeters. Other devices may be pocket-sized such as the medical sensor 1965 designed to fit inside of a pocket 1960. A pocket-sized device may be 20-1500 cubic centimeters and weigh between 200-3000 grams depending on the location of the pocket. Devices that may be carried by a military medic may include one or more of an impedance threshold device coupled to a facemask or a mouthpiece, a portable and self-contained suction device, tourniquets, an intravenous delivery system, an airway management device, a diagnostic device comprising at least one of a blood pressure cuff, a pulse oximeter, a stethoscope, a thermometer, a medication kit, a stretcher, or a surgical kit, as examples only and not limiting of the disclosure. Other devices may be wearable such as the devices 1970 and 1975 shown in FIG. 19. These may include computing devices and/or medical sensors. The computing devices may include a heads-up device, a watch, a smartphone device, or a computer tablet. In general, the total weight of medical devices associated with any one military caregiver may be less than 45 kg as the medical gear is likely carried in addition to weapons, tactical equipment, and special clothing or protective wear.

In an implementation, it may be necessary for the medical and computing devices forming the patient ecosystem in a military setting to be distributed over the body of a caregiver or over the bodies of multiple caregivers at least in order to enable the caregivers to carry this equipment. Thus, at least two of the medical devices may be disposed at different locations on a caregiver or medic and/or distributed amongst two or more caregivers such as the caregivers 1940a and 1940b shown caring for the victim 1950. As discussed above, the devices may be pocket-sized, wearable, or a combination thereof.

As an additional factor, due to the acute unpredictability of a military care environment, particular equipment and particular caregivers may be unavailable, possibly suddenly. Therefore, a fast and nimble method of information exchange that does not require a remote server is particularly desirable. A decentralized network 210 provides such a method. Furthermore, if devices are physically distributed, as described above, then the singularized medical device that provides a hub of care, for example, may instead be a decentralized network of individualized sensors configured to share data and/or computing resources. This may be of particular importance when devices like the ruggedized patient-monitor/defibrillator 1910 or the ruggedized portable ventilator 1920 are unavailable to provide centralized sensor capabilities. In order for this decentralized network to function as a unified source of patient care, the decentralized network 210 provides an appropriate communications network.

The medical and computing devices 1965. 1970, 1975, and 1978 shown in FIG. 19 provide an example of a distributed ecosystem. For example, the devices in this distributed ecosystem may include a Bluetooth^{®} and/or Wi-Fi enabled SpO2 sensor 1985, a Bluetooth^{®} and/or Wi-Fi enabled continuous blood pressure sensor 1986, and/or a Bluetooth^{®} and/or Wi-Fi enabled EtCO2 sensor 1987. In an example, a first device node formed by the first device 1965 may collect a first physiological parameter and a second device node formed by the second device 1970 may collect a second physiological parameter. These nodes may publish the physiological parameters to the decentralized network 210 and a third device node formed by the third device 1978 different and physically separate from the devices 1965 and 1970 may subscribe to, receive, and monitor this data. As an example, the caregiver 1940b may need to leave the scene or be suddenly unable to provide care due to an injury. In this case, the third device 1978 may exit the decentralized network but a fourth device 1975 may enter or already be in the decentralized network and may take over the monitoring of the physiological parameters provided by the devices 1970 and 1965 without an interruption in care to re-configure devices.

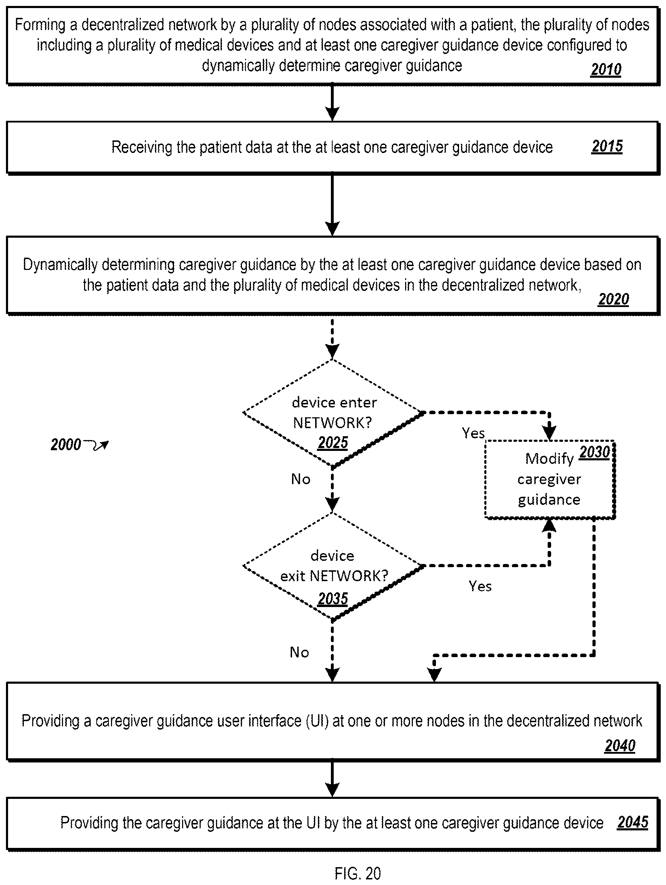

Referring to FIG. 20, an example of a method of determining and modifying caregiver guidance based on a decentralized network device inventory is shown. The method 2000 is, however, an example only and not limiting. The method 2000 can be altered, e.g., by having stages added, removed, rearranged, combined, and/or performed concurrently.

Emergent medical care focuses, at least initially, on timely management of a patient's presenting conditions. Such medical care may occur, for example, in response to a 911 call or in a military setting. The goal of timely managing the patient's presenting conditions is to provide interventions that stabilize the patient and keep the patient alive long enough for the patient to receive treatments for diagnosed etiologies of the presenting conditions. As an example, a person under the care of emergency medical services may present with cardiac arrest. The emergent care may include defibrillation to restore a normal heart rhythm. This intervention for cardiac arrest most likely does not depend upon the specific etiology of the cardiac arrest and may stabilize and keep the patient alive long enough to receive treatments for the etiology of the cardiac arrest. For example, following a defibrillation intervention, treatments for the etiology of cardiac arrest, such as surgery and/or medications may vary depending on the etiology or diagnosis. For example, cardiac arrest may be consistent with various diagnoses such as coronary artery disease, valvular heart disease, or a congenital heart defect.

Guidance for a caregiver and/or a medical device is likely to improve the efficacy and efficiency of interventions provided during emergent care. Further and more substantial improvements in efficacy and efficiency are realized by tailoring the guidance to the physiologic status of the patient. Monitoring, analyzing, and evaluating physiologic data relevant to the patient presentation provides adaptable and data-driven guidance that is sensitive to the particular context of the patient's physiologic status. The guidance also determines, monitors, and updates the parameters of these interventions based on the physiologic data from the patient. Thus, while the guidance may enable decisions regarding which interventions to provide but it is not limited to such decisions. Further, in some cases, the physiologic data that is monitored, analyzed, and evaluated may provide indications of etiology and may enable interventions and treatments directed at that etiology.

The clinical guidance engine 228 may provide caregiver guidance tailored to the context of presenting conditions of a patient before an accurate diagnosis is likely or in some cases before such diagnosis is even possible. For example, the clinical guidance engine 228 may subscribe to and receive physiological and other medical data for the patient via the decentralized network 210 and, based on this information, identify therapeutic interventions tailored to alleviate physiological conditions indicated by the data which in some cases pose an acute and immediate risk to the patient's life. Once the patient is stabilized, the clinical guidance engine 228 may provide further guidance in identifying a diagnosis, or specific root cause, and identifying and implementing treatments based on the diagnosis. Additionally, the clinical guidance engine 228 may monitor the patient for indicators of a resumption of an acute and possibly life-threatening medical condition.

The clinical guidance engine 228 may provide guidance based at least in part on the specific medical devices available to treat and monitor the patient. The decentralized network 210 enables the generation of an inventory and the evaluation of that inventory by the clinical guidance engine 228. The inventory may be a database structured inventory published to the network. In some implementations, this inventory may be a blockchain ledger. For example, the clinical guidance engine 228 may evaluate a protocol based on an inventory of available equipment. Based on the available equipment, the clinical guidance engine 228 may provide instructions on preparing this equipment for deployment, provide instructions for use, or modify a protocol based on the inventory.

In various implementations, the clinical guidance engine 228 may provide the caregiver guidance at a caregiver guidance interface device 2130. For example, the caregiver guidance interface device 2130 may be a mobile device, such as the computer tablet shown in FIG. 21. The caregiver guidance interface device 2130 may enable the caregiver to provide input to the clinical guidance engine 228 such as spoken input or input entered at a touchscreen. The caregiver guidance interface device 2130 may also enable the caregiver to receive output from the clinical guidance engine 228 such as prompts, instructions, reminders, etc. as visible, audible, and/or tactile output. In various implementations, the caregiver interface device may include one or more of a tablet, a smartphone, a heads-up display device, a watch and/or a laptop. Additionally or alternatively the caregiver may receive guidance via a speaker, an earpiece, a medical device user interface or combinations thereof.

For the method 2000, at the stage 2010, the method includes forming the decentralized network by the plurality of nodes including at least one caregiver guidance device configured to dynamically determine caregiver guidance. The caregiver guidance device may be a mobile computing device (e.g., the caregiver guidance interface device 2130 in FIGS. 21-23) or may be a medical device. The clinical guidance engine 228 may be incorporated into one or more of the mobile device or a medical device to generate and provide the caregiver guidance.

At the stage 2015, the method 2000 includes receiving the patient data at the at least one caregiver guidance device.

At the stage 2020, the method 2000 includes dynamically determining caregiver guidance by the at least one caregiver guidance device based on the patient data and the plurality of medical devices in the decentralized network 210. For example, the clinical guidance engine 228 may determine or identify caregiver guidance based on a first group of medical devices available for patient care as evidenced by network messages to the decentralized network 210.

At the stage 2025, the method 2000 optionally includes detecting whether or not a medical device and/or computing device has entered the decentralized network 210. Similarly, at the stage 2035, the method 2000 optionally includes detecting whether or not a medical device and/or computing device has exited the decentralized network 210. In either of these cases, the method 2000 may include modifying previously determined caregiver guidance at the stage 2030 in response to the change in the medical device ecosystem for the patient. For example, if a medical device in the first group of devices exits the decentralized network 210, the clinical guidance engine 228 may modify the determined guidance to account for the unavailability of the exited device. Similarly, if a new device enters the decentralized network the clinical guidance engine 228 may modify the determined guidance to account for the availability of the new device.

At the stage 2040, the method 2000 includes providing a caregiver guidance user interface (UI) at one or more nodes in the decentralized network 210. In an implementation, the caregiver guidance UI may be disposed at a same device as the clinical guidance engine 228, a different device, or a combination thereof. One or more of the clinical guidance engine 228 and the caregiver guidance UI may be distributed resources across more than one device in a patient care ecosystem. Thus the caregiver guidance UI may be provided at one or more nodes of the decentralized network 210. At the stage 2045, the method 2000 includes providing the caregiver guidance at the caregiver guidance UI by the at least one caregiver guidance device.

Referring to FIG. 21, an example of a caregiver guidance UI is shown. A quantity of each component in FIG. 21 is an example only and other quantities of each, or any, component could be used.

The clinical guidance engine 228 (either as a component of the caregiver guidance interface device 2130 and/or communicatively coupled to the caregiver guidance interface device 2130) may receive network messages and/or may couple to a services library 2110. One or more of the devices in the decentralized network 210 may include the services library 2110. For example, the edge server 260 and/or one or more of the patient charting device 250, the CAD interface device 253, and the mobile device 225 may include the services library 2110. The caregiver guidance interface device 2130 may be a same device as one of 260, 250, 253, or 225 or may be an additional device or group of devices in the decentralized network 210. The caregiver guidance interface device 2130 may either communicatively couple to or include one or more of the clinical guidance engine 228 and the services library 2110.

The services library 2110 may include a medical device drivers library 2120, a medical device operation and assembly instruction library 2122, a medical device specifications library 2124, and/or a medical device identification library 2126. In an implementation, the medical device drivers enable nodes in the decentralized network 210 to communicate with the medical device(s) 2190 via the decentralized network 210 in order to provide data and/or instructions to the medical devices, which may include closed loop control instructions, and/or to request and receive data and information from the medical devices. The medical device operation and assembly instructions provide written, verbal, and/or graphic instructions usable by the caregiver interface device to provide instructions to the caregiver on how to use and assemble a particular medical device. The medical device specifications provide operational, data reporting and formatting, and self-testing specifications for a particular medical device. The medical device identification information provide make and model information in order to properly reference the other libraries with regard to other specific information.

The services library 2110 may include medical device information for devices that are not included in the decentralized network 210. The services library 2110 may tag a subset of the EMS medical device information as medical device information corresponding to the medical devices in the decentralized network 210 based on the published network messages. In this manner, the services library may limit the information provided to the caregiver guidance device to that pertaining to the tagged subset.

Based on the network messages and/or information stored in the services library 2110, the clinical guidance engine 228 may provide a connected devices window 2140 at the caregiver guidance UI 2135 that displays the various connected devices and equipment. The clinical guidance engine 228 may update the connected devices window 2140 as devices enter and exit the decentralized network 210. The connected devices window may include a device status window 2141, a device search control 2142, and/or a device verification control 2143. The device status window 2141 may display an amount of battery life, connection status, and a unique name for each of the medical devices in the decentralized network 210. The device verification control 2143 may enable the caregiver guidance interface device 2130 to cause an indicator to flash at a particular item of medical equipment to identify the device and/or confirm that the medical device is in fact present at the scene and/or in use on the patient. In some examples where multiple medical events are occurring at the same time, such as a scene of a mass casualty or collision, there may be multiple rescue teams operating multiple medical treatment devices within close proximity of one another. In such situations, it may be easy to mix up mobile devices network with respective medical treatment devices. Therefore, in some implementations, when the device verification control 2143 is selected, the caregiver guidance interface device 2130 may generate an instruction signal that causes all connected medical equipment to generate an indication of being network with the caregiver guidance interface device 2130. In some implementations, upon receiving a verification signal from the caregiver guidance interface device 2130, the medical equipment may output a visual and/or audible indication of being connected to the caregiver guidance interface device 2130. In some examples, the indication can be a flashing light and/or a tonal sound pulse. The device search control 2142 may enable a display at the mobile device 2130 to display wireless communication links via the decentralized network that are available between the caregiver guidance interface device 2130 and various medical equipment.

In an implementation, the caregiver guidance UI may provide a graphical representation 2150 of the devices available in the decentralized network 210. In some examples, the UI may provide a menu that asks a caregiver if they want to add a device to the display based on the availability of the device in decentralized network 210. The caregiver may then manually add devices to the graphic display 2150.

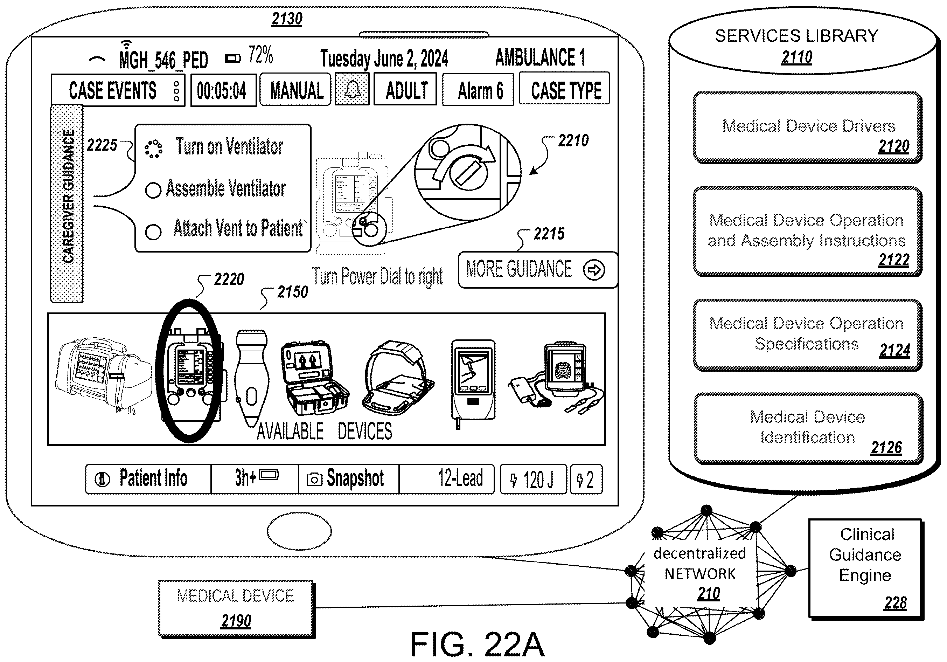

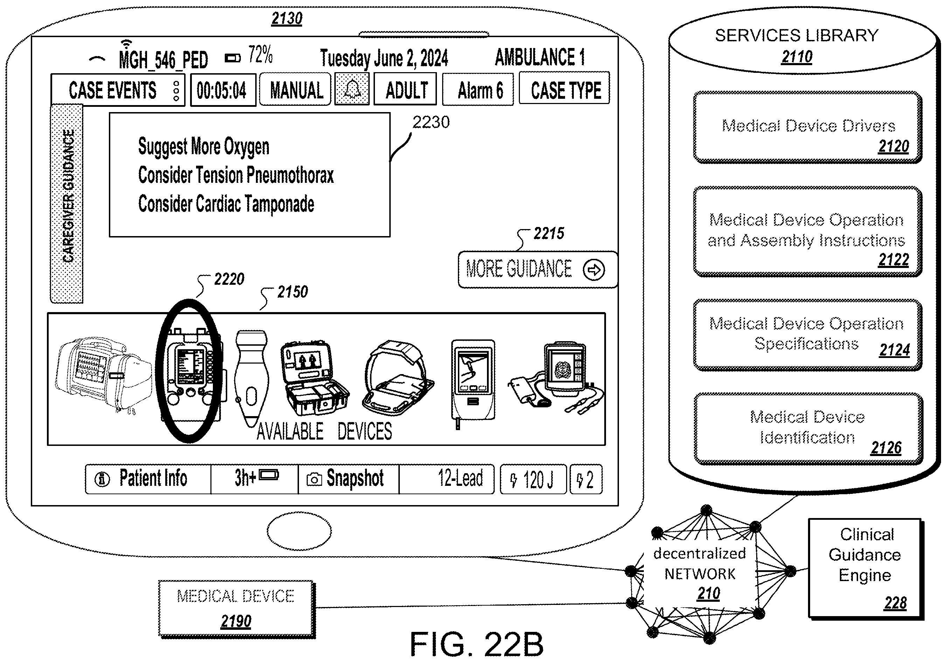

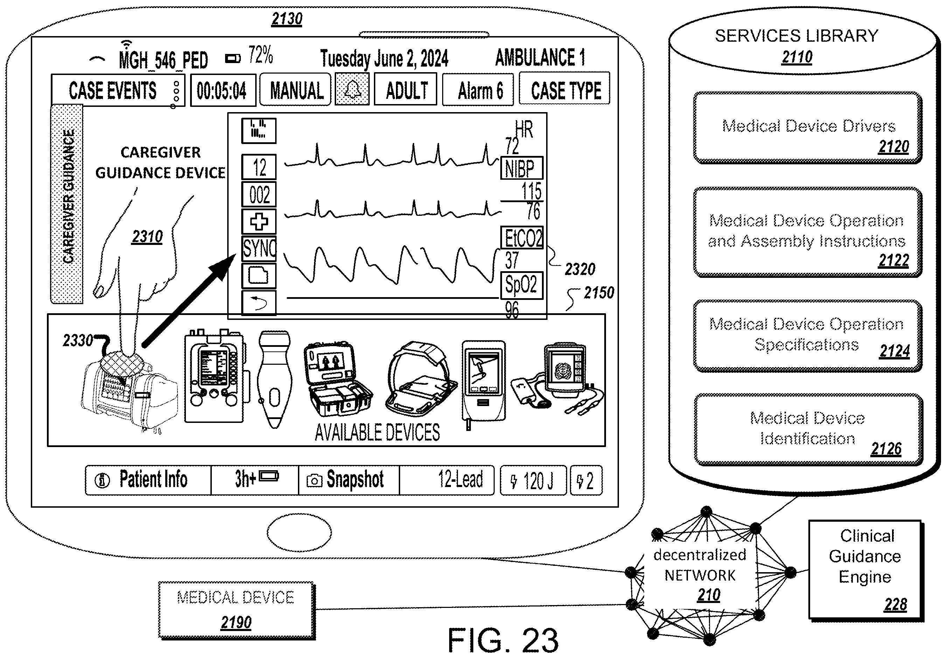

Referring to FIGS. 22A, 22B, and 23, examples of information provided at a caregiver guidance user interface are shown. A quantity of each component in FIGS. 22A, 22B, and 23 is an example only and other quantities of each, or any, component could be used.

Using the information from the services library 2110 and information gathered via the decentralized network 210 from the various medical devices and computing devices in the decentralized network 210, the clinical guidance engine 228 may provide various forms of caregiver guidance at the caregiver guidance interface device 2130.

For example, as shown in FIG. 22A, the information may include assembly graphics 2210 and a selectable control 2215 to request further guidance. The caregiver guidance UI 2135 may include activity sequence instructions 2225 and may indicate a current stage of progress for those instructions. The caregiver guidance UI 2135 may further include an indicator 2220 that identifies the particular medical device corresponding to the instructions. In an example, display 2150 may be interactive and provide selectable controls that enable a caregiver to request instructions and guidance for a particular medical device.

As another example, as shown in FIG. 22B, the caregiver guidance UI 2135 may provide suggested or recommended patient treatments and/or suggested diagnoses 2230. The clinical guidance engine 228 may determine these suggestions and/or recommendations based on physiological information, caregiver input for observational information, patient evaluation scores, etc. as received via the decentralized network 210. As discussed above, the clinical guidance engine 228 may dynamically adjust the suggestions and/or recommendations 2230 based on changes to the inventory of medical devices in the patient ecosystem.

As a further example, as shown in FIG. 23, the clinical guidance engine 228 may provide a user control 2330 associated with a graphical representation of a medical device in the decentralized network 210. The clinical guidance engine 228 may receive or capture a selection of the user control (e.g., by a touchscreen gesture 2310). In response to the selection of the user control, the caregiver guidance interface device 2130 may subscribe to operational interface information for the medical device associated with the user control 2330. The medical device may publish to the decentralized network 210 sufficient information for the clinical guidance engine 228 to provide the operational interface information 2320 at the caregiver guidance interface device 2130. In an implementation, the operational interface information 2320 may visually replicate the appearance of the medical device's operational interface or may provide the information as a second visual representation so as to provide the same information but with a different visual format.

Referring to FIG. 24, an example of computing and medical device nodes within a transport vehicle is shown. A quantity of each component in FIG. 24 is an example only and other quantities of each, or any, component could be used.

In an implementation, as shown by the example of the system 2400, the decentralized network 210 may include nodes formed by one or more mobile computing devices having the network protocol communication stack provided by the network communications driver 399. Each device may be configured to publish data to and subscribe and receive data from the decentralized network 210. These devices may include a patient charting application and a CAD-NAV application. In an implementation, the patient charting application is on a patient charting device 250 and the CAD-NAV application is on the CAD-NAV device 253. In an example, the CAD-NAV device 253 is a separate device from the patient charting device 250. This separation may be functionally necessary because the EMS driver 2430 needs the CAD-NAV device 253 in the front section 2410 of an EMS vehicle in order to take advantage of the navigation information provided by the CAD-NAV application. The EMS crew 2440 needs to use the patient charting device 250 in the rear section 2420 of the EMS vehicle or outside the EMS vehicle in order to document and record patient data and to access possible caregiver guidance provided by the device 250. Unlike the decentralized network connection, a direct connection between the CAD-NAV device 253 and the patient charting device 250 may not allow any of the data sources not in direct communication to the device 250 to publish any new information to the charting device 250. Additionally, without the decentralized network, new information entered into the charting device 250 may not propagate to the entire suite of devices involved in patient care thus limiting or preventing this information from informing and improving the care provided by this suite of devices. Further, in the absence of a decentralized network, each individual paired connection between two devices must be established and maintained. Transport of information beyond the pair of connected devices may require sequential transfers of information and multiple individual paired connections. For example, a Bluetooth^{®} enabled multipoint connection may allow one device to choose between two sources of data and access both of those sources in sequence or in an alternating fashion. In contrast, the decentralized network, such as a mesh network, enables bi-directional exchanges of information between multiple nodes without requiring any sequencing or alternation.

**In** some examples, the caregiver guidance interface device 2130 may be the same device as the patient charting device 250. Thus the patient charting device 250 may need to be within reasonable proximity to the patient 599 and/or the caregiving crew 2440. In some cases, the two devices may exchange information in order to improve care provided to the patient 599.

To provide a complete and accurate record of each encounter that includes patient and encounter information and that provides comprehensive and adapted caregiver guidance, an EMS caregiver may complete an electronic patient care record (ePCR) using the patient charting application. ePCRs include data fields configured to store a comprehensive set of patient and encounter information according to a schema that controls the structure of the data provided to the digital record. In some examples, the schema may be a multi-agency standard that provides a compliance architecture to allow transfer of data and data interoperability between individual agency systems and enables entry of data in a centralized database. The patient charting application may exchange information with the medical devices and/or other computing devices to populate fields of the ePCR, to provide caregiver guidance in conjunction with ePCR population, and to share information that may be primarily or most readily available from the ePCR. For example, the ePCR may be a source of demographic information, incident information, insurance information, and health history information gathered by the caregiver and/or provided to the ePCR from a source like the CAD-NAV device. The ePCR may also be a source of caregiver observation information based on patient triage protocols executed by the caregiver. These may include, for example, but are not limited to AVPU, eyes-verbal-motor information, symptom-allergy-medication-pertinent history (SAMPLE) or other information gathered based on a caregiver's personal observation.

In an implementation, the patient charting device may receive patient data from other devices in the decentralized network and map this data to one or more data fields in the ePCR and store the patient data in the ePCR. This may automate data population with benefits of improved accuracy due to a lack of human intervention and the contemporaneous nature of the data (as compared with entries by caregivers after an incident as ended). Further, the timely recordation of data may enable the provision of the ePCR data to other devices in the decentralized network to facilitate care. For example, devices providing treatments that depend on body weight, patient age, patient gender, and/or patient history may subscribe to and receive ePCR data and determine treatments accordingly. In an implementation, devices that may receive data from and/or provide data to the ePCR may include, but are not limited to, at least one of a patient-monitor, a patient-monitor/defibrillator, a portable ventilation device, an automated external defibrillator (AED), or an automated compression device. In an implementation, the patient charting device 250 may publish one or more of patient demographic data, SAMPLE data, and/or any other data recorded in the ePCR or provided to the patient charting device 250. In this manner, the patient charting device 250 and the patient charting application do not merely serve a record keeping function but dynamically and actively contribute information to the ecosystem of devices providing patient care in a manner that may actively guide or determine that care.

In an implementation, the CAD-NAV device 253 may include a positioning system such as a Global Positioning System (GPS), for example. The driver 2430 may use the CAD-NAV application for navigation assistance. Additionally, the CAD-NAV application may perform calculations about vehicle speed, the travel time between locations, and estimated times of arrival. The CAD-NAV device may provide the navigation and calculations with or without a connection to the cloud CAD service 257. With the connection to the cloud CAD service 257, the device 253 may provide an interface that enables the cloud CAD service 257 to assign patient incidents, or calls, to the EMS vehicle. These incidents may be pre-scheduled (e.g., an inter-hospital transfer, transportation for medical services like dialysis, etc.) or emergency (i.e., not prescheduled and resulting from, for example, a call to 911). The application may provide various mapping services including but not limited to a map with a route to the target destination, including address and directions, controls for setting time stamps for the incident, viewing alerts, viewing notes, and/or toggling the map view (for example between zooming to the destination location, viewing the entire route, and following the vehicle on the map).

When the CAD interface device 253 is in communication with the cloud CAD service 257, the CAD interface application may provide dispatch information received from the CAD service 257 and enable communications between the EMS driver and the CAD service 257. For example, the CAD interface application may provide information including but not limited to information about incident identifiers and type, the location for the incident, notes and alerts for the incident, patient information for the patient associated with the incident (e.g., referring doctor, prior incidents, home address, employer information, and/or insurance or medical bill payor information, time stamps entered for the incident, and/or duration on each unit status for the incident, according to embodiments of the present disclosure. The application may further allow the EMS driver to register a response accepting a particular incident assignment and may display a set of incidents that have been assigned to the unit throughout a shift, including open and completed incidents along with status information for each incident.

Based on communications via the decentralized network 210, the patient charting device 250 may receive information from the CAD service via the CAD-NAV device without a communicative coupling between the patient charting device and a remote patient charting server. This may provide a faster and more efficient means of information exchange and also more accurate because database information does not have to be sorted and tagged in order to ensure that the data corresponds to the correct incident and patient.

In an implementation, the patient charting device may receive location information from the CAD-NAV device. The location information may include a current EMS vehicle GPS location and a patient transport destination. The CAD-NAV application and/or the patient charting application may determine a travel time from the current EMS vehicle GPS location to the patient transport destination. With this information, the patient charting application may generate a caregiver recommendation based at least in part on the location information and provide the caregiver recommendation via a caregiver guidance user interface (UI) 2135. The caregiver guidance UI 2135 may be disposed at the patient charting device 250 or another medical device or computing device in the decentralized network 210. In an implementation, the clinical guidance engine 228 may subscribe to the data from the CAD-NAV device 253 and/or the patient charting device 250 and use the location information to determine caregiver guidance.

The caregiver guidance based on location may be based in part on a sorting of patient care protocol tasks into on-scene tasks prior to transport and in-ambulance tasks during transport. Based on the distance to a destination hospital along with the tasks and the patient state, the patient charting device 250 may generate a caregiver alert to begin patient transport and transition from the on-scene tasks to the in-ambulance tasks. For example, based on a patient's injury status, they may need several treatments. If they are close to a hospital (i.e., within 5-10 minutes), then it may be more beneficial to provide the minimal treatments on scene and expedite transport. However, if the delivery to the hospital will be delayed, due to traffic or weather, for example, or if the time to the hospital is more than 10 minutes, for example in a rural area, then the caregiver guidance may recommend more treatments on scene. Where it improves patient outcomes, it may be beneficial to reduce on-scene care time and expedite transport. Tracking vehicle locations and transport times along with patient care procedures done on-scene or on-route to the destination hospital and patient outcomes may provide quality control metrics to guide EMS agencies in refining protocols based on transport times and distances and may enable real-time alerts to caregivers to either stay on scene or transport.

In an implementation, the patient charting application or the caregiver guidance UI may adjust care delivery parameters where these parameters are sensitive to time and distance. For instance, the patient charting application or the clinical guidance engine 228 may change a recommended delivery rate or volume or a medication dosage based on the travel time to the patient transport destination.

In an implementation, the decentralized network communication between the CAD-NAV device 253 and the patient charting device 250 may also enable prioritized diversions due to ambulance stacking. The patient charting device 250 may receive information via the decentralized network 210 about both the current medical status of a patient (e.g., from the medical devices) and the dispatch status and location of ambulances (e.g., from the CAD-NAV device 253). This may enable triage decisions about destinations based not only on distance and patient condition but also on emergency room entry times. Patients may be diverted based on care needs and the acuity of those needs.

In an implementation, the patient charting application may document a particular patient condition in the ePCR and flag that condition as requiring specialty care at a destination hospital (e.g., a cardiac catheterization lab, a psychiatric facility, a specialized burn or trauma unit, as examples not limiting of the disclosure). The patient charting device 250 may publish this information to the decentralized network 210 and the CAD-NAV device may subscribe and receive this information along with other medical devices 2490 available in the decentralized network 210. The other medical devices may adjust care based on this information and concurrently, the CAD NAV device may modify an original patient transport destination based on this identified patient need. If a connection is available to the cloud-based CAD service 257, the CAD-NAV device may report this change to the cloud-based CAD service 257. The CAD-NAV device may determine this destination adjustment based on distance and determine the closest appropriate facility. The CAD-NAV device may then publish this information to the decentralized network 210. Any medical device 2490 that is able to transmit information to a destination hospital may then initiate that transmission to the newly identified destination. Concurrently, the patient charting application implemented by the patient charting device 250 may automatically record this information in the ePCR along with updated any relevant insurance coverage information based on the new destination. Automated entry into the ePCR of a change in destination based on an original destination not being the closest appropriate facility expedites billing procedures as well. Without this automated entry, billing services need to manually determine from the patient medical data in the ePCR that a facility was or was not a closest appropriate facility.

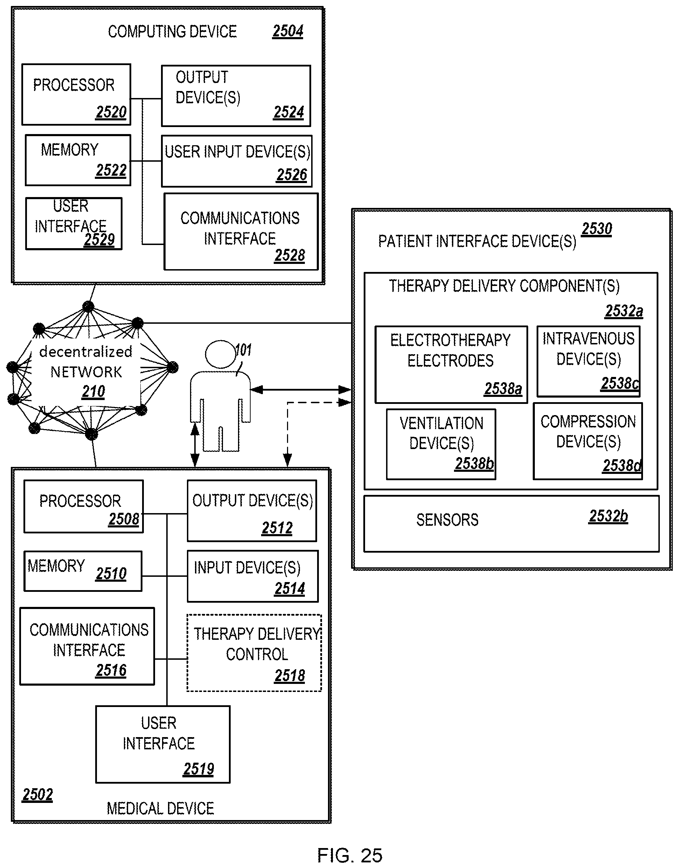

Referring to FIG. 25, examples of components of various devices discussed herein are shown schematically. These devices may include one or more medical devices 2502, one or more computing devices 2504, and one or more patient interface devices 2530. One or more of the medical devices 2502, the computing devices 2504, and the patient interface devices 2530 may form nodes in the decentralized network 210 as described herein. The computing device 2504 may include a user interface 2529 (e.g., a touchscreen or other display device and/or audio output device) and/or the medical device may include a user interface 2519 (e.g., a touchscreen or other display device and/or audio output device). In an implementation, the medical device 2504 may monitor and/or provide diagnostic care for the patient 101 /or provide medical therapy as an intervention via the patient interface devices 2530. The computing device 2504 may be a portable computing device such as a tablet, laptop, smartphone, watch, heads-up device, other wearable device, or combinations thereof. The computing device 2504 may be adapted to function as a medical device or be a display screen of an additional medical device such as when monitoring continuous NIBP measurements. In an implementation, the computing device 2504 may not be a therapeutic medical device configured to deliver medical therapy to the patient. In such an implementation, the computing device 2504 may be limited to patient-monitoring and/or diagnostic care, such as tracking a patient status via one or more display screens and/or controlling one or more functional operations at the medical treatment device 2502 as described in the embodiments herein.

The medical device 2502 and one or more computing devices 2504 may be communicatively coupled via the decentralized network 210 via wired and/or wireless communications links. The wired communications links may include a wired electrically coupling, an optical coupling via an optical cable, etc. The wireless communications link may include coupling via a radio frequency or other transmission media and/or via a network such as a local area network, an ad hoc network, a mesh network, a cellular and/or another communications network, a computer network, etc. The communications link as described herein may utilize protocols such as, for example, 802.11, ZigBee^{®}, Bluetooth^{®}, etc. The communications link may include near field communications which may be implemented via a communications RFID tag. In an implementation, one or more of the computing device 2504 or the medical device 2502 may include a communications link to a cellular network, a satellite network, and/or a computer network (e.g., an Internet Protocol (IP) network). In various implementations, the communicative couplings described herein may provide secure and/or authenticated communications channels. In an implementation, the devices described herein may encrypt and/or decrypt the data transmitted and/or received via the communicative couplings.

In some embodiments, the memory 2510 of the medical treatment device 2502, and similarly memory 2522 of mobile computing device 2504, may include a data store, also referred to as a data repository, and corresponding data storage circuitry including one or more of non-transitory or non-volatile computer readable media, such as flash memory, solid state memory, magnetic memory, optical memory, cache memory, combinations thereof, and others. The data store can be configured to store executable instructions and data used for operation of the medical treatment device 2502 and/or computing device 2504. In certain implementations, the data storage processing circuitry, in combination with the data store, can include executable instructions that, when executed on the processor 2508 and/or 2520, are configured to cause the at least one processor 2508 and/or 2520 to perform one or more functions of the medical device(s) 2502 and the computing device 2504, as described herein.

In FIG. 25, the components 2508, 2510, 2512, 2514, 2516, 2518, and 2519 are communicatively coupled (directly and/or indirectly) to each other for bi-directional communication. Similarly, the components 2520, 2522, 2524, 2526, 2528, and 2529 are communicatively coupled (directly and/or indirectly) to each other for bi-directional communication. In some implementations, the components 2508, 2510, 2516, and/or 2518 of the medical device 2502 may be combined into one or more discrete components and components 2516 and/or 2518 may be part of the processor 2508. The processor 2508 and the memory 2510 may include and/or be coupled to associated circuitry in order to perform the functions described herein. Additionally, the components 2520, 2522, and 2528 of computing device 2504 may be combined into one or more discrete components and component 2528 may be part of the processor 2520. The processor 2520 and the memory 2522 may include and/or be coupled to associated circuitry in order to perform the functions described herein.

In some implementations, the medical device 2502 may include the therapy delivery control module 2518. For example, the therapy delivery control module 2518 may be an electrotherapy delivery circuit that includes one or more high-voltage capacitors configured to store electrical energy for a pacing pulse or a defibrillating pulse. The electrotherapy delivery circuit may further include resistors, additional capacitors, relays and/or switches, electrical bridges such as an H-bridge (e.g., including a plurality of insulated gate bipolar transistors or IGBTs), voltage measuring components, and/or current measuring components. As another example, the therapy delivery control module 2518 may be a compression device electro-mechanical controller configured to control a mechanical compression device. As a further example, the therapy delivery control module 2518 may be an electro-mechanical controller configured to control drug delivery, temperature management, ventilation, chest compressions, and/or other type of therapy delivery.

The medical device 2502 may incorporate and/or be configured to couple to one or more patient interface devices 2530. The patient interface devices 2530 may include one or more therapy delivery component(s) 2532a and one or more sensor(s) 2532b. Similarly, the computing device 2504 may be adapted for medical use and may incorporate and/or be configured to couple to the one or more patient interface device(s) 2530.

The sensor(s) 2532b may include sensing electrodes and/or other sensors as shown for example in FIG. 2A. In some implementations, the information obtained from the sensors 2532b can be used to generate information displayed at the medical device 2502 and/or at the computing device 2504. Sensing electrodes may include cardiac sensing electrodes. The cardiac sensing electrodes may be conductive and/or capacitive electrodes configured to measure changes in a patient's electrophysiology to measure the patient's ECG information. The sensing electrodes may further measure the transthoracic impedance and/or a heart rate of the patient. The sensors 2532b may include spirometry sensors, flow sensors, pressure sensors, oxygen and/or carbon dioxide sensors such as, for example, one or more of pulse oximetry sensors, oxygenation sensors (e.g., muscle oxygenation/pH), O2 gas sensors and capnography sensors, impedance sensors, and combinations thereof. The sensors 2532b may include temperature sensors such as an infrared thermometer, a contact thermometer, a remote thermometer, a liquid crystal thermometer, a thermocouple, a thermistor, etc. and may measure patient temperature internally and/or externally. The sensors 2532b may include chest compression sensors such as one or more motion sensors including, for example, one or more accelerometers, one or more force sensors, one or more magnetic sensors, one or more velocity sensors, one or more displacement sensors, etc. The chest compression sensors may provide one or more signals indicative of the chest motion to the medical device 2502 via a wired and/or wireless connection. The chest compression sensors may be, for example, but not limited to, a compression puck, a smartphone, a hand-held device, a wearable device, etc. The chest compression sensors may be configured to detect chest motion imparted by a rescuer and/or an automated chest compression device (e.g., a belt-based system, a piston-based system, etc.). The chest compression sensor may provide signals indicative of chest compression data including displacement data, velocity data, release velocity data, acceleration data, force data, compression rate data, dwell time data, hold time data, blood flow data, blood pressure data, etc. In an implementation, sensing electrodes and/or electrotherapy electrodes 2538a may include or be configured to couple to the chest compression sensor.

In various implementations, the sensors 2532b may include one or more sensor devices configured to provide sensor data that includes, for example, but not limited to electrocardiogram (ECG), blood pressure, heart rate, respiration rate, heart sounds. lung sounds, respiration sounds, end tidal CO₂, saturation of muscle oxygen (SMO₂), oxygen saturation (e.g., SpO₂ and/or PaO₂), cerebral blood flow, point of care laboratory measurements (e.g., lactate, glucose, etc.), temperature, electroencephalogram (EEG) signals, brain oxygen level, tissue pH, tissue fluid levels, images and/or videos via ultrasound, laryngoscopy, and/or other medical imaging techniques, near-infrared spectroscopy, pneumography, cardiography, and/or patient movement. Images and/or videos may be two-dimensional or three-dimensional, such a various forms of ultrasound imaging.

The one or more therapy delivery components 2532a may include electrotherapy electrodes (e.g., the electrotherapy electrodes 2538a), ventilation device(s) (e.g., the ventilation devices 2538b), intravenous device(s) (e.g., the intravenous devices 2538c), compression device(s) (e.g., the compression devices 2538d), etc. For example, the electrotherapy electrodes 2538a may include defibrillation electrodes, pacing electrodes, and combinations thereof. The ventilation devices 2538b may include a tube, a mask, an abdominal and/or chest compressor (e.g., a belt, a cuirass, etc.), etc. and combinations thereof. The intravenous devices 2538c may include drug delivery devices, fluid delivery devices, and combinations thereof. The compression devices 2538d may include mechanical compression devices such as abdominal compressors, chest compressors, belts, pistons, and combinations thereof. In various implementation, the therapy delivery component(s) 2532a may be configured to provide sensor data and/or be coupled to and/or incorporate sensors. For example, the electrotherapy electrodes 2538a may provide sensor data such as transthoracic impedance. ECG, heart rate, etc. Further the electrotherapy electrodes 2538a may include and or be coupled to a chest compression sensor. As another example, the ventilation devices 2538b may be coupled to and/or incorporate flow sensors, gas species sensors (e.g., oxygen sensor, carbon dioxide sensor, etc.), etc. As a further example, the intravenous devices 2538c may be coupled to and/or incorporate temperature sensors, flow sensors, blood pressure sensors, etc. As yet another example, the compression devices 2538d may be coupled to and/or incorporate chest compression sensors, patient position sensors, etc. The therapy delivery control module 2518 may be configured to couple to and control the therapy delivery component(s) 2532a, respectively.

The patient interface devices 2530 may provide patient data. The display screens of the medical device 2502 and/or the computing device 2504 may display the patient data. For example, the medical device 2502 may process signals received from the sensor(s) 2532b and/or the therapy delivery component(s) 2532a to determine the patient data. Similarly, the computing device 2504 may process signals received from the patient interface devices 2530 to determine patient data.

The wearable cardioverter defibrillator (WCD) 205 (e.g., as shown in FIG. 2A) is external and wearable by a patient around the patient's torso. Such a wearable device can be, for example, capable of and designed for moving with the patient as the patient goes about their daily routine. In one example scenario, a WCD 205 can be worn nearly continuously or substantially continuously for a week, two weeks, a month, two or three months, or more at a time. During the period of time in which a WCD 205 is worn by the patient, the WCD can be configured to continuously or substantially continuously monitor the vital signs of the patient and, upon detecting a treatable arrhythmia, deliver one or more therapeutic electrical pulses to the patient. Such therapeutic shocks can be cardioversion defibrillation shocks. In some embodiments, the WCD 205 may instead be configured as a therapeutic device configured deliver other and/or additional types of shocks, such as pacing, non-cardioversion defibrillation shocks, or transcutaneous electrical nerve stimulation (TENS) pulses.

The WCD 205 can include one or more of the following: a garment configured to be worn about the patient's torso, one or more externally-worn sensors configured to output one or more physiological signals (e.g., one or more ECG sensing electrodes, motion sensors, cardiovibration sensors, tissue fluid sensors, etc.), one or more therapy electrodes, a medical device controller, a connection pod, or a patient interface pod. In examples, at least some of the components of the WCG 205 can be configured to be mounted on or affixed to the garment, such as by mating hooks, hook-and-loop fabric strips, receptacles (e.g., pockets), and the like. For instance, sensing electrodes may be mounted on the garment by hook-and-loop fabric strips on the electrodes and the garment, and therapy electrodes may be mounted on the garment by being inserted into receptacles of the garment. In examples, at least some of the components of the WCD 205 can be permanently integrated into the garment, such as by being sewn into or permanently adhered onto the garment. In examples, at least some of the components may be connected to each other through cables, through sewn-in connections (e.g., wires woven into the fabric of the garment), through conductive fabric of the garment, and/or the like. Other component configurations are also possible, such as adhesive patches containing sensing and/or therapy electrodes that are applied directly to the patient's skin.

The sensing electrodes can be configured to detect one or more cardiac signals, including ECG signals. Example sensing electrodes include a metal electrode with an oxide coating such as tantalum pentoxide electrodes. For example, by design, digital sensing electrodes can include skin-contacting electrode surfaces that may be deemed polarizable or non-polarizable depending on a variety of factors including the metals and/or coatings used in constructing the electrode surface. For example, the electrode surfaces can be based on stainless steel, noble metals such as platinum, or Ag-AgCl. In some examples, sensing electrodes can be used with an electrolytic gel dispersed between the electrode surface and the patient's skin. In certain implementations, the sensing electrodes can be dry electrodes that do not need an electrolytic material. As an example, such a dry electrode can be based on tantalum metal and having a tantalum pentoxide coating.

In implementations, the sensing electrodes and/or therapy electrodes may be operatively coupled to the medical device controller via the connection pod. The connection pod may be configured to perform at least some processing on signals received from the electrodes before the signals are passed onto the medical device controller. For example, the connection pod can include a signal processor configured to amplify, filter, and digitize these cardiac signals prior to transmitting the cardiac signals to the medical device controller. In implementations, the connection pod may be configured to be mounted onto the garment as well, such as by snapping into framing configured to receive the connection pod located at the base of the garment on the back.

Using the cardiac signals, the medical device controller is configured to monitor the patient to detect any life-threatening arrhythmias or other cardiac events. Alternatively, as shown, the WCD 205 may be connected to the decentralized network 210. As such, some or all of the detection of life-threatening arrhythmias or other cardiac events may be performed using the decentralized network 210, such as the edge server 260. If it is determined that the patient is experiencing a treatable arrhythmia, the medical device controller initiates a treatment sequence. The medical device controller first activates an alarm configured to alert the patient that a therapeutic shock will be imminently delivered to the patient. The alarm may be delivered, for example, using sound, lights, haptic vibrations, and/or the like. For instance, the medical device controller may issue an alarm sound, issue verbal instructions using a speaker (e.g., on the medical device controller, on the patient interface pod, etc.), activate lights on the medical device controller, activate a buzzer (e.g., located in the connection pod, on the patient interface pod, etc.), and so on. The alarm may also escalate over time, such as by becoming louder and/or by other elements being added to the alarm, such as verbal instructions telling the patient to press a response button or for bystanders to stand back from the patient.

Once the alarm is activated, the patient may have a predetermined amount of time before a therapeutic shock is delivered. If the patient is conscious, the patient can delay or cancel the therapeutic shock by pressing one or more response buttons. In examples, such response buttons may be located on the medical device controller. In examples, such response buttons may be located on the patient interface pod. which may be configured to be attached to one of the straps of the garment or elsewhere on the garment or patient's clothing.

If the patient does not respond via the one or more response buttons within the predetermined amount of time, the medical device controller proceeds to activate a therapeutic shock. The therapeutic shock may be charged using a parallel-connected capacitor bank of a plurality of capacitors (e.g., two, three. four, or more capacitors). In implementations, the therapeutic shock is a defibrillation pulse delivering between 60 and 180 **J** of energy. In implementations, the defibrillation pulse is a biphasic truncated exponential waveform, whereby the signal can switch between a positive and a negative portion (e.g., charge directions). The therapeutic shock is also delivered Yia the therapy electrodes. Example therapy electrodes can include conductive metal electrodes such as stainless-steel electrodes that include, in certain implementations, one or more conductive gel deployment devices configured to deliver conductive gel between the metal electrode and the patient's skin prior to delivery of the therapeutic shock.

While certain embodiments have been described. these embodiments have been presented by way of example only and are not intended to limit the scope of the present disclosures. Indeed, the novel methods, apparatuses and systems described herein can be embodied in a variety of other forms: furthermore, various omissions, substitutions and changes in the form of the methods, apparatuses and systems described herein can be made without departing from the spirit of the present disclosures. The accompanying clauses and their equivalents are intended to cover such forms or modifications as would fall within the scope and spirit of the present disclosures.

We also disclose embodiments of the disclosure in the following numbered clauses.
1. A computer-implemented method of transferring data between medical devices associated with a patient, the computer-implemented method comprising:
   forming a decentralized network by a plurality of nodes associated with the patient, the plurality of nodes comprising a plurality of medical devices, wherein each medical device of the plurality of medical devices comprises:
      a communications interface comprising a signal transmitter and a network communications driver,
      at least one patient interface device configured to gather patient data from the patient, and
      a processor, memory, and associated circuitry, the processor of each of the plurality of medical devices being configured to generate a network message comprising device capabilities of a respective medical device;
   publishing the network message to the decentralized network by each medical device;
   ranking the device capabilities of two or more first medical devices in the decentralized network by a second medical device in the decentralized network to identify one or more candidate data sources;
   selecting a desired source for the patient data from the one or more candidate data sources based on the ranking;
   publishing a request for the patient data indicating the desired source by the second medical device;
   subscribing to the desired source by the second medical device;
   publishing the patient data to the decentralized network by the desired source in response to the request;
   routing the patient data from the desired source to the second medical device by the decentralized network, and
   consuming the patient data from the desired source by the second medical device.
2. The computer-implemented method of clause 1, comprising providing an indication at one or more nodes of associations between data consumers and data suppliers in the decentralized network.
3. The computer-implemented method of clause 2, comprising:
   joining the decentralized network by a mobile computing device, and
   displaying the indication on a display screen of the mobile computing device.
4. The computer-implemented method of clause 3, comprising:
   displaying the indication on the display screen as part of a caregiver decision support user interface.
5. The computer-implemented method of clause 2, comprising:
   providing the indication at each of the data suppliers in response to a query at a caregiver decision support screen or another decentralized network device to determine which data supplier is providing data to a particular data consumer.
6. The computer-implemented method of clause 1, wherein the device capabilities of the respective medical device comprise one or more types of patient data available from the respective medical device.
7. The computer-implemented method of clause 6, comprising:
   determining a desired type of patient data by the second medical device,
   identifying the two or more first medical devices as potential sources for the desired type of patient data,
   selecting the desired source from the potential sources.
8. The computer-implemented method of clause 7,
   wherein the desired type of patient data is one of a plurality of types of patient data available from the desired source,
   the computer-implemented method comprising:
      publishing the desired type of patient data to the decentralized network by the desired source without publishing other types of patient data available from the desired source.
9. The computer-implemented method of clause 7,
   wherein the device capabilities of the respective medical device comprise one or more of a patient data sampling frequency or a patient data resolution associated with each of the one or more types of patient data,
   the computer-implemented method comprising selecting the desired source based at least in part on one or more of the patient data sampling frequency or the patient data resolution.
10. The computer-implemented method of clause 9, comprising:
   determining a desired data compression for a patient data type based at least in part on one or more of the patient data sampling frequency or the patient data resolution,
   publishing the request for the patient data with an indication of the desired data compression,
   compressing the patient data by the desired source in response to the published request, and
   publishing the compressed patient data to the decentralized network by the desired source.
11. The computer-implemented method of clause 7, comprising:
   receiving an indication by the second medical device that the desired source has exited the decentralized network,
   identifying a substitute source for the desired type of patient data in the decentralized network by the second medical device, and
   re-publishing the request for the patient data by the second medical device with an indication of the substitute source.
12. The computer-implemented method of clause 7,
   wherein the two or more first medical devices are previously identified sources for the desired type of patient data,
   the computer-implemented method comprising:
      joining the decentralized network by one or more third medical devices, wherein at least one of the one or more third medical devices is capable of providing the desired type of patient data,
      publishing network messages of the one or more third medical devices to the decentralized network by the one or more third medical devices,
      ranking the device capabilities of the two or more first medical devices with the one or more third medical devices, and
      selecting an updated desired source.
13. The computer-implemented method of clause 6, wherein the device capabilities of the respective medical device comprise data transmission parameters for the respective medical device.
14. The computer-implemented method of clause 13, wherein the data transmission parameters comprise one or more of a signal strength, an indication of a wired communicative coupling, or an indication of a wireless communicative coupling.
15. The computer-implemented method of clause 13, wherein the data transmission parameters comprise a decentralized network routing protocol for the respective medical device.
16. The computer-implemented method of clause 1, comprising:
   assigning a patient identification code to the patient,
   generating a patient-specific network access code from the patient identification code by a first medical device of the plurality of medical devices or by a mobile computing device,
   receiving join requests at the first medical device from a remainder of the plurality of medical devices or at the mobile computing device from the plurality of medical devices,
   providing the patient-specific network access code to the remainder of the plurality of medical devices by the first medical device or to the plurality of medical devices by the mobile computing device, and
   forming the decentralized network based on the patient-specific network access code.
17. The computer-implemented method of clause 16, comprising:
   assigning the patient identification code by a computer aided dispatch (CAD) service, and
   sending the patient identification code from the CAD service to the first medical device or the mobile computing device.
18. The computer-implemented method of clause 16, comprising:
   affixing a quick response (QR) code or bar code comprising the patient identification code to the patient by a caregiver to assign the patient identification code, and
   scanning the QR code or bar code by the first medical device or the mobile computing device to generate the patient-specific network access code.
19. The computer-implemented method of clause 16, wherein the mobile computing device comprises a patient charting device, a dispatch interface device, or a combination thereof.
20. The computer-implemented method of clause 16, comprising:
   enabling an exchange of handshake information amongst a collection of medical devices and computing devices prior to an arrival at a patient scene of any of the collection of medical devices and computing devices to pre-configure the collection of medical devices and computing devices for decentralized network entry,
   generating an inventory list, and
   storing the inventory list in a storage location accessible to the collection of medical devices,
   wherein the collection of medical devices and computing devices includes the plurality of medical devices and the mobile computing device and is associated with at least one of an EMS agency, an EMS transport vehicle, or an EMS crew.
21. The computer-implemented method of clause 20, wherein the storage location is a central registration service, a peer-to-peer storage, a blockchain ledger, or a combination thereof.
22. The computer-implemented method of clause 1, comprising:
   joining the decentralized network by at least one additional medical device comprising the network interconnection protocol stack, and
   permanently exiting the decentralized network by at least one of the plurality of medical devices in the decentralized network.
23. The computer-implemented method of clause 22,
   wherein the plurality of medical devices are associated with a basic life support (BLS) emergency medical services (EMS) crew, and
   wherein the at least one additional medical device is associated with an advanced life support (ALS) EMS crew.
24. The computer-implemented method of clause 23, wherein the plurality of medical devices comprise an automated external defibrillator and trauma kit.
25. The computer-implemented method of clause 23,
   wherein the plurality of medical devices are associated with and an EMS agency, and
   wherein the at least one additional medical device is associated with a hospital.
26. The computer-implemented method of clause 23,
   wherein the plurality of medical devices comprises a physiological sensor coupled to the patient,
   the computer-implemented method comprising:
      forming the decentralized network with nodes comprising a patient charting device,
      receiving physiological parameter values by the patient charting device from the physiological sensor via the decentralized network,
      monitoring the physiological parameter values for the patient by the BLS EMS crew at the patient charting device,
      joining the decentralized network by at least one ALS medical device,
      permanently exiting the decentralized network by the patient charting device, and
      receiving the physiological parameter values by the at least one ALS medical device from the physiological sensor via the decentralized network,
   wherein the at least one ALS medical device receives the physiological parameter values without a communicative coupling between the at least one ALS medical device and the physiological sensor other than through the decentralized network.
27. The computer-implemented method of clause 26, wherein the at least one ALS medical device comprises one or more of an ALS patient monitor, an ALS patient monitor/defibrillator, or a critical care ventilator.
28. The computer-implemented method of clause 26, wherein the physiological sensor comprises an SpO2 sensor.
29. The computer-implemented method of clause 22,
   wherein the plurality of medical devices comprises a BLS respiratory distress management (RDM) system coupled to the patient via a ventilation hose and managing respiratory distress based on a set of RDM parameters,
   the computer-implemented method comprising:
      joining the decentralized network by an ALS critical care ventilator,
      receiving the set of RDM parameters by the ALS critical care ventilator via the decentralized network from the BLS RDM system,
      setting operational parameters for the ALS critical care ventilator to the set of RDM parameters,
      moving a connection port of the ventilation hose coupled to the patient from the BLS RDM system to the ALS critical care ventilator during a single inter-breath time interval,
      exiting the decentralized network by the BLS RDM system, and
      providing ventilation support for the patient with the ALS critical care ventilator based on the set of RDM parameters,
      wherein the ALS critical care ventilator receives the set of RDM parameters from the BLS RDM system without a communicative coupling between the BLS RDM system and the ALS critical care ventilator other than through the decentralized network.
30. The computer-implemented method of clause 29, comprising
   receiving the set of RDM parameters by the ALS critical care ventilator via the decentralized network from the BLS RDM system,
   setting the operational parameters for the ALS critical care ventilator to the set of RDM parameters, and
   moving the connection port of ventilation hose coupled to the patient from the BLS RDM system to the ALS critical care ventilator,
   all during a single inter-breath delivery time interval.
31. The computer-implemented method of clause 1, wherein the plurality of medical devices comprises a first medical device and a first sensor, the computer-implemented method comprising:
   initiating closed loop control of the first medical device based on first patient data from the first sensor by a node in the decentralized network, and
   in response to the initiating:
      publishing first patient data to the decentralized network by the first sensor, and
      subscribing to the first patient data by the first medical device,
      receiving the first patient data at the first medical device via the decentralized network, and
      operating the first medical device in closed loop control based on the first patient data.
32. The computer-implemented method of clause 31, wherein the first medical device comprises a portable ventilator and the first sensor comprises an oxygen saturation sensor.
33. The computer-implemented method of clause 31, comprising:
   permanently exiting the decentralized network by the node in the decentralized network, and
   maintaining the closed loop control of the first medical device in without the exited node based on a continued provision of the first patient data from the first sensor to the first medical device via the decentralized network.
34. The computer-implemented method of clause 33, comprising:
   entering the decentralized network by a new medical device or a new sensor,
   publishing the network message to the decentralized network by the new medical device or the new sensor, wherein the network message indicates new patient data available from the new medical device or the new sensor,
   determining by the first medical device that the new patient data is interchangeable with the first patient data,
   subscribing to the new patient data by the first medical device,
   receiving the new patient data via the decentralized network, and
   modifying the closed loop control of the first medical device to operate based on the new patient data instead of the first patient data.
35. The computer-implemented method of clause 33, comprising:
   forming the decentralized network with nodes comprising a mobile computing device, wherein the node initiating the closed loop control is the mobile computing device.
36. The computer-implemented method of clause 31, comprising initiating the closed loop control by another medical device of the plurality of medical devices.
37. The computer-implemented method of clause 36, wherein the first medical device comprises a portable ventilator, the first sensor comprises an oxygen saturation sensor, and the another medical device comprises a patient monitor/defibrillator.
38. The computer-implemented method of clause 1, comprising:
   forming the decentralized network by the plurality of nodes comprising at least one device configured to calculate a patient evaluation score,
   receiving constituent information for the patient evaluation score,
   calculating the patient evaluation score based on the constituent information,
   publishing the patient evaluation score to the decentralized network, and
   utilizing the patient evaluation score at a node in the decentralized network other than the at least one device configured to calculate the patient evaluation score,
   wherein the utilizing comprises:
      displaying the patient evaluation score,
      incorporating the patient evaluation score into a patient data analysis, or
      a combination thereof.
39. The computer-implemented method of clause 38,
   wherein the constituent information comprises caregiver observation input,
   the computer-implemented method comprising:
      forming the decentralized network by the plurality of nodes, the plurality of nodes comprising at least one device configured to receive caregiver observation input,
      receiving the caregiver observation input at the at least one device configured to receive caregiver observation input,
      calculating the patient evaluation score based at least in part on the caregiver observation input by the at least one device configured to calculate the patient evaluation score,
      publishing the patient evaluation score to the decentralized network by the at least one device configured to calculate the patient evaluation score, and
      displaying the patient evaluation score at the node in the decentralized network other than the at least one device configured to calculate the patient evaluation score.
40. The computer-implemented method of clause 39,
   wherein the constituent information comprises physiological sensor measurements,
   the computer-implemented method comprising:
      collecting one or more physiological sensor measurements at one or more respective patient interface devices,
      publishing the one or more physiological sensor measurements to the decentralized network,
      receiving the one or more physiological sensor measurements via the decentralized network at the at least one device configured to calculate the patient evaluation score, and
      calculating the patient evaluation score based on the one or more physiological sensor measurements.
41. The computer-implemented method of clause 40, wherein the patient evaluation score comprises a modified early warning score (MEWS), the computer-implemented method comprising:
   collecting a blood pressure (BP) measurement, a temperature measurement, a heart rate (HR) measurement, and a respiratory rate (RR) measurement at a BP sensor, a temperature sensor, a HR sensor, and a RR sensor, respectively, wherein the BP sensor, the temperature sensor, the HR sensor, and the RR sensor are all nodes in the decentralized network,
   publishing the BP measurement, the temperature measurement, the HR measurement, and the RR measurement to the decentralized network,
   receiving caregiver evaluations of alertness, voice, pain, and unresponsive (AVPU) scale at the at least one device configured to receive the caregiver observation input,
   publishing the AVPU scale to the decentralized network,
   receiving the BP measurement, the temperature measurement, the HR measurement, the RR measurement, and the AVPU scale at the at least one device configured to calculate the MEWS,
   calculating the MEWS at the at least one device configured to calculate the patient evaluation score, and
   publishing the MEWS to the decentralized network.
42. The computer-implemented method of clause 38, comprising:
   receiving the patient evaluation score at a decentralized network node comprising a single parameter physiological sensor configured to measure a single physiological parameter,
   receiving the patient evaluation score via the decentralized network, and
   displaying the patient evaluation score and the single physiological parameter at the single parameter physiological sensor.
43. The computer-implemented method of clause 42, wherein the patient evaluation score is a Glasgow coma score (GCS) score and the single parameter physiological sensor is a blood glucose monitor.
44. The computer-implemented method of clause 42, wherein the single parameter physiological sensor is a disposable sensor.
45. The computer-implemented method of clause 38, wherein
   the at least one device configured to calculate the patient evaluation score comprises a compensatory reserve index (CRI) sensor configured to calculate a CRI for the patient, and
   the plurality of nodes comprises an ultrasound imaging device,
   the computer-implemented method comprising:
      publishing the CRI to the decentralized network by the CRI sensor, and
      displaying the CRI at the ultrasound imaging device.
46. The computer-implemented method of clause 38, comprising generating an alert at one or more nodes of the decentralized network based on the patient evaluation score,
   wherein the calculating of the patient evaluation score requires information from at least two different nodes in the decentralized network.
47. The computer-implemented method of clause 1, comprising forming the decentralized network in a military environment, wherein each of the plurality of medical devices is configured to be carried in a backpack, worn by military personnel, or a combination thereof.
48. The computer-implemented method of clause 47, wherein the plurality of medical devices comprises at least one of:
   a ruggedized patient monitor weighing less than 5 kg,
   a ruggedized patient monitor/defibrillator weighing less than 6 kg,
   an impedance threshold device coupled to a facemask or a mouthpiece,
   a ruggedized portable ventilator weighing less than 5 kg, or
   a portable and self-contained suction device.
49. The computer-implemented method of clause 48, wherein the ruggedized patient monitor, the ruggedized patient monitor/defibrillator, and the ruggedized portable ventilator are configured to withstand a shock g-force of 65-85 g.
50. The computer-implemented method of clause 47, wherein the plurality of medical devices comprises at least one of:
   a tourniquet,
   an intravenous delivery system,
   an airway management device,
   a diagnostic device comprising at least one of a blood pressure cuff, a pulse oximeter, a stethoscope, or a thermometer,
   a medication kit,
   a stretcher, or
   a surgical kit.
51. The computer-implemented method of clause 47, wherein
   the plurality of medical devices is associated with one or more military caregivers, and
   a total weight of one or more medical devices associated with any one military caregiver is less than 45 kg.
52. The computer-implemented method of clause 47, comprising forming the decentralized network in the military environment without Internet access for the decentralized network.
53. The computer-implemented method of clause 52,
   wherein at least one of the plurality of medical devices includes a Wi-Fi chip,
   the computer-implemented method comprising:
      disabling Wi-Fi frequency transmissions for the at least one of the plurality of medical devices that includes the Wi-Fi chip.
54. The computer-implemented method of clause 47, the computer-implemented method comprising:
   collecting a first physiological parameter at a first node of the plurality of nodes and a second physiological parameter at a second node of the plurality of nodes,
   publishing the first physiological parameter and the second physiological parameter to the decentralized network by the first node and the second node, respectively, and
   monitoring the first physiological parameter and the second physiological parameter by a third node different from the first node and the second node.
55. The computer-implemented method of clause 54, wherein the third node comprises a ruggedized patient monitor, a ruggedized patient monitor/defibrillator, or a ruggedized portable ventilator.
56. The computer-implemented method of clause 54, wherein the plurality of nodes comprises a computer tablet or smartphone and the third node is one of the computer tablet or the smartphone.
57. The computer-implemented method of clause 54, comprising:
   exiting the decentralized network by the third node,
   entering the decentralized network by a fourth node, and
   monitoring the first physiological parameter and the second physiological parameter by the fourth node.
58. The computer-implemented method of clause 47, wherein at least two of the plurality of medical devices are disposed at different locations on a military caregiver.
59. The computer-implemented method of clause 58, wherein all of the plurality of medical devices are pocket-sized, wearable, or a combination thereof.
60. The computer-implemented method of clause 47, wherein the plurality of medical devices are distributed amongst two or more military caregivers.
61. The computer-implemented method of clause 1, comprising:
   forming the decentralized network by the plurality of nodes comprising at least one caregiver guidance device configured to dynamically determine caregiver guidance,
   receiving the patient data at the at least one caregiver guidance device,
   dynamically determining caregiver guidance by the at least one caregiver guidance device based on the patient data and the plurality of medical devices in the decentralized network,
   providing a caregiver guidance user interface (UI) at one or more nodes in the decentralized network, and
   providing the caregiver guidance at the caregiver guidance UI by the at least one caregiver guidance device.
62. The computer-implemented method of clause 61, wherein the plurality of medical devices comprises the at least one caregiver guidance device.
63. The computer-implemented method of clause 61, wherein the at least one caregiver guidance device is a mobile computing device.
64. The computer-implemented method of clause 61, wherein providing the caregiver guidance UI comprises providing the caregiver guidance UI at the at least one caregiver guidance device.
65. The computer-implemented method of clause 61, wherein providing the caregiver guidance UI comprises providing a distributed UI at two or more nodes of the decentralized network.
66. The computer-implemented method of clause 61, wherein the dynamically determining the caregiver guidance comprises:
   determining the caregiver guidance based on the plurality of medical devices in the decentralized network,
   exiting the decentralized network by at least one medical device of the plurality of medical devices,
   modifying the caregiver guidance in response to the exiting, and
   providing the modified caregiver guidance at the caregiver guidance UI.
67. The computer-implemented method of clause 61, wherein the dynamically determining the caregiver guidance comprises:
   determining the caregiver guidance based on the plurality of medical devices in the decentralized network,
   entering the decentralized network by at least one new medical device,
   modifying the caregiver guidance in response to the entering, and
   providing the modified caregiver guidance at the caregiver guidance UI.
68. The computer-implemented method of clause 61, comprising:
   provisioning at least one services library with EMS medical device information,
   forming the decentralized network by the plurality of nodes comprising the at least one services library,
   tagging a subset of the EMS medical device information as medical device information corresponding to the plurality of medical devices based on the published network messages, and
   providing at least a portion of the tagged subset of the EMS medical device information to the at least one caregiver guidance device.
69. The computer-implemented method of clause 68, wherein the tagged subset of the EMS medical device information comprises at least one of medical device drivers, medical device operation and assembly instructions, medical device operation specifications, or medical device identification information.
70. The computer-implemented method of clause 61 comprising:
   providing a graphical representation of at least one of the plurality of medical devices at the caregiver guidance UI.
71. The computer-implemented method of clause 70, comprising:
   providing a user control associated with the graphical representation,
   receiving a selection of the user control,
   receiving, via the decentralized network, operational interface information from the graphically represented at least one medical device, and
   displaying the operational interface information at the caregiver guidance UI.
72. The computer-implemented method of clause 1, comprising:
   joining the decentralized network by at least one mobile computing device having the network communication protocol stack,
   subscribing to the first patient data by the at least one mobile computing device, and
   publishing second patient data to the decentralized network by the at least one mobile computing device.
73. The computer-implemented method of clause 72,
   wherein the at least one mobile computing device comprises a patient charting device,
   the computer-implemented method comprising:
      receiving the first patient data at the patient charting device,
      mapping the first patient data to one or more data fields in an electronic patient care record (ePCR),
      storing the first patient data in the ePCR.
74. The computer-implemented method of clause 73,
   wherein the at least one of the plurality of medical devices comprises at least one of a patient monitor, a patient monitor/defibrillator, a portable ventilation device, an automated external defibrillator (AED), or an automated compression device.
75. The computer-implemented method of clause 73, comprising:
   receiving the second patient data comprising one or more of patient demographic data or symptom-allergy-medication-pertinent history (SAMPLE) data, and
   publishing at least one of the patient demographic data or the SAMPLE data to the decentralized network.
76. The computer-implemented method of clause 73, comprising
   wherein the at least one mobile computing device comprises an in-vehicle computer aided dispatch (CAD) and navigation interface (CAD-NAV) device configured to communicate with a CAD service and with a global positioning system (GPS).
77. The computer-implemented method of clause 76, comprising:
   receiving the second patient data at the patient charting device from the CAD service via the CAD-NAV device and the decentralized network without a communicative coupling between the patient charting device and a remote patient charting server.
78. The computer-implemented method of clause 76, comprising:
   receiving location information at the patient charting device from the CAD-NAV device, the location information comprising a current EMS vehicle GPS location and a patient transport destination,
   determining a travel time from the current EMS vehicle GPS location to the patient transport destination
   generating a caregiver recommendation by the patient charting device based at least in part on the location information,
   providing the caregiver recommendation via a user interface of the patient charting device.
79. The computer-implemented method of clause 78,
   wherein generating the caregiver recommendation comprises sorting patient care protocol tasks into on-scene tasks prior to transport and in-ambulance tasks during transport,
   the computer-implemented method comprising generating a caregiver alert to begin patient transport and transition from the on-scene tasks to the in-ambulance tasks.
80. The computer-implemented method of clause 78, wherein the caregiver recommendation comprises one of a fluid delivery rate or volume or a medication dosage based on the travel time to the patient transport destination.
81. The computer-implemented method of clause 78, comprising:
   documenting at least one patient condition in the ePCR,
   providing the at least one patient condition to the CAD-NAV device,
   modifying the patient transport destination by the CAD-NAV device based on the at least one patient condition, and
   reporting the modified patient transport destination to the CAD.
82. The computer-implemented method of clause 78, comprising:
   documenting at least one patient condition in the ePCR,
   providing the at least one patient condition to the CAD-NAV device,
   determining closest appropriate facility information for the at least one patient condition by the CAD-NAV device based on the location information, and
   providing the closest appropriate facility information to the patient charting device, and
   automatically recording the closest appropriate facility information in the ePCR.
83. The computer-implemented method of clause 1, wherein the plurality of medical devices comprises a patient sensor hub.
84. The computer-implemented method of clause 1, comprising forming the decentralized network with at least one edge server.
85. The computer-implemented method of clause 1, comprising forming the decentralized network without any nodes having a communicative coupling to the Internet or a remote server.
86. The computer-implemented method of clause 1, wherein at least one of the plurality of medical devices is non-wearable and configured to be supported by a surface in a patient care environment separate from the patient.
87. The computer-implemented method of clause 1, wherein the plurality of medical devices are external medical devices.
88. The computer-implemented method of clause 1, wherein the plurality of medical devices are mobile medical devices.
89. The computer-implemented method of clause 1, wherein the plurality of medical devices comprises two or more of a patient monitor, a patient monitor/defibrillator, an automated external defibrillator, a portable ventilator, and an automated compression device.
90. The computer-implemented method of clause 1, wherein the network communications driver comprises a network interconnection protocol stack wherein at least one layer of the network interconnection protocol stack for each of the plurality of medical devices is a same layer as each other of the plurality of medical devices.
91. The computer-implemented method of clause 90, wherein each of the plurality of medical devices has a same data link layer, network layer, transport layer, and session layer as each other of the plurality of medical devices.
92. The computer-implemented method of clause 90, wherein the plurality of nodes comprises at least one computing device comprising the network communications driver provisioned with a network interconnection protocol stack having at least one same layer as each of the plurality of medical devices.
93. The computer-implemented method of clause 90, wherein the network communications driver is a first network communications driver associated with a first medical device manufacturer, the computer-implemented method comprising:
   requesting to join the decentralized network by at least one medical device associated with a second medical device manufacturer,
   utilizing a second network communications driver that allows the at least one medical device associated with the second medical device manufacturer to participate in the decentralized network as a data supplier and disallows participation as a data consumer, and
   entering the decentralized network by the at least one medical device associated with the second medical device manufacturer based on the second network communications driver.
94. The computer-implemented method of clause 93, comprising retrieving the second network communications driver from a cloud server.
95. The computer-implemented method of clause 1, wherein the communications interface of at least one of the plurality of medical devices comprises a short-range wireless interface configured to join the decentralized network via a dongle on the at least one of the plurality of medical devices, via an edge server, or via an intermediary medical device of the plurality of medical devices.
96. The computer-implemented method of clause 1, wherein forming the decentralized network comprises forming a mesh network.
97. The computer-implemented method of clause 96, comprising forming a mesh network with a partial mesh topology.
98. The computer-implemented method of clause 96, comprising forming a mesh network with a full mesh topology.
99. The computer-implemented method of clause 1, wherein the decentralized network includes a first subset of devices with an application layer configured to enable publish and subscribe functions and a second subset of devices with an application layer configured to enable publish functions without subscribe functions.
100. The computer-implemented method of clause 1, wherein the signal transmitter is configured for wired and/or wireless communications.
101. The computer-implemented method of clause 1, wherein ranking the device capabilities comprises:
   comparing the device capabilities for the two or more first medical devices to data subscription criteria for the second medical device,
   identifying one first medical device as a singular data source, and
   identifying the singular data source as the candidate data source.
102. The computer-implemented method of clause 1, wherein ranking the device capabilities comprises:
   comparing the device capabilities for the two or more first medical devices to data subscription criteria for the second medical device, and
   identifying one or more of the first medical devices as non-candidate data sources.
103. The computer-implemented method of clause 1, wherein ranking the device capabilities comprises:
   comparing the device capabilities for the two or more first medical devices to data subscription criteria for the second medical device,
   identifying two or more of the first medical devices as candidate data sources,
   generating a compatibility score for each candidate data source, and
   ranking the candidate data sources based on the compatibility scores.
104. The computer-implemented method of clause 103, wherein generating the compatibility score comprises generating the compatibility score based on an evaluation of device capabilities of each candidate data source and the data subscription criteria.
105. The computer-implemented method of clause 104 wherein the data subscription criteria comprise data types, sampling rates types of communicative coupling, sampling rates, and data tolerances.
106. The computer-implemented method of clause 103, comprising identifying exchangeable and nonexchangeable data sources.
107. The computer-implemented method of clause 106, comprising:
   detecting a degradation in data provision from the desired source,
   selecting an exchangeable data source as a new desired data source,
   publishing a request for patient data indicating the new desired data source, and
   subscribing to the new desired data source by the second medical device.
108. The computer-implemented method of clause 107, comprising:
   comparing the degradation in data provision from the desired source to a threshold degradation, and
   publishing the request for patient data indicating the new desired data source when the degradation reaches the threshold degradation.
109. The computer-implemented method of clause 1, wherein the plurality of medical devices comprises two or more of a patient monitor/defibrillator, an automated external defibrillator (AED), or a wearable cardio-defibrillator (WCD), a trauma kit, an automated compression device, a portable ventilation device, a drug delivery device, and an ultrasound imaging device.
110. The computer-implemented method of clause 1, wherein the network communications driver comprises an application layer configured to implement a first wired subscription paradigm and a second wireless subscription paradigm, the method comprising:
   implementing the second wireless subscription paradigm,
   receiving a radio-silence request, and
   switching to the first wired subscription paradigm.
111. A system for transferring data between medical devices associated with a patient, the system comprising:
   a plurality of medical devices communicatively coupled via a decentralized network, wherein each of the plurality of medical devices is a node in the decentralized network, and
   wherein each medical device of the plurality of medical devices comprises:
      a communications interface comprising a signal transmitter and a network communications driver,
      at least one patient interface device configured to gather patient data from the patient, and
      a processor, memory, and associated circuitry,
   and wherein at least one first medical device of the plurality of medical devices comprises an application layer of the network communications driver configured to cause a respective processor to:
      receive network messages comprising device capabilities of other medical devices of the plurality of medical devices;
      rank the device capabilities of two or more of the other medical devices in the to identify one or more candidate data sources in the decentralized network;
      select a desired source for the patient data from the one or more candidate data sources based on the ranking;
      publish a request for the patient data indicating the desired source by the at least one first medical device;
      subscribe to the desired source;
      receive the patient data from the desired source via the decentralized network; and
      consume the patient data from the desired source by the at least one first medical device.
112. The system of clause 111, wherein the plurality of medical devices comprises two or more of a patient monitor/defibrillator, an automated external defibrillator (AED), or a wearable cardio-defibrillator (WCD), a trauma kit, an automated compression device, a portable ventilation device, a drug delivery device, and an ultrasound imaging device.
113. The system of clause 111, wherein the decentralized network comprises at least one edge server.
114. The system of clause 111, wherein none of the nodes of the decentralized network are communicatively coupled to the Internet or a remote server.
115. The system of clause 111, wherein at least one of the plurality of medical devices is non-wearable and configured to be supported by a surface in a patient care environment separate from the patient.
116. The system of clause 111, wherein the plurality of medical devices are external medical devices.
117. The system of clause 111, wherein the plurality of medical devices are mobile medical devices.
118. The system of clause 111, wherein the network communications driver comprises a network interconnection protocol stack wherein at least one layer of the network interconnection protocol stack for each of the plurality of medical devices is a same layer as each other of the plurality of medical devices.
119. The system of clause 118, wherein each of the plurality of medical devices has a same data link layer, network layer, transport layer, and session layer as each other of the plurality of medical devices.
120. The system of clause 118, wherein the plurality of nodes comprises at least one computing device comprising the network communications driver provisioned with a network interconnection protocol stack having at least one same layer as each of the plurality of medical devices.
121. The system of clause 118,
   wherein the network communications driver is a first network communications driver associated with a first medical device manufacturer, and
   wherein the decentralized network comprises
   at least one medical device associated with a second medical device manufacturer that is provisioned
   to participate in the decentralized network as a data publisher without data subscription.
122. The system of clause 111, wherein the communications interface of at least one of the plurality of medical devices comprises a short-range wireless interface configured to join the decentralized network via a dongle on the at least one of the plurality of medical devices, via an edge server, or via an intermediary medical device of the plurality of medical devices.
123. The system of clause 111, wherein the decentralized network comprises a mesh network.
124. The system of clause 123, wherein the mesh network has a partial mesh topology.
125. The system of clause 123, wherein the mesh network has a full mesh topology.
126. The system of clause 111, wherein the decentralized network includes a first subset of devices with an application layer configured to enable publish and subscribe functions and a second subset of devices with an application layer configured to enable publish functions without subscribe functions.
127. The system of clause 111, wherein the signal transmitter is configured for wired and/or wireless communications.
128. The system of clause 111, wherein to rank the device capabilities, the application layer is configured to cause the respective processor to:
   compare the device capabilities for the two or more other medical devices to data subscription criteria for the at least one first medical device,
   identify one of the other medical devices as a singular data source, and
   identify the singular data source as the candidate data source.
129. The system of clause 111, wherein to rank the device capabilities, the application layer is configured to cause the respective processor to:
   compare the device capabilities for the two or more other medical devices to data subscription criteria for the at least one first medical device, and
   identify one or more of the other medical devices as non-candidate data sources.
130. The system of clause 111, wherein to rank the device capabilities, the application layer is configured to cause the respective processor to:
   compare the device capabilities for the two or more other medical devices to data subscription criteria for the at least one first medical device,
   identify two or more of the other medical devices as candidate data sources,
   generate a compatibility score for each candidate data source, and
   rank the candidate data sources based on the compatibility scores.
131. The system of clause 130, wherein to generate the compatibility score, the application layer is configured to cause the respective processor to generate the compatibility score based on an evaluation of device capabilities of each candidate data source and the data subscription criteria.
132. The system of clause 131 wherein the data subscription criteria comprise data types, sampling rates types of communicative coupling, sampling rates, and data tolerances.
133. The system of clause 130, wherein the application layer is configured to cause the respective processor to identify exchangeable and nonexchangeable data sources.
134. The system of clause 133, wherein the application layer is configured to cause the respective processor to:
   detect a degradation in data provision from the desired source,
   select an exchangeable data source as a new desired data source,
   publish a request for patient data indicating the new desired data source, and
   subscribe to the new desired data source by the at least one first medical device.
135. The system of clause 134, wherein the application layer is configured to cause the respective processor to:
   compare the degradation in data provision from the desired source to a threshold degradation, and
   publish the request for patient data indicating the new desired data source when the degradation reaches the threshold degradation.
136. The system of clause 111, wherein the decentralized network comprises at least one mobile computing device.
137. The system of clause 136, wherein at least one mobile computing device is configured to display a caregiver decision support user interface with an indication of associations between data consumers and data suppliers within the decentralized network.
138. The system of clause 111, wherein the device capabilities comprise one or more types of patient data available from the respective medical device, and one or more of a patient data sampling frequency, data transmission parameters, or a patient data resolution associated with each of the one or more types of patient data.
139. The system of clause 138, wherein the data transmission parameters comprise one or more of a signal strength, an indication of a wired communicative coupling, an indication of a wireless communicative coupling, or routing protocol.
140. The system of clause 111, wherein at least one node of the decentralized network is configured to:
   receive a patient identification code for the patient,
   generate a patient-specific network access code from the patient identification code,
   receive join requests from one or more of the plurality of medical devices, and
   provide the patient-specific network access code to the remainder of the plurality of medical devices by the first medical device or to the plurality of medical devices by the mobile computing device to form the decentralized network based on the patient-specific network access code.
141. The system of clause 140, wherein the patient identification code is a code assigned by a computer aided dispatch service or a quick response code affixed to the patient.
142. The system of clause 111, wherein the plurality of medical devices are configured to exchange handshake information to generate an inventory list for the decentralized network.
143. The system of clause 142, wherein the inventory list is available to the decentralized network as a central registration service, a peer-to-peer storage, a blockchain ledger, or a combination thereof.
144. The system of clause 111, wherein each of the plurality of medical devices is configured to be carried in a backpack, worn by military personnel, or a combination thereof.
145. The system of clause 144, wherein the plurality of medical devices comprises at least one of:
   a ruggedized patient monitor weighing less than 5 kg,
   a ruggedized patient monitor/defibrillator weighing less than 6 kg,
   an impedance threshold device coupled to a facemask or a mouthpiece,
   a ruggedized portable ventilator weighing less than 5 kg, or
   a portable and self-contained suction device.
146. The system of clause 145, wherein the ruggedized patient monitor, the ruggedized patient monitor/defibrillator, and the ruggedized portable ventilator are configured to withstand a shock g-force of 65-85 g.
147. The system of clause 144, wherein the plurality of medical devices comprises at least one of:
   a tourniquet,
   an intravenous delivery system,
   an airway management device,
   a diagnostic device comprising at least one of a blood pressure cuff, a pulse oximeter, a stethoscope, or a thermometer,
   a medication kit,
   a stretcher, or
   a surgical kit.
148. The system of clause 144, wherein
   the plurality of medical devices is associated with one or more military caregivers, and
   a total weight of one or more medical devices associated with any one military caregiver is less than 45 kg.
149. The system of clause 144, wherein the decentralized network in the military environment lacks Internet access.
150. The system of clause 111,
   wherein the decentralized network comprises one or more of at least one caregiver guidance device configured to dynamically determine caregiver guidance, a patient charting device configured to generate an ePCR, an in-vehicle computer aided dispatch (CAD) and navigation interface (CAD-NAV) device configured to communicate with a CAD service and with a global positioning system (GPS), and a patient sensor hub.

## Claims

1. A computer-implemented method of transferring data between medical devices associated with a patient (101), the computer-implemented method comprising:
forming a decentralized network (210) by a plurality of nodes associated with the patient, the plurality of nodes comprising a plurality of medical devices (2502), wherein each medical device of the plurality of medical devices comprises:
a communications interface comprising a signal transmitter and a network communications driver (399),
at least one patient interface device (270, 2530) configured to gather patient data from the patient, and
a processor, memory, and associated circuitry, the processor of each of the plurality of medical devices being configured to generate a network message comprising device capabilities of a respective medical device;
publishing the network message to the decentralized network by each medical device;
ranking the device capabilities of two or more first medical devices in the decentralized network by a second medical device in the decentralized network to identify one or more candidate data sources;
selecting a desired source for the patient data from the one or more candidate data sources based on the ranking;
publishing a request for the patient data indicating the desired source by the second medical device;
subscribing to the desired source by the second medical device;
publishing the patient data to the decentralized network by the desired source in response to the request;
routing the patient data from the desired source to the second medical device by the decentralized network, and
consuming the patient data from the desired source by the second medical device.

2. The computer-implemented method of claim 1, comprising providing an indication at one or more nodes of associations between data consumers and data suppliers in the decentralized network.

3. The computer-implemented method of claim 2, comprising:
joining the decentralized network by a mobile computing device, and
displaying the indication on a display screen of the mobile computing device; and optionally,
displaying the indication on the display screen as part of a caregiver decision support user interface.

4. The computer-implemented method of claim 2, comprising:
providing the indication at each of the data suppliers in response to a query at a caregiver decision support screen or another decentralized network device to determine which data supplier is providing data to a particular data consumer.

5. The computer-implemented method of claim 1, wherein the device capabilities of the respective medical device comprise one or more types of patient data available from the respective medical device; and the method comprises:
determining a desired type of patient data by the second medical device,
identifying the two or more first medical devices as potential sources for the desired type of patient data,
selecting the desired source from the potential sources.

6. The computer-implemented method of claim 5,
wherein the device capabilities of the respective medical device comprise one or more of a patient data sampling frequency or a patient data resolution associated with each of the one or more types of patient data,
the computer-implemented method comprising selecting the desired source based at least in part on one or more of the patient data sampling frequency or the patient data resolution.

7. The computer-implemented method of claim 5, comprising:
receiving an indication by the second medical device that the desired source has exited the decentralized network,
identifying a substitute source for the desired type of patient data in the decentralized network by the second medical device, and
re-publishing the request for the patient data by the second medical device with an indication of the substitute source.

8. The computer-implemented method of any preceding claim, comprising:
assigning a patient identification code to the patient,
generating a patient-specific network access code from the patient identification code by a first medical device of the plurality of medical devices or by a mobile computing device,
receiving join requests at the first medical device from a remainder of the plurality of medical devices or at the mobile computing device from the plurality of medical devices,
providing the patient-specific network access code to the remainder of the plurality of medical devices by the first medical device or to the plurality of medical devices by the mobile computing device, and
forming the decentralized network based on the patient-specific network access code.

9. The computer-implemented method of claim 8, comprising:
affixing a quick response (QR) code or bar code comprising the patient identification code to the patient by a caregiver to assign the patient identification code, and
scanning the QR code or bar code by the first medical device or the mobile computing device to generate the patient-specific network access code.

10. The computer-implemented method of claim 8, wherein the mobile computing device comprises a patient charting device, a dispatch interface device, or a combination thereof.

11. The computer-implemented method of any preceding claim, comprising:
joining the decentralized network by at least one additional medical device comprising the network interconnection protocol stack, and
permanently exiting the decentralized network by at least one of the plurality of medical devices in the decentralized network; and
wherein the plurality of medical devices are associated with a basic life support, BLS, emergency medical services, EMS, crew, and
wherein the at least one additional medical device is associated with an advanced life support, ALS EMS crew.

12. The computer-implemented method of any preceding claim, wherein the plurality of medical devices comprises a first medical device and a first sensor, the computer-implemented method comprising:
initiating closed loop control of the first medical device based on first patient data from the first sensor by a node in the decentralized network, and
in response to the initiating:
publishing first patient data to the decentralized network by the first sensor, and
subscribing to the first patient data by the first medical device,
receiving the first patient data at the first medical device via the decentralized network, and
operating the first medical device in closed loop control based on the first patient data.

13. The computer-implemented method of any preceding claim, comprising:
forming the decentralized network by the plurality of nodes comprising at least one device configured to calculate a patient evaluation score,
receiving constituent information for the patient evaluation score,
calculating the patient evaluation score based on the constituent information,
publishing the patient evaluation score to the decentralized network, and
utilizing the patient evaluation score at a node in the decentralized network other than the at least one device configured to calculate the patient evaluation score,
wherein the utilizing comprises:
displaying the patient evaluation score,
incorporating the patient evaluation score into a patient data analysis, or
a combination thereof.

14. The computer-implemented method of any preceding claim, comprising:
forming the decentralized network by the plurality of nodes comprising at least one caregiver guidance device configured to dynamically determine caregiver guidance,
receiving the patient data at the at least one caregiver guidance device,
dynamically determining caregiver guidance by the at least one caregiver guidance device based on the patient data and the plurality of medical devices in the decentralized network,
providing a caregiver guidance user interface, UI, at one or more nodes in the decentralized network, and
providing the caregiver guidance at the caregiver guidance UI by the at least one caregiver guidance device.

15. The computer-implemented method of claim 14, wherein the dynamically determining the caregiver guidance comprises:
determining the caregiver guidance based on the plurality of medical devices in the decentralized network,
exiting the decentralized network by at least one medical device of the plurality of medical devices,
modifying the caregiver guidance in response to the exiting, and
providing the modified caregiver guidance at the caregiver guidance UI.

16. The computer-implemented method of any preceding claim, comprising forming the decentralized network without any nodes having a communicative coupling to the Internet or a remote server.
